# EUROPEAN PATENT APPLICATION

(11) **EP 4 019 627 A1**
(43) Date of publication of application: **29.06.2022**
(21) Application number: 20217457.9
(22) Date of filing: 28.12.2020
(51) Int. Cl.: C12N 9/02, C12Q 1/26, C12P 7/22

(54) **NEW ENZYMES AND METHODS FOR BENZOIC ACID-RELATED METABOLIC PATHWAYS**

(71) Applicant: Koninklijke Nederlandse Akademie van Wetenschappen, 1011 JV Amsterdam (NL)
(72) Inventor: LUBBERS, Ronnie Johannes Maria, 3453 NZ Utrecht (NL); DE VRIES, Ronald Peter, 3723 XT Bilthoven (NL)
(74) Representative: Nederlandsch Octrooibureau

(57) **Abstract**

The present invention relates to enzymes from a cluster related to metabolism of benzoic acid, and to host cells comprising these enzymes, and to methods for using the enzymes. The enzymes are useful for the production of protocatechuic acid.

## Description

### Field of the invention

The present invention relates to the field of biotechnology; specifically the production of compounds of benzoic acid-related metabolic pathways such as triple-substituted benzenes like protocatechuic acid and hydroxyquinol.

### Background of the invention

Benzoic acid and compounds of its related pathways have great industrial potential as they can be used directly as building blocks or processed further to create value-added compounds. Benzoic acid is a simple aromatic carboxylic acid which naturally occurs in plants. Protocatechuic acid (PCA), also known as 3,4-dihydroxybenzoic acid, is an aromatic compound which can be a chemical building block for polymers and plastics. Additionally, it has many pharmaceutical properties such as antibacterial, anticancer, antidiabetic, anti-ulcer, antiviral, nematicidal, anti-atherosclerotic, and hyperlipidemic activities. Hydroxyquinol has similar utility. Currently, many aromatic compounds are produced using petroleum-based resources, which is not sustainable and generates unwanted by-products. Accordingly, there is a need for production methods that can utilize renewable and sustainable feedstocks, that have decreased energy requirements, and that result in products with higher purity and less side products than existing processes. An approach that has been researched is biorefining, which uses microbial fermentation to produce chemical building blocks and fuels (Hasunuma et al., 2013; Kawai et al., 2013). Various different homocyclic aromatic metabolic pathways that can lead to the formation of benzoic acid-related compounds such as substituted benzenes exist in bacteria and fungi; an overview is given in the review by Lubbers and co-workers (2019).

In *Aspergillus niger,* benzoic acid is degraded in three steps (Boscloo et al., 1990). Firstly, benzoic acid is p-hydroxylated to p-hydroxybenzoic acid by the benzoate-4-monooxygenase (BphA) (Faber et al., 2001). Then, p-hydroxybenzoic acid is m-hydroxylated to protocatechuic acid by p-hydroxybenzoate-m-hydroxylase (PhhA) (Lubbers et al., 2019). Finally, the ring of protocatechuic acid is cleaved to form 3-carboxy-cis,cis-muconic acid, which is catalyzed by protocatechuate 3,4-dioxygenase (PrcA), and is further degraded through the oxoadipate pathway. The *Aspergillus niger* genes PhhA and PrcA were identified by Lubbers and co-workers (2019).

Guo et al. (2019) utilized a co-culture engineering approach to achieve protocatechuic acid and hydroquinone production in *E. coli,* by co-culturing strains engineered in the shikimate pathway to generate the pathway intermediate 4-hydroxybenzoate (4HB) which was subsequently converted to protocatechuic acid by a strain expressing *Pseudomonas aeruginosa* 4-hydroxybenzoate hydroxylase PobA, or to hydroquinone by a strain expressing *Candida parapsilosis* CBS604 4-hydroxybenzoate 1-hydroxylase Mnx1.

Okai et al. (2016) achieved protocatechuic acid production in engineered *Corynebacterium glutamicum* strain ATCC21420 expressing *E*. *coli* chorismate-pyruvate lyase UbiC.

Williams et al. (2012) demonstrated production of protocatechuic acid in *Bacillus thuringiensis kurstaki* ATCC33679 by cultivation under iron restrictive growth conditions.

Weber et al. (2012) demonstrated the conversion of 3-dehydroshikimate of the aromatic amino acid biosynthesis pathway via protocatechuic acid and catechol into cis,cis-muconic acid in an engineered *Saccharomyces cerevisiae* CEN.PK strain expressing heterologous 3-dehydroshikimate dehydratase, protocatechuic acid decarboxylase composed of three different subunits, and catechol 1,2-dioxygenase.

Omori et al. (1988) demonstrated conversion of trans-ferulic acid to protocatechuic acid in *Pseudomonas sp.* HF-1 by a strain which was defective in protocatechuic acid catabolism after mutagenesis with nitrosoguanidine.

The use of current microbial cell factories in production processes of protocatechuic acid and other benzoic-acid related compounds is associated with disadvantages, such as susceptibility to phage infections, expensive nutrient requirements, low obtained productivities and/or yields, low product purity because of by-product formation, low tolerance to industrial process conditions and toxicity issues associated with the feedstocks, metabolic pathways and/or formed (by-)products. Accordingly, there is a need for improved cell factories and processes for biological production of benzoic acid-related compounds such as protocatechuic acid and hydroxyquinol.

### Summary of the invention

The inventors have found a new enzyme with a useful function. The invention therefore provides the use of a functional enzyme with protocatechuic hydroxylase (Phy) activity for the production of hydroxyquinol. Preferably the enzyme is PhyA from *Aspergillus niger,* preferably from *Aspergillus niger* CBS 120.49 or a derivative thereof. The PhyA preferably has at least 60%, preferably at least 80%, more preferably at least 90%, most preferably 100% sequence identity with SEQ ID NO: 1, or is encoded by a polynucleotide which has at least 60%, preferably at least 80%, more preferably at least 90%, most preferably 100% sequence identity with SEQ ID NO: 2.

The invention also provides a host cell comprising a genetic modification that reduces or eliminates the expression of a functional enzyme with protocatechuic hydroxylase (Phy) activity as defined above, as compared to a corresponding wild type cell lacking the genetic modification. Preferably the host cell is a microbial cell, preferably a fungal cell, more preferably a filamentous fungal cell. Preferably the host cell further comprises a genetic modification that reduces or eliminates the expression of a functional enzyme with protocatechuate 3,4-dioxygenase (Prc) activity as compared to a corresponding wild type cell lacking the genetic modification, preferably wherein the functional enzyme with Prc activity is from *Aspergillus niger,* more preferably from *Aspergillus niger* CBS 120.49, wherein the functional enzyme with Prc activity has at least 60%, preferably at least 80%, more preferably at least 90%, most preferably 100% sequence identity with SEQ ID NO: 4, or is encoded by a polynucleotide which has at least 60%, preferably at least 80%, more preferably at least 90%, most preferably 100% sequence identity with SEQ ID NO: 5. The host cell is preferably capable of producing at least 0.8 mM of protocatechuic acid, more preferably capable of producing at least 1.5 mM of protocatechuic acid, or capable of converting at least 40% of a substrate into protocatechuic acid, preferably capable of converting at least 80% of a substrate into protocatechuic acid. The substrate preferably comprises at least one of protocatechuic aldehyde, benzoic acid, benzaldehyde, benzyl alcohol, p-anisic acid, p-anisaldehyde, p-anisyl alcohol, p-hydroxybenzoic acid, p-hydroxybenzaldehyde, p-hydroxybenzyl alcohol, m-hydroxybenzoic acid, p-coumaric acid, caffeic acid, and cinnamyl alcohol.

The invention also provides a host cell comprising a genetic modification that increases the expression of a functional enzyme with protocatechuic hydroxylase (Phy) activity, as compared to a corresponding wild type cell lacking the genetic modification. A host cell is preferably a filamentous fungal cell, preferably *Aspergillus niger,* wherein the host cell further comprises a genetic modification that reduces or eliminates the expression of a functional enzyme with protocatechuate 3,4-dioxygenase (PrcA) activity as compared to a corresponding wild type cell lacking the genetic modification, and wherein the host cell is capable of converting at least 40% of a substrate into protocatechuic acid. The invention also provides a lysate of such host cells.

The invention also provides an expression vector comprising a nucleic acid construct comprising a nucleotide sequence that has at least 60%, 65%, 70%, 75%, 80%, 85%, 90%, 95%, 96%, 97%, 98%, 99%, or 100% sequence identity with SEQ ID NO: 2 or 10, or wherein the expression vector consists of a polynucleotide that has at least 60%, 65%, 70%, 75%, 80%, 85%, 90%, 95%, 96%, 97%, 98%, 99%, or 100% sequence identity with SEQ ID NO: 3 or 13. The invention also provides a polypeptide product expressed from such an expression vector, wherein the polypeptide preferably has at least 80% sequence identity with SEQ ID NO: 1.

The invention provides a method for the production of protocatechuic acid, comprising the steps of: culturing a cell as defined above under conditions conducive to the production of protocatechuic acid, and, optionally, isolating and/or purifying the protocatechuic acid from the cell and/or the culture medium. Also provided is a method for the production of protocatechuic acid, comprising the steps of: contacting a lysate as defined above with a substrate as defined above under conditions conducive to the production of protocatechuic acid, and, optionally, isolating and/or purifying the protocatechuic acid from the reaction medium.

### Brief description of the drawings

Figure 1 - Protocatechuic acid conversion by PhyA-His6 using cell free extracts of three independent recombinant *E*. *coli* strains (PhyA.1, PhyA.2 and PhyA.3) and an *E.colistrain* harboring the pET-23 plasmid without insert. The reactions contained 500 µM protocatechuic acid and were incubated for 1h at 30 °C.
Figure 2 - Visualisation of PhyA-His by SDS-PAGE and Western blotting using a monoclonal antibody against the histidine-tag. PhyA-His was isolated and purified from three independent recombinant *E. coli* strains (PhyA.1, PhyA.2 and PhyA.3). The estimated molecular mass of PhyA-His, 49.3 kDa, corresponded with the expected mass.
Figure 3 - Protocatechuic acid conversion by purified PhyA-His from three independent recombinant *E. coli* strains (PhyA.1, PhyA.2 and PhyA.3). The reactions contained 100 µM protocatechuic acid and were incubated for 1h at 30°C.
Figure 4 - Growth profile of the *A. niger* reference strain and deletion mutants *ΔphyA, ΔprcA, ΔhqdA, ΔphyAΔprcA,* and *ΔprcAΔhqdA* on aromatic compounds. Agar plates were incubated at 30°C for 7 days.

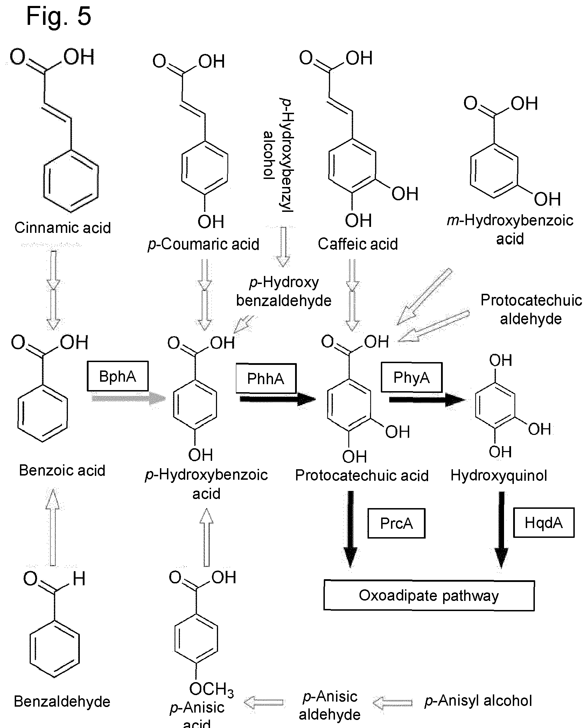

Figure 5 - Benzoic acid metabolic pathway in *A. niger.* Arrows in black are enzymes identified by the inventors. In grey, conversions with enzymes that were previously characterized. In white, conversions observed in *A. niger* with no genes identified.

### Description of the invention

### Uses

The inventors have surprisingly found new enzymes that have utility in metabolic pathways related to benzoic acid. It was also found that varying the production of one or more enzymes participating in the pathway, referred to herein as expression of those enzymes, can lead in a host cell to the useful production of intermediate compounds. Accordingly, the invention relates to enzymes from a cluster related to metabolism of benzoic acid, to host cells comprising these enzymes, and to methods for using the enzymes and cells.
In the context of the invention, the term "production" of a compound refers to biosynthetic production and thus it can refer to production of said compound by a cell and/or a cell lysate and/or a functional enzyme. Production of a compound by a cell can be interpreted to encompass accumulation of said compound within the cell and/or excretion of said compound to the culture medium and/orthe culture headspace. Production of a compound by a cell lysate and/or a functional enzyme can be interpreted to encompass its formation in a reaction medium. Said compound may accumulate in the culture medium and/or reaction medium or be removed from the culture medium and/or reaction medium simultaneously with its formation by means of isolation and/or purification using known techniques in the art.
Accumulation, as used herein, refers to an increase in the levels of the compound at hand where its production exceeds its consumption or deterioration. Preferably, an accumulating compound is produced by a cell, and the excess amount has no further substantial role or no further role in any pathway. This can advantageously be achieved by disruption of one or more enzymes that would normally use the compound as a reaction substrate, or by increasing the activity of one or more enzymes that would normally form the compound as a reaction product, or by influencing the transport of such reaction product..
In a first aspect, the invention provides the use of a functional enzyme with protocatechuate hydroxylase (Phy) activity for the production of hydroxyquinol.
An "enzyme" is a polypeptide that can catalyse a reaction. An enzyme that shows activity in catalysing its associated reaction is a functional enzyme. The functional enzyme may be used in any form including, but not limited to, a crude enzyme preparation, a commercially available enzyme preparation, an enzyme further purified from a commercially available preparation, an enzyme isolated from its source by a combination of known isolation and/or purification methods, an immobilized enzyme, whole (optionally permeabilized and/or immobilized) cells that naturally or through genetic modification express the enzyme, or a cell lysate comprising the enzyme. The functional enzyme may be an isolated, synthetic or recombinant enzyme. The functional enzyme may be present in a suitable composition that allows for optimal enzymatic stability and/or activity. Such compositions are known to the skilled person and preferably further comprise salt buffers and/or antifreezes such as glycerol. In a preferred embodiment, a functional enzyme isolated from its source, more preferably an immobilized enzyme, is used. In another preferred embodiment, whole (optionally permeabilized and/or immobilized) cells that naturally or through genetic modification express the functional enzyme are used.
Means of immobilizing cells and enzymes are known in the art and are discussed in standard handbooks, such as Guisan, J.M., Bolivar, J.M., López-Gallego, F., Rocha-Martin, J. (Eds.), Immobilization of Enzymes and Cells: Methods and Protocols, Springer US, USA, 2020. In an embodiment, the functional enzyme with protocatechuate hydroxylase (Phy) activity is used in an immobilized state with an appropriate carrier. As a non-limiting example, the enzyme may be bound directly to a membrane, granules or a polymer such as a resin having one or more functional groups or it may be bound to the resin through bridging compounds having one or more functional groups such as glutaraldehyde.
Suitable forms include isolated and/or purified naturally occurring enzymes, i.e. as they can be isolated and/or purified from a wild type source organism. Suitable forms may additionally include mutants of naturally occurring enzymes, which can for example be synthetically made or made by modifying the nucleotide sequence encoding for said enzymes using mutagenesis techniques known to the person skilled in the art, such as random mutagenesis, site-directed mutagenesis, directed evolution, gene shuffling, CRISPR/Cas-mediated mutagenesis and the like, so that the resulting nucleotide sequence encodes an enzyme that differs by at least one amino acid from the naturally occurring enzyme. Said suitable enzyme forms are selected to have improved properties with respect to (stereo)selectivity and/or activity and/or stability and/or solvent resistance and/or pH and/or temperature profiles as compared to the naturally occurring enzyme.

In an embodiment, the functional enzyme is isolated and/or purified after its expression by a host cell. The host cell may be naturally expressing said enzyme, or its expression may be introduced by means of genetic engineering as discussed below. As used herein, the term "isolated" means that the material is separated from its original environment. An isolated enzyme polypeptide is a polypeptide that is substantially free of cellular material (when produced by a cell) or chemical precursors or other chemicals (when synthetically made). The enzyme polypeptide may be isolated directly from cultured cells or from the culture medium if it is excreted, preferably they are isolated from cells. A signal sequence may be used to facilitate secretion and isolation, by linking (fusing) it to the enzyme polypeptide of interest using well-known molecular toolbox techniques. Signal sequences are typically characterized by a core of hydrophobic amino acids, which may be cleaved from the mature polypeptide during secretion in one or more cleavage events. Such signal peptides can contain processing sites that allow cleavage of the signal sequence from the mature polypeptide as they pass through the secretory pathway. The signal sequence directs secretion of the polypeptide, such as from a host cell expressing said polypeptide. The polypeptide can then be readily isolated and/or purified from the culture medium. Methods for isolation and/or purification of enzymes are known to the skilled person and are discussed in standard handbooks, such as Sambrook and Russel, Molecular Cloning: A Laboratory Manual, 3rd edition, Cold Spring Harbor Laboratory, Cold Spring Harbor Laboratory Press, NY, 2001 or Ausubel F. et al, eds., Current protocols in molecular biology, Green Publishing and Wiley Interscience, NY, 2003. Examples of isolation and/or purification methods include chromatographic methods such as gel-filtration chromatography, ion-exchange chromatography, immunoaffinity chromatography, metal affinity chromatography, gel-filtration chromatography, fractionation with precipitants such as ammonium sulfate and polyethylene glycol, gel electrophoresis and salting out and dialysis. Preferably, metal affinity chromatography is used. Isolation and/or purification may optionally be enhanced by linking the enzyme polypeptide to a sequence that facilitates purification, such as with a GST domain, using well-known molecular toolbox techniques. In some embodiments, the enzyme polypeptide is linked (fused) to a hexa-histidine peptide, such as the tag provided in a pET23b vector (Genescript Biotech, Leiden, The Netherlands), among others, many of which are commercially available. As, for example, described in Gentz et al., Proc. Natl. Acad. Sci. USA 86:821-824 (1989), hexa-histidine peptide provides for convenient purificaton of the fusion protein.
The term reaction medium should be interpreted to encompass any suitable medium that can enable the enzymatic reaction to take place. Suitable reaction media and conditions conducive to the production of a compound by a cell lysate and/or a functional enzyme of the invention will be known to the skilled person and are discussed in standard handbooks, such as Bisswanger H, Practical Enzymology, 2nd edition, Wiley-VCH Verlag GmbH & Co. KgaA, 2012. Typically, besides the cell lysate and/or the functional enzyme and/or the composition comprising said enzyme, reaction media will comprise the reactants, enzyme co-factors such as metals where applicable, and suitable buffered solutions.
In an embodiment, a cell lysate comprising a functional enzyme with protocatechuate hydroxylase (Phy) activity or an isolated and/or a purified functional enzyme with protocatechuate hydroxylase (Phy) activity and/or a composition comprising a functional enzyme with protocatechuate hydroxylase (Phy) activity, preferably an isolated and/or purified functional enzyme, is used for the production of hydroxyquinol. Said production may occur in a suitable reaction medium. In a preferred embodiment, the reaction medium comprises at least, besides the functional enzyme with protocatechuate hydroxylase activity, NADH, preferably about 1 mM NADH, protocatechuic acid, preferably about 100 µM protocatechuic acid, and Mcllvaine buffer, preferably at pH 7.0. Preferably, said production occurs by incubation at about 30°C, preferably for about 1 hour.

A functional enzyme with protocatechuate hydroxylase activity, which is also known as protocatechuate hydroxylase or protocatechuic acid hydroxylase or salicylate hydroxylase or Phy, is an enzyme (EC 1.14.13.1) which is able to catalyze the decarboxylation of protocatechuic acid to hydroxyquinol. Protocatechuic acid is also known as protocatechuate, 3,4-dihydroxybenzoic acid, or 4-carboxy-1,2-dihydroxybenzene. Hydroxyquinol is also known as 1,2,4-benzenetriol, benzene-1,2,4-triol, 1,2,4-trihydroxybenzene, hydroxyhydroquinone, or 1,3,4-benzenetriol. A skilled person understands that the quinone and hydroquinone can readily interconvert, particularly in biological systems. In preferred embodiments, the enzyme with Phy activity is from a microbial cell, more preferably from a fungal cell, even more preferably a filamentous fungal cell. In most preferred embodiments, the enzyme with Phy activity is PhyA from *Aspergillus niger* (SEQ ID NO: 1) or has high analogy thereto as defined later herein.
A functional enzyme with protocatechuate 3,4-dioxygenase activity, which is also known as protocatechuate 3,4-dioxygenase or protocatechuic acid 3,4-dioxygenase or Prc, is an enzyme (EC 1.13.11.3) which catalyzes the conversion of protocatechuic acid to 3-carboxy-cis,cis-muconic acid by ring-cleavage. 3-carboxy-cis,cis-muconic acid is also known as 3-carboxy-cis,cis-muconate, beta-carboxy-cis,cis-muconate, beta-carboxy-cis-cis-muconate, cis,cis-butadiene-1,2,4-tricarboxylate or cis-cis-butadiene-1,2,4-tricarboxylate. In preferred embodiments, the enzyme with Prc activity is from a microbial cell, more preferably from a fungal cell, even more preferably a filamentous fungal cell. In most preferred embodiments, the enzyme with Prc activity is PrcA from *Aspergillus niger* (SEQ ID NO: 4) or has high analogy thereto as defined later.
A functional enzyme with hydroxyquinol 1,2-dioxygenase activity, which is also known as hydroxyquinol 1,2-dioxygenase or Hqd, is an enzyme (EC 1.13.11.37) which catalyzes the conversion of hydroxyquinol to maleylacetic acid by ring-cleavage. Maleylacetic acid is also known as maleylacetate, (2Z)-4-oxohex-2-enedioic acid, 2-maleylacetate or 4-oxohex-2-enedioate. In preferred embodiments, the enzyme with Hqd activity is from a microbial cell, more preferably from a fungal cell, even more preferably a filamentous fungal cell. In most preferred embodiments, the enzyme with Hqd activity is HqdA from *Aspergillus niger,* preferably HqdA (SEQ ID NO: 8) or has high analogy thereto as defined later.
Enzyme activity can be determined by monitoring the decrease of reactant concentration or the increase of product concentration using chromatographic techniques known to the skilled person, such as gas chromatography (GC), high performance liquid chromatography (HPLC), or GC or liquid chromatography with advantageously coupled mass spectrometric methods, such as LC-MS or GC-MS. A preferred method for the detection and/or quantification of benzoic-acid related compounds such as, but not limited to, protocatechuic acid and hydroxyquinol is HPLC. As an example, chromatographic separation may be carried out in Dionex ICS-5000+ chromatography system, equipped with an Acclaim MixedMode WAX-1 LC Column (3×150 mm) and a UV detector (225, 250 or 280 nm), said system being supplied by Thermo Scientific, with the chromatographic separation being carried out according to manufacturer's recommendation using isocratic elution (30°C; flow rate: 0.25 mL/min) with 25 mM potassium monophosphate, 0.8 mM pyrophosphate, pH 6.0, in 50% acetonitrile, as described in Dilokpimol et al. 2017.
Enzyme activity may be determined in vitro or in vivo by measuring the respective catalysed reaction rates using culture broths, whole cells, cell-free growth media supernatants, cell lysates, cell-free extracts, isolated and/or purified, optionally immobilized, enzymes or compositions comprising said enzymes. Preferably, enzyme activity is determined using culture broths, more preferably cell lysates and most preferably purified enzymes. Methods for the preparation and execution of said measurements are known to in the art and are discussed, for example, in Bisswanger H, Practical Enzymology, 2nd edition, Wiley-VCH Verlag GmbH & Co. KgaA, 2012.

In an embodiment, the use of a functional enzyme with protocatechuate hydroxylase activity from *Aspergillus niger* (PhyA), preferably from *Aspergillus niger* CBS 120.49 or a derivative thereof, for the production of hydroxyquinol is provided. Suitable forms of the functional enzyme are preferably the ones described elsewhere herein.
As used herein, a derivative of a host cell has its conventional meaning used in the art, and refers to an engineered version of that host cell, or to a host cell that has otherwise been mutated as compared to the named host cell. For example, derivatives of *Aspergillus niger* CBS 120.49 are *Aspergillus niger* CBS 138852 which is *ΔkusA,* or CBS145984 which has a repaired pyrG.
In an embodiment, the use of a functional enzyme with protocatechuate 3,4-dioxygenase activity from *Aspergillus niger* (PrcA), preferably from *Aspergillus niger* CBS 120.49 or a derivative thereof, for the production of 3-carboxy-cis,cis-muconic acid is provided. Suitable forms of the functional enzyme are preferably the ones described elsewhere herein.
In an embodiment, the use of a functional enzyme with hydroxyquinol 1,2-dioxygenase activity from *Aspergillus niger* (HqdA), preferably from *Aspergillus niger* CBS 120.49 or a derivative thereof, for the production of maleylacetic acid is provided. Suitable forms of the functional enzyme are preferably the ones described elsewhere herein.

In preferred embodiments, the functional enzyme with Phy activity has at least 60%, 65%, 70%, 75%, 80%, 85%, 90%, 91%, 92%, 93%, 94%, 95%, 96%, 97%, 98%, 99%, or 100%, preferably at least 80%, 85%, 90%, 91%, 92%, 93%, 94%, 95%, 96%, 97%, 98%, 99%, or 100%, more preferably at least 80%, even more preferably at least 90%, most preferably 100% sequence identity with SEQ ID NO:1, or is encoded by a nucleotide sequence which has at least 60%, 65%, 70%, 75%, 80%, 85%, 90%, 91%, 92%, 93%, 94%, 95%, 96%, 97%, 98%, 99%, or 100%, preferably at least 80%, 85%, 90%, 91%, 92%, 93%, 94%, 95%, 96%, 97%, 98%, 99%, or 100%, more preferably at least 80%, even more preferably at least 90%, most preferably 100%, sequence identity with SEQ ID NO: 2. In other preferred embodiments, the functional enzyme with Phy activity has at least 90% sequence identity with SEQ ID NO:1 or is encoded by a nucleotide sequence which has at least 90% sequence identity with SEQ ID NO: 2. In still preferred embodiments, the functional enzyme with Phy activity has at least 95% sequence identity with SEQ ID NO:1 or is encoded by a nucleotide sequence which has at least 95% sequence identity with SEQ ID NO: 2. In more preferred embodiments, the functional enzyme with Phy activity has at least 96% sequence identity with SEQ ID NO:1 or is encoded by a nucleotide sequence which has at least 96% sequence identity with SEQ ID NO: 2. In still more preferred embodiments, the functional enzyme with Phy activity has at least 97% sequence identity with SEQ ID NO:1 or is encoded by a nucleotide sequence which has at least 97% sequence identity with SEQ ID NO: 2. In still more preferred embodiments, the functional enzyme with Phy activity has at least 98% sequence identity with SEQ ID NO:1 or is encoded by a nucleotide sequence which has at least 98% sequence identity with SEQ ID NO: 2. In even more preferred embodiments, the functional enzyme with Phy activity has at least 99% sequence identity with SEQ ID NO:1 or is encoded by a nucleotide sequence which has at least 99% sequence identity with SEQ ID NO: 2. In most preferred embodiments, the functional enzyme with Phy activity has 100% sequence identity with SEQ ID NO:1 or is encoded by a nucleotide sequence which has 100% sequence identity with SEQ ID NO: 2.

In preferred embodiments, the functional enzyme with Prc activity has at least 60%, 65%, 70%, 75%, 80%, 85%, 90%, 91%, 92%, 93%, 94%, 95%, 96%, 97%, 98%, 99%, or 100%, preferably at least 80%, 85%, 90%, 91%, 92%, 93%, 94%, 95%, 96%, 97%, 98%, 99%, or 100%, more preferably at least 80%, even more preferably at least 90%, most preferably 100% sequence identity with SEQ ID NO:4, or is encoded by a nucleotide sequence which has at least 60%, 65%, 70%, 75%, 80%, 85%, 90%, 91%, 92%, 93%, 94%, 95%, 96%, 97%, 98%, 99%, or 100%, preferably at least 80%, 85%, 90%, 91%, 92%, 93%, 94%, 95%, 96%, 97%, 98%, 99%, or 100%, more preferably at least 80%, even more preferably at least 90%, most preferably 100%, sequence identity with SEQ ID NO: 5. In other preferred embodiments, the functional enzyme with Prc activity has at least 90% sequence identity with SEQ ID NO:4 or is encoded by a nucleotide sequence which has at least 90% sequence identity with SEQ ID NO: 5. In still preferred embodiments, the functional enzyme with Prc activity has at least 95% sequence identity with SEQ ID NO:4 or is encoded by a nucleotide sequence which has at least 95% sequence identity with SEQ ID NO: 5. In more preferred embodiments, the functional enzyme with Prc activity has at least 96% sequence identity with SEQ ID NO:4 or is encoded by a nucleotide sequence which has at least 96% sequence identity with SEQ ID NO: 5. In still more preferred embodiments, the functional enzyme with Prc activity has at least 97% sequence identity with SEQ ID NO:4 or is encoded by a nucleotide sequence which has at least 97% sequence identity with SEQ ID NO: 5. In still more preferred embodiments, the functional enzyme with Prc activity has at least 98% sequence identity with SEQ ID NO:4 or is encoded by a nucleotide sequence which has at least 98% sequence identity with SEQ ID NO: 5. In even more preferred embodiments, the functional enzyme with Prc activity has at least 99% sequence identity with SEQ ID NO:4 or is encoded by a nucleotide sequence which has at least 99% sequence identity with SEQ ID NO: 5. In most preferred embodiments, the functional enzyme with Prc activity has 100% sequence identity with SEQ ID NO:4 or is encoded by a nucleotide sequence which has 100% sequence identity with SEQ ID NO: 5.

In preferred embodiments, the functional enzyme with Hqd activity has at least 60%, 65%, 70%, 75%, 80%, 85%, 90%, 91%, 92%, 93%, 94%, 95%, 96%, 97%, 98%, 99%, or 100%, preferably at least 80%, 85%, 90%, 91%, 92%, 93%, 94%, 95%, 96%, 97%, 98%, 99%, or 100%, more preferably at least 80%, even more preferably at least 90%, most preferably 100% sequence identity with SEQ ID NO:7, or is encoded by a nucleotide sequence which has at least 60%, 65%, 70%, 75%, 80%, 85%, 90%, 91%, 92%, 93%, 94%, 95%, 96%, 97%, 98%, 99%, or 100%, preferably at least 80%, 85%, 90%, 91%, 92%, 93%, 94%, 95%, 96%, 97%, 98%, 99%, or 100%, more preferably at least 80%, even more preferably at least 90%, most preferably 100%, sequence identity with SEQ ID NO: 8. In other preferred embodiments, the functional enzyme with Hqd activity has at least 90% sequence identity with SEQ ID NO:7 or is encoded by a nucleotide sequence which has at least 90% sequence identity with SEQ ID NO: 8. In still preferred embodiments, the functional enzyme with Hqd activity has at least 95% sequence identity with SEQ ID NO:7 or is encoded by a nucleotide sequence which has at least 95% sequence identity with SEQ ID NO: 8. In more preferred embodiments, the functional enzyme with Hqd activity has at least 96% sequence identity with SEQ ID NO:7 or is encoded by a nucleotide sequence which has at least 96% sequence identity with SEQ ID NO: 8. In still more preferred embodiments, the functional enzyme with Hqd activity has at least 97% sequence identity with SEQ ID NO:7 or is encoded by a nucleotide sequence which has at least 97% sequence identity with SEQ ID NO: 8. In still more preferred embodiments, the functional enzyme with Hqd activity has at least 98% sequence identity with SEQ ID NO:7 or is encoded by a nucleotide sequence which has at least 98% sequence identity with SEQ ID NO: 8. In even more preferred embodiments, the functional enzyme with Hqd activity has at least 99% sequence identity with SEQ ID NO:7 or is encoded by a nucleotide sequence which has at least 99% sequence identity with SEQ ID NO: 8. In most preferred embodiments, the functional enzyme with Hqd activity has 100% sequence identity with SEQ ID NO:7 or is encoded by a nucleotide sequence which has 100% sequence identity with SEQ ID NO: 8.
The nucleotide sequence encoding for the functional enzymes according the invention may be codon optimized. A codon optimized nucleotide sequence is a sequence wherein the codon usage bias has been mitigated through selection of alternative codons without altering the encoded polypeptide. For example, rare codons can be replaced by more common codons, or regions comprising many identical codons can be interrupted by substitution of synonymic codons to reduce demand for the many identical codons. Codon optimization is known in the art, and bioinformatics tools for codon optimization are freely available on the internet from various providers. Accordingly, in some embodiments, the functional enzyme with Phy activity is encoded by a nucleotide sequence which has at least 60%, 65%, 70%, 75%, 80%, 85%, 90%, 91%, 92%, 93%, 94%, 95%, 96%, 97%, 98%, 99%, or 100%, preferably at least 80%, 85%, 90%, 91%, 92%, 93%, 94%, 95%, 96%, 97%, 98%, 99%, or 100%, more preferably at least 80%, even more preferably at least 90%, most preferably 100% sequence identity with SEQ ID NO: 10. In preferred embodiments, the functional enzyme with Phy activity is encoded by a nucleotide sequence which has at least 90% sequence identity with SEQ ID NO: 10. In still preferred embodiments, the functional enzyme with Phy activity is encoded by a nucleotide sequence which has at least 95% sequence identity with SEQ ID NO: 10. In more preferred embodiments, the functional enzyme with Phy activity is encoded by a nucleotide sequence which has at least 96% sequence identity with SEQ ID NO: 10. In still more preferred embodiments, the functional enzyme with Phy activity is encoded by a nucleotide sequence which has at least 97% sequence identity with SEQ ID NO: 10. In still preferred embodiments, the functional enzyme with Phy activity is encoded by a nucleotide sequence which has at least 98% sequence identity with SEQ ID NO: 10. In even more preferred embodiments, the functional enzyme with Phy activity is encoded by a nucleotide sequence which has at least 99% sequence identity with SEQ ID NO: 10. In most preferred embodiments, it is encoded by a nucleotide sequence which has 100% sequence identity with SEQ ID NO: 10.
In some embodiments, the functional enzyme with Prc activity is encoded by a nucleotide sequence which has at least 60%, 65%, 70%, 75%, 80%, 85%, 90%, 91%, 92%, 93%, 94%, 95%, 96%, 97%, 98%, 99%, or 100%, preferably at least 80%, 85%, 90%, 91%, 92%, 93%, 94%, 95%, 96%, 97%, 98%, 99%, or 100%, more preferably at least 80%, even more preferably at least 90%, most preferably 100% sequence identity with SEQ ID NO:11. In preferred embodiments, the functional enzyme with Prc activity is encoded by a nucleotide sequence which has at least 90% sequence identity with SEQ ID NO: 11. In still preferred embodiments, the functional enzyme with Prc activity is encoded by a nucleotide sequence which has at least 95% sequence identity with SEQ ID NO: 11. In more preferred embodiments, the functional enzyme with Prc activity is encoded by a nucleotide sequence which has at least 96% sequence identity with SEQ ID NO: 11. In still more preferred embodiments, the functional enzyme with Prc activity is encoded by a nucleotide sequence which has at least 97% sequence identity with SEQ ID NO: 11. In still preferred embodiments, the functional enzyme with Prc activity is encoded by a nucleotide sequence which has at least 98% sequence identity with SEQ ID NO: 11. In even more preferred embodiments, the functional enzyme with Prc activity is encoded by a nucleotide sequence which has at least 99% sequence identity with SEQ ID NO: 11. In most preferred embodiments, it is encoded by a nucleotide sequence which has 100% sequence identity with SEQ ID NO: 11.
In some embodiments, the functional enzyme with Hqd activity is encoded by a nucleotide sequence which has at least 60%, 65%, 70%, 75%, 80%, 85%, 90%, 91%, 92%, 93%, 94%, 95%, 96%, 97%, 98%, 99%, or 100%, preferably at least 80%, 85%, 90%, 91%, 92%, 93%, 94%, 95%, 96%, 97%, 98%, 99%, or 100%, more preferably at least 80%, even more preferably at least 90%, most preferably 100% sequence identity with SEQ ID NO:12. In preferred embodiments, the functional enzyme with Hqd activity is encoded by a nucleotide sequence which has at least 90% sequence identity with SEQ ID NO: 12. In still preferred embodiments, the functional enzyme with Hqd activity is encoded by a nucleotide sequence which has at least 95% sequence identity with SEQ ID NO: 12. In more preferred embodiments, the functional enzyme with Hqd activity is encoded by a nucleotide sequence which has at least 96% sequence identity with SEQ ID NO: 12. In still more preferred embodiments, the functional enzyme with Hqd activity is encoded by a nucleotide sequence which has at least 97% sequence identity with SEQ ID NO: 12. In still preferred embodiments, the functional enzyme with Hqd activity is encoded by a nucleotide sequence which has at least 98% sequence identity with SEQ ID NO: 12. In even more preferred embodiments, the functional enzyme with Hqd activity is encoded by a nucleotide sequence which has at least 99% sequence identity with SEQ ID NO: 12. In most preferred embodiments, it is encoded by a nucleotide sequence which has 100% sequence identity with SEQ ID NO: 12.

### Host cells

Throughout this document, the terms "host cell" and "cell" are used interchangeably and can be interpreted to encompass any cell capable of expressing, preferably expressing, at least one of the functional enzymes according to the invention. Said capability can be naturally present in the cell or be introduced to it via means of genetic engineering or modification. The terms may also be interpreted to encompass any genetically modified cell that has reduced expression of a functional enzyme according to the invention, or in which said expression has been eliminated, as compared to a corresponding wild type cell lacking the genetic modification. The term "wild type" refers to a cell as it occurs in nature.
The expressed functional enzyme may be homologous or heterologous to the host cell. A homologous enzyme is a polypeptide that is native, or encoded by a nucleotide sequence that is native, i.e. naturally present in the same organism, as the host cell. A heterologous enzyme is a polypeptide that is derived from, or encoded by a nucleotide sequence that is derived from, a different organism to the host cell. A heterologous enzyme may also be an enzyme encoded by a recombinant nucleotide sequence as discussed below. Molecular toolbox techniques to achieve and/or enhance and/or decrease homologous or heterologous expression of a functional enzyme by a host cell are well-known in the art and are further discussed below.
Suitable host cells that can be used according to the invention are well-known in the art. Suitable host cells can be mammalian, insect, plant, or microbial cells. Examples of mammalian cells include Chinese hamster ovary (CHO) cells, COS cells, 293 cells, PerC6^{™} cells and hybridomas. Examples of insect cells include Sf9 and Sf21 cells and derivatives thereof. In some embodiments, the host cell is a microbial cell. The microbial host cell can be prokaryotic or eukaryotic. A microbial prokaryote is a unicellular organism that lacks a membrane-bound nucleus, mitochondria, and other membrane-bound organelles. Examples of microbial prokaryotes include bacteria and archaea, of which bacteria are preferred. Bacterial host cells include both Gram-negative and Gram-positive organisms and can be selected from suitable groups known in the art such as the group of *Absidia, Achromobacter, Acinetobacter, Aeribacillus, Aneurinibacillus, Agrobacterium, Aeromonas, Alcaligenes, Arthrobacter, Arzoarcus, Azomonas, Azospirillum, Azotobacter, Bacillus, Beijerinckia, Bradyrhizobium, Brevibacills, Burkholderia, Byssochlamys, Citrobacter, Clostridium, Comamonas, Cupriavidus, Corynebacterium, Deinococcus, Escherichia, Enterobacter, Flavobacterium, Fusobacterium, Gossypium, Klebsiella, Lactobacillus, Listeria, Megasphaera, Micrococcus, Mycobacterium, Norcadia, Porphyromonas, Propionibacterium, Pseudomonas, Ralstonia, Rhizobium, Rhodopseudomonas, Rhodospirillum, Rodococcus, Roseburia, Shewanella, Streptomycetes, Xanthomonas, Xylella, Yersinia, Treponema, Vibrio, Streptococcus, Lactococcus, Zymomonas, Staphylococcus, Salmonella, Sphingomonas, Sphingobium, Novosphingobium, Brucella* and *Microscilla.* Preferably, the bacterial cell is selected from a species from the group consisting of *Aeribacillus pallidus, Aneurinibacillus terranovensis, Bacillus subtilis, Bacillus amyloliquefaciens, Bacillus coagulans, B. Irribbensis, Bacillus licheniformis, B. puntis, Bacillus megaterium, Bacillus halodurans, Bacillus pumilus, Brevibacillus thermoruber, Brevibacillus panacihumi, Cupriavidus basilensis, D. Iruznetsovii, D. thermophila, G. Iraustophilus, Gluconobacter oxydans, Caulobacter crescentus CB 15, Methylobacterium extorquens, Rhodobacter sphaeroides, Pelotomaculum thermopropionicum, Pseudomonas zeaxanthinifaciens, Pseudomonas putida, Paracoccus denitrificans, Escherichia coli, Corynebacterium glutamicum, Staphylococcus carnosus, Streptomyces lividans, Sinorhizobium melioti, Sphingobium sp., Novosphingobium sp., Sphingomonas henshuiensis,* and *Rhizobium radiobacter.* Within the species *Pseudomonas putida,* strains S12 and KT2440 are preferred. Within the species *Sphingobium sp.,* strains SYK-6 and 66-54 are preferred. Within the species *Sphingomonas hengshuiensis,* strain WHSC-8 is preferred. Within the species *Novosphingobium sp.,* strain AAP93 is preferred. A microbial eukaryote is a unicellular or multicellular organism which has a nucleus enclosed within a nuclear envelope and contains membrane-bound organelles such as mitochondria and a Golgi apparatus. Examples of suitable microbial eukaryotes are protozoa, algae and fungi. In preferred embodiments, the host cell is a fungal cell. Particularly when compared to bacteria, fungi, have many attractive features for industrial fermentation processes, including e.g. their high tolerance to acids, ethanol and other harmful compounds, their high osmo-tolerance, their high fermentative capacity and for some yeasts their capability of anaerobic growth. Fungal host cells can be selected from suitable groups known in the art such as yeasts of the group *Saccharomyces, Kluyveromyces, Candida, Pichia, Schizosaccharomyces, Hansenula, Kloeckera, Schwanniomyces, Yarrowia, Cryptococcus, Debaromyces, Saccharomycecopsis, Saccharomycodes, Wickerhamia, Debayomyces, Hanseniaspora, Ogataea, Kuraishia, Komagataella, Metschnikowia, Williopsis, Nakazawaea, Torulaspora, Bullera, Rhodotorula, Sporobolomyces* and filamentous fungi. Within yeasts, the species *Kluyveromyces lactis, Saccharomyces cerevisiae, Hansenula polymorpha* (also known as *Ogataea henricii*), *Yarrowia lipolytica, Candida tropicalis* and *Pichia pastoris* (also known as *Komagataella pastoris*) are preferred. In more preferred embodiments, the host cell is a filamentous fungal cell. A filamentous fungal cell according to the invention includes all members of the subdivisions Eumycota and Oomycota (preferably as defined by Hawksworth et al., In, Ainsworth and Bisby's Dictionary of The Fungi, 8th edition, CAB International, University Press, Cambridge, UK, 1995). Filamentous fungi are characterized by a mycelial wall composed of chitin, cellulose, glucan, chitosan, mannan, and other complex polysaccharides. Vegetative growth is by hyphal elongation and carbon catabolism is obligatorily aerobic. Filamentous fungal host cells can be selected from suitable groups known in the art such as *Acremonium, Agaricus, Aspergillus, Aureobasidium, Chrysosporium, Coprinus, Cryptococcus, Filibasidium, Fusarium, Humicola, Magnaporthe, Mucor, Myceliophthora, Neocallinastix, Neurospora, Paecilomyces, Penicillium, Piromyces, Panerochaete, Pleurotus, Schizophyllum, Talaromyces, Thermoascus, Thielavia, Tolypocladium, Ustilago and Trichoderma.* Within filamentous fungi, the species *Aspergillus niger, Aspergillus nidulans, Aspergillus fumigatus, Aspergillus oryzae,, Aspergillus vadensis, Penicillium chrysogenum, Penicillium citrinum, Penicillium rubens, Penicillium oxalicum, Penicillium subrubescens, Rasamsonia emersonii, Talaromyces emersonii, Acremonium chrysogenum, Trichoderma reesei, Aspergillus sojae,* and *Chrysosporium lucknowense* are preferred. Several strains belonging to the these groups are readily accessible to the public in a number of well-known collections, such as the American Type Culture Collection (ATCC), Deutsche Sammlung von Mikroorganismen and Zellkulturen GmbH (DSM), Centraalbureau Voor Schimmelcultures (CBS), and Agricultural Research Service Patent Culture Collection, Northern Regional Research Center (NRRL). Examples of such strains include *Aspergillus niger* CBS 513.88, N593, CBS 120.49, N402, ATCC 1015 *Aspergillus oryzae* ATCC 20423, IFO 4177, ATCC 1011, ATCC 9576, ATCC 14488-14491, ATCC 11601, ATCC12892, *Aspergillus vadensis* CBS 113365, CBS 102787, IMI 142717, IBT 24658, CBS 113226, *Penicillium chrysogenum* CBS 455.95, *Penicillium citrinum* ATCC 38065, *Penicillium chrysogenum* P2, Wisconsin 54-1255, *Penicillium subrubescens* CBS 132785, FBCC 1632, *Talaromyces emersonii* CBS 393.64, *Acremonium chrysogenum* ATCC 36225 or ATCC 48272, *Trichoderma reesei* ATCC 26921 or ATCC56765 or ATCC 26921, *Aspergillus sojae* ATCC11906, *Chrysosporium lucknowense* ATCC44006 and derivatives thereof. In even more preferred embodiments, the host cell is a cell selected from the group of *Aspergillus.* In most preferred embodiments, the host cell is an *Aspergillus niger* cell, preferably *Aspergillus niger* strain 120.49 or a derivative thereof. Preferably, the host cell has a high tolerance to low pH, i.e. capable of growth at a pH equal to or lower than 5.0, 4.0, 3.0, 2.9, 2.8, 2.7, 2.6, 2.5 or 2.4, a high tolerance to organic acids and/or a high tolerance to elevated temperatures.

Throughout this document, the term "expression" by a host cell can be interpreted to encompass any step starting from transcription of a gene into an mRNA transcript, leading to a polypeptide such as an enzyme. Accordingly, in the context of the invention, expression of a functional enzyme by the host cell can be considered to include any step involved in the production of said enzyme including, but not limited to, transcription, post-transcriptional modification, translation, post-translational modification, and secretion. Any of these steps may be naturally present in the host cell or may be introduced or modified via means of genetic engineering or modification as discussed below.
Increased expression of a functional enzyme by a host cell includes embodiments wherein any of the abovementioned steps involved in the production of said enzyme are increased as compared to a corresponding wild type cell that has not been genetically modified, resulting in increased levels of enzyme transcript, enzyme polypeptide, and/or enzyme activity compared to said non-modified cell. Preferably, the levels of enzyme transcript, enzyme polypeptide, and/or enzyme activity in the host cell are increased by at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, more preferably at least 30%, even more preferably at least 40%, most preferably at least 50%, as compared to a corresponding wild type cell lacking the genetic modification.
Reduced expression of a functional enzyme by a host cell refers to embodiments wherein any of the abovementioned steps involved in the production of said enzyme are reduced as compared to a corresponding wild type cell that has not been genetically modified, resulting in decreased levels of enzyme transcript, enzyme polypeptide, and/or enzyme activity compared to said non-modified cell. Preferably, the levels of enzyme transcript, enzyme polypeptide, and/or enzyme activity in the host cell are decreased by at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, more preferably at least 30%, even more preferably at least 40%, most preferably at least 50%, as compared to a corresponding wild type cell lacking the genetic modification. Reduced expression can also be a reduction of 100%, which may mean that no expression can be detected. Most preferably a reduction of expression is a reduction by 100%, wherein the absence of detectable enzymatic activity is the most preferred result of said reduction.
The differences in levels of enzyme transcript, enzyme polypeptide and/or enzyme activity between cells can be measured by the skilled person using standard techniques in the art, as described in handbooks such as Sambrook and Russel, Molecular Cloning: A Laboratory Manual, 3rd edition, Cold Spring Harbor Laboratory, Cold Spring Harbor Laboratory Press, NY, 20011, Ausubel F. et al, eds., Current protocols in molecular biology, Green Publishing and Wiley Interscience, NY, 2003, and Bisswanger H, Practical Enzymology, 2nd edition, Wiley-VCH Verlag GmbH & Co. KgaA, 2012. Examples of methods of assessing differences in enzyme transcript levels include quantitative PCR and RNA sequencing. Examples of methods of assessing differences in enzyme polypeptide levels include SDS-PAGE and quantitative proteomics. Examples of methods of assessing differences in enzyme activity levels include enzymatic assays performed on whole cells and/or cell lysates.
Elimination of expression of a functional enzyme refers to embodiments wherein the activity of the functional enzyme is eliminated which can be the result of a disruption of any of the abovementioned steps involved in the production of said enzyme by the host cell. This means that embodiments wherein the enzyme transcript and/or enzyme polypeptide are still present should not be excluded, as long as the activity of the functional enzyme is eliminated. As an example, an enzyme polypeptide might require a post-translational modification in order to be converted to a functional enzyme and the host cell might lack the capability of performing this post-translational modification; said cell will have no expression of a functional enzyme even though the enzyme polypeptide is present.
In the context of this invention, a cell capable of expressing a functional enzyme is a cell that comprises the genetic information required for expressing said enzyme, preferably a nucleic acid encoding said enzyme. This does not mean that the enzyme is necessarily expressed. As an example, a cell may comprise a nucleic acid encoding an enzyme wherein the nucleic acid is under the control of a promoter that responds to a particular induction; the promoter is not necessarily induced and thus the enzyme is not necessarily expressed, whereas the cell is in fact capable of such expression. In preferred embodiments, a cell capable of expressing a functional enzyme is a cell that expresses said functional enzyme. Optionally, it is a cell that a skilled person can routinely induce to commence such expression.
In the context of the invention, a host cell that has reduced expression of a functional enzyme, or in which said expression has been eliminated, may or may not comprise the genetic information required for expressing said enzyme. The skilled person is aware of methods that result in decreased expression of genes without altering the gene sequence per se. As an example, RNA interference can be used (for a review see Wilson and Doudna, 2013, Ann Rev Biophys 42:217-239). In another example, the host cell may have reduced or no expression of a transcription factor that is necessary for the expression of the gene encoding for the relevant enzyme as compared to a corresponding non-modified cell. In another example, the gene encoding for the enzyme polypeptide is deleted or inactivated.

Accordingly, in a second aspect, the invention provides a host cell capable of expressing, preferably expressing, a functional enzyme according to the invention. Further, this aspect provides a host cell wherein the expression of a functional enzyme according to the invention has been increased, reduced, or eliminated via means of genetic modification as compared to a corresponding wild type cell lacking the genetic modification.
In an embodiment, a host cell comprising a genetic modification that reduces or eliminates the expression of a functional enzyme with protocatechuic hydroxylase (Phy) activity as defined herein, as compared to a corresponding wild type cell lacking the genetic modification is provided.
In an embodiment, a host cell comprising a genetic modification that increases the expression of a functional enzyme with protocatechuic hydroxylase (Phy) activity as defined herein, as compared to a corresponding wild type cell lacking the genetic modification is provided.
In an embodiment, a host cell comprising a genetic modification that reduces or eliminates the expression of a functional enzyme with protocatechuate 3,4-dioxygenase (Prc) activity as defined herein, as compared to a corresponding wild type cell lacking the genetic modification is provided. In an embodiment, a host cell comprising a genetic modification that increases the expression of a functional enzyme with protocatechuate 3,4-dioxygenase (Prc) activity as defined herein, as compared to a corresponding wild type cell lacking the genetic modification is provided.
In another embodiment, a host cell comprising a genetic modification that reduces or eliminates the expression of a functional enzyme with hydroxyquinol 1,2-dioxygenase (Hqd) activity as defined herein, as compared to a corresponding wild type cell lacking the genetic modification is provided. In an embodiment, a host cell comprising a genetic modification that increases the expression of a functional enzyme with hydroxyquinol 1,2-dioxygenase (Hqd) activity as defined herein, as compared to a corresponding wild type cell lacking the genetic modification is provided.

In an embodiment, a host cell comprising a genetic modification that reduces or eliminates the expression of a functional enzyme with protocatechuic hydroxylase (Phy) activity and a further genetic modification that reduces or eliminates the expression of a functional enzyme with protocatechuate 3,4-dioxygenase (Prc) activity as defined herein, as compared to a corresponding wild type cell lacking the genetic modification, wherein the functional enzyme with protocatechuate 3,4-dioxygenase (Prc) activity has at least 60%, preferably at least 80%, more preferably at least 90%, most preferably 100% sequence identity with SEQ ID NO: 4, or is encoded by a polynucleotide which has at least 60%, preferably at least 80%, more preferably at least 90%, most preferably 100% sequence identity with SEQ ID NO: 5, is provided. Preferably, the functional enzyme with protocatechuate 3,4-dioxygenase (Prc) activity is PrcA from *Aspergillus niger,* more preferably from *Aspergillus niger* CBS 120.49 or a derivative thereof.

In an embodiment, a host cell comprising a genetic modification that reduces or eliminates the expression of a functional enzyme with protocatechuate 3,4-dioxygenase (Prc) and a further genetic modification that increases the expression of a functional enzyme with protocatechuic hydroxylase (Phy) activity as defined herein, as compared to a corresponding wild type cell lacking the genetic modifications is provided. Optionally and preferably, said host cell further comprises a genetic modification that reduces or eliminates the expression of a functional enzyme with hydroxyquinol 1,2-dioxygenase (Hqd) activity as defined herein, as compared to a corresponding wild type cell lacking the genetic modification.

In an embodiment, a host cell comprising a genetic modification that reduces or eliminates the expression of a functional enzyme with protocatechuic hydroxylase (Phy) activity and a further genetic modification that increases the expression of a functional enzyme with protocatechuate 3,4-dioxygenase (Prc) activity as defined herein, as compared to a corresponding wild type cell lacking the genetic modifications is provided.
In an embodiment, a host cell comprising a genetic modification that increases the expression of a functional enzyme with protocatechuic hydroxylase (Phy) activity and a further genetic modification that increases the expression of an enzyme with hydroxyquinol 1,2-dioxygenase (Hqd) activity as defined herein, as compared to a corresponding wild type cell lacking the genetic modifications is provided.

The host cells according to the invention have advantageous properties for the production of compounds involved in benzoic acid-related pathways including triple-substituted benzenes such as protocatechuic acid and hydroxyquinol. In preferred embodiments, the host cells produce protocatechuic acid, more preferably accumulate protocatechuic acid, most preferably accumulate protocatechuic acid at levels higher than wild type host cells. In other preferred embodiments, the host cells produce hydroxyquinol, more preferably accumulate hydroxyquinol, most preferably accumulate hydroxyquinol at levels higher than wild type host cells.
A cell according to the invention is able to produce said compounds when cultured as further discussed below. Preferably, the cell is cultured under oxygen-limited or aerobic conditions, more preferably under aerobic conditions. The produced compound may accumulate within the cell and/or be excreted to the culture medium and/or culture headspace. When the compound is excreted to the culture medium, it may accumulate in the medium or be isolated and/or purified from the medium during cultivation using known techniques in the art. In some embodiments, a triple-substituted benzene, preferably selected from protocatechuic acid and hydroxyquinol, more preferably protocatechuic acid, accumulates within the cell. In preferred embodiments, a triple-substituted benzene, preferably selected from protocatechuic acid and hydroxyquinol, more preferably protocatechuic acid, is excreted to the culture medium, preferably accumulates in the culture medium.
Preferred cells according to the invention are capable of producing, preferably producing, more preferably accumulating, even more preferably excreting to the culture medium at least 0.8, 1.0, 1.5, 2.0, 3.0, 3.5, 4.0, 4.5, 5.0, 5.5, 6.0, 6.5, 7.0, 7.5, 8.0, 8.5, 9.0, 9.5, 10, 11, 12, 13, 14, 15, 16, 17, 18, 19, 20, 25, 30, 35, 40, 45, 50, 55, 60, 65, 70, 75, 80, 85, 90, 95, or 100 mM of a triple-substituted benzene, preferably at least 0.8 mM, more preferably at least 1.5 mM, even more preferably at least 2 mM.
More preferred cells according to the invention are capable of producing, preferably producing, more preferably accumulating, even more preferably excreting to the culture medium at least 150 mM, 200 mM, 250 mM, 300 mM, 350 mM, 400 mM, 450 mM, 500 mM, 550 mM, 600 mM, 650 mM, 700 mM, 750 mM, 800 mM, 850 mM, 900 mM, 950 mM, or 1000 mM of a triple-substituted benzene. Suitable substrates to be converted into the relevant compounds by the enzymes, host cells and/or lysates of host cells of the invention will be known to the skilled person and are further discussed below. In cell cultures, the substrate can be any suitable carbon source that can be used in the growth medium. The substrate may be the same carbon source as the one used for the growth of the cell and/or may be a separate carbon source which is additionally present in the growth medium, preferably it is the same carbon source. The skilled person understands that suitable substrates may be added externally to the growth medium, or they may already be present in said media. Said substrates may be added or may be already present in a mixture. Highly preferred carbon sources comprise hexoses, or a mixture of hexoses and pentoses.
In some embodiments, the growth medium comprises lignocellulosic material, also known as second-generation feedstock. Such material includes any lignocellulose and/or hemicellulose-based materials. Such material may be sourced from agricultural, industrial or municipal, preferably agricultural, waste streams. Examples of suitable materials include, but are not limited to, (agricultural) biomass, commercial organic matter, building and demolition waste, municipal solid waste, virgin biomass such as waste paper and garden waste, or non-virgin biomass. General forms of biomass include trees, shrubs and pastures, wheat, wheat straw, sugarcane bagasse, switchgrass, Japanese pampas grass, corn, corn stover, corn cob, canola stalk, soybean stalk, sweet corn, corn kernels, products and by-products from cereal milling (including wet milling and dry milling), such as corn, wheat, and barley, often referred to as "bran or fiber", and municipal solids. Biomass can also be, but is not limited to, grassy materials, agricultural residues, forestry residues, municipal solid waste, waste paper, and pulp and paper mill residues. Agricultural biomass includes branches, shrubs, tows, corn and corn straw, energy crops, forests, fruits, flowers, cereals, pastures, herbaceous crops, leaves, bark, needles, logs, roots, young trees, short term rotating woody crops, shrubs, switch herbs, trees, vegetables, fruits, vines, sugar beet pulp, wheat middlings, oat hulls, and hard and soft timber (not including toxic wood). Furthermore, agricultural biomass includes organic waste materials resulting from agricultural processes including agriculture and forestry activities, particularly forestry wood waste. Agricultural biomass may be any of the foregoing alone, or any combination or mixture thereof.
In preferred embodiments, the substrate comprises, essentially consists of, or consists of, preferably comprises, at least one of protocatechuic aldehyde, benzoic acid, benzaldehyde, benzyl alcohol, *p*-anisic acid, *p*-anisaldehyde, *p*-anisyl alcohol, *p*-hydroxybenzoic acid, *p-*hydroxybenzaldehyde, 3,4-dihydroxybenzaldehyde, *p*-hydroxybenzyl alcohol, *m*-hydroxybenzoic acid, *p*-coumaric acid, caffeic acid, cinnamic acid, and cinnamyl alcohol, preferably at least one of benzaldehyde, *p*-hydroxybenzaldehyde and 3,4-dihydroxybenzaldehyde.
Preferred cells according to the invention are able to convert at least 20%, 25%, 30%, 35%, 40%, 45%, 50%, 55%, 60%, 65%, 70%, 75%, 80%, 85%, 90%, 95%, or 100%, preferably at least 40%, more preferably at least 80%, of a substrate into a triple-substituted benzene, preferably protocatechuic acid and/or hydroxyquinol.
The produced amount and/or substrate conversion is preferably obtained after 2 weeks, 1 week, 6 days, 5 days, 4 days, 3 days, 2 days, 24 hours, 15 hours, 10 hours, 5 hours, 4 hours, 3 hours, 2 hours or 1 hour of culture, preferably after 24 hours of culture, more preferably after 15 hours of culture and most preferably after 10 hours of culture. The production of the relevant compounds can be monitored using chromatographic techniques on samples obtained from the growth medium, culture broth, culture supernatant, and/or culture headspace, preferably culture supernatant, as described elsewhere herein.

Accordingly, in a preferred embodiment a host cell as defined herein is provided, capable of producing at least 0.8 mM of protocatechuic acid, more preferably capable of producing at least 1.5 mM of protocatechuic acid, or capable of converting at least 40% of a substrate into protocatechuic acid, preferably capable of converting at least 80% of a substrate into protocatechuic acid, preferably after at most 2 weeks of culture, more preferably after at most 24 hours of culture. The substrate preferably comprises, essentially consists of, or consists of, more preferably comprises at least one of protocatechuic aldehyde, benzoic acid, benzaldehyde, benzyl alcohol, p-anisic acid, p-anisaldehyde, p-anisyl alcohol, p-hydroxybenzoic acid, p-hydroxybenzaldehyde, p-hydroxybenzyl alcohol, m-hydroxybenzoic acid, p-coumaric acid, caffeic acid and cinnamyl alcohol. Preferably, said produced amount and/or substrate conversion is obtained after 24 hours of culture.
In another preferred embodiment a host cell as defined herein is provided, wherein the host cell is a filamentous fungal cell, preferably *Aspergillus niger,* wherein the host cell comprises a genetic modification that reduces or eliminates the expression of a functional enzyme with protocatechuic hydroxylase (Phy) activity, and further comprises a genetic modification that reduces or eliminates the expression of a functional enzyme with protocatechuate 3,4-dioxygenase (PrcA) activity as compared to a corresponding wild type cell lacking the genetic modifications, wherein the host cell is capable of converting at least 40% of a substrate as defined herein into protocatechuic acid. Preferably, said substrate conversion is obtained after 24 hours of culture.

Useful enzymatic activities for the production of compounds according to the invention are additionally present in cell lysates of the host cells. Accordingly, in an embodiment, cell lysates of host cells according to the invention are provided. As used herein, the term "cell lysing" refers to a process of rupturing the cell wall and/or cell membrane of a cell as to obtain a cell lysate which comprises intracellular material and cellular debris. The exact method of cell lysing is not a critical feature of the invention. Such methods are well-known in the art (examples can be found in standard handbooks such as Sambrook and Russel, Molecular Cloning: A Laboratory Manual, 3rd edition, Cold Spring Harbor Laboratory, Cold Spring Harbor Laboratory Press, NY, 2001 or Ausubel F. et al, eds., Current protocols in molecular biology, Green Publishing and Wiley Interscience, NY, 2003) and include mechanical, chemical, enzymatic or physical treatment of cells or combinations of said treatments. Examples of mechanical treatment include cell homogenization using a French pressure cell press, a sonicator, a homogenizer, a ball mill, a rod mill, a pebble mill, a bead mill, a high pressure grinding roll, a vertical shaft impactor, an industrial blender, a high shear mixer, a paddle mixer, a polytron homogenizer and the like. Examples of chemical treatment include raising a pH of a cell, for example by adding a base to a cell culture, contacting a cell with a chemical and the like. Examples of enzymatic treatment include lysis of a cell wall or cell membrane of a cell by contacting the cell with one or more enzymes, such as proteases, cellulases, hemicellulases, chitinases, pectinases, and combinations thereof. Examples of physical treatment include heating a cell, drying a cell and the like. The cell lysate used may or may not still comprise the cellular debris, preferably it does not comprise the cellular debris. Methods for separating and/or removing cellular debris are well known in the art, for example centrifugation or filtration can be used. As an example, centrifugation for 5-10 min at 3000-4000 x g may be used to separate the cellular debris. A cell lysate not comprising the cellular debris may be called a cell-free extract, and use thereof is also provided.

### Genetic modifications and expression vectors

For expression of a functional enzyme in a host cell according to the invention, as well as for genetic modification of a host cell resulting in increased, reduced or eliminated expression of a functional enzyme according to the invention, the cell can be transformed with a nucleic acid or nucleic acid construct described herein by any method known to the person skilled in the art. Such methods are e.g. known from standard handbooks, such as Sambrook and Russel, Molecular Cloning: A Laboratory Manual, 3rd edition, Cold Spring Harbor Laboratory, Cold Spring Harbor Laboratory Press, NY, 2001; Ausubel F. et al, eds., Current protocols in molecular biology, Green Publishing and Wiley Interscience, NY, 2003; Sherman et al., Methods Yeast Genetics, Cold Spring Harbor Laboratory, NY, 1978; Guthrie et al. (Eds.) Guide To Yeast Genetics and Molecular Biology Vol. 194, Academic Press, San Diego, 1991; Sudbery, P. E., Genetic Engineering of Yeast, in Biotechnology Set, Second Edition (eds H.-J. Rehm and G. Reed), Wiley-VCH Verlag GmbH, Weinheim, Germany, 2001. Examples include transformation using competent or supercompetent cells, electroporation, use of transfection lipids, use of transfection polymers, or gymnotic transformation. A preferred method is electroporation.
For transformation of filamentous fungal cells, mycelia, spores, and/or protoplasts, preferably protoplasts, may be used, as described in standard handbooks such as Moore, M., Genetic engineering of fungal cells, In Biotechnology Vol. III (eds H. W. Doelle and E. J. Dasilva), EOLSS, Ontario, Canada, 2007. Additional suitable methods for transformation of *Aspergillus* cells are described in Kowalczyk et al., 2017, Yelton et al., Proceedings of the National Academy of Sciences USA 81: 1470-1474, 1984.
A "nucleic acid construct" may be defined as a nucleotide sequence which is isolated from a naturally occurring (native) gene or which has been modified to contain segments of nucleotide sequences which are combined or juxtaposed in a manner which would not otherwise exist in nature. An isolated nucleotide sequence is a DNA or RNA sequence which is not immediately contiguous with one or both the nucleotide sequences (on the 5' and 3' ends) with which it is naturally immediately contiguous in the genome of the organism it is derived from.
Optionally, mutants of naturally occurring genes are made by modifying the nucleotide sequence encoding the naturally occurring genes using well-known mutagenesis techniques such as random mutagenesis, site-directed mutagenesis, directed evolution, gene shuffling and the like, so that the resulting nucleotide sequence encodes an enzyme that differs by at least one amino acid from the naturally occurring enzyme, said enzyme having improved properties with respect to (stereo)selectivity and/or activity and/or stability and/or solvent resistance and/or pH and/or temperature profiles as compared to the naturally occurring enzyme. Optionally, a nucleotide sequence present in a nucleic acid construct is operably linked to one or more regulatory sequences, which direct expression of said nucleotide sequence in a cell. Such regulatory sequences typically at least include a promoter that functions to control the transcription of the coding sequence, which is usually located upstream of, and preferably operably linked to the coding sequence. In addition to the promoter, the upstream transcription regulatory sequences may comprise further elements such as enhancers, upstream activating sequences, transcription factor binding sites, repressor and activator protein binding sites and the like. The promoter sequence will usually include the transcription initiation site(s).
Downstream of the promoter and transcription initiation site(s), the nucleic acid construct may optionally comprise the translation initiation sequences, such as the eukaryotic Kozak consensus sequence, surrounding the translation initiation codon, i.e. the first codon of the coding sequence. The coding sequence is terminated with a translation stop codon. Downstream of the coding sequence, the expression construct may optionally comprise a 3'-untranslated region containing one or more transcription termination sites, e.g. a terminator, which preferably also includes a polyadenylation site. Preferably, the terminator is endogenous to the host cell (in which the polypeptide is to be expressed).
Nucleic acid sequences described herein may be codon optimized. Codon optimization adapts the codon usage towards the codon bias of the organism where the nucleic acid sequence is expressed in. Codon optimization generally helps to increase the production level of the encoded polypeptide in the host cell. Many algorithms are available to the person skilled in the art for codon optimization. A preferred method is the "Guided random method based on a Monte Carlo alogorithm" available via the internet at genomes.urv.es/OPTIMIZER/ (P. Puigbò, E. Guzman, A. Romeu, and S. Garcia-Vallve. Nucleic Acids Res. 2007 July; 35 (Web Server issue): W126-W131).
As used herein the term "disruption" of a functional enzyme encompasses all possible host cell modifications that result in said enzyme activity being decreased or eliminated as compared to a corresponding cell that has not been modified and/or said enzyme activity no longer being detectable in the host cell and/or culture medium containing the host cell, as compared to a corresponding cell that has not been modified and/or culture medium containing a corresponding cell that has not been modified. Non-limiting examples of host cell modifications include modification of any step of the expression of a functional enzyme such as transcription, post-transcriptional modification, translation, post-translational modification, and secretion. Preferably, a disruption is achieved by deleting a gene encoding for a functional enzyme and/or its regulatory sequences, said disruption resulting in elimination of the enzyme transcript and, thereby, the enzyme activity. Said disruption may preferably be achieved by homologous recombination, following e.g. transformation of a host cell with a marker gene flanked by homologous regions to the targeted locus as described elsewhere herein. The disruption may preferably be assayed using molecular methods known to the skilled person, such as PCR and/or Southern blotting, or by enzymatic activity assays as discussed elsewhere herein.
"Gene" is defined herein as a DNA nucleotide sequence comprising a region (transcribed region), which is transcribed into an RNA molecule (e.g. an mRNA) in a cell, operably linked to suitable regulatory regions (e.g. a promoter). A gene will usually comprise several operably linked fragments, such as a promoter, a 5' leader sequence, a coding region and 5' a 3'-nontranslated sequence (3'-end) comprising a polyadenylation site.
As used herein the term "heterologous sequence" or "heterologous nucleic acid" encompasses a sequence that is not naturally found operably linked as neighbouring sequence of said first nucleotide sequence, which is preferably derived from a different organism from the host cell. As used herein, the term "heterologous" may also mean "recombinant". "Recombinant" refers to a genetic entity distinct from that generally found in nature. As applied to a nucleotide sequence or nucleic acid molecule, this means that said nucleotide sequence or nucleic acid molecule is the product of various combinations of cloning, restriction and/or ligation steps, and other procedures that result in the production of a construct that is distinct from a sequence or molecule found in nature. A recombinant nucleic acid can be a nucleic acid that comprises sequences from more than one single source. A recombinant polypeptide such as an enzyme is one expressed from a recombinant nucleic acid.
"Operably linked" is defined herein as a configuration in which a control sequence is appropriately placed at a position relative to the nucleotide sequence coding for the polypeptide of the invention such that the control sequence directs the production/expression polypeptide of the invention in a cell and/or in a subject. "Operably linked" may also be used for defining a configuration in which a sequence is appropriately placed at a position relative to another sequence coding for a functional domain such that a chimeric polypeptide is encoded in a cell and/or in a subject. Throughout this disclosure, any nucleic acid sequence coding for a functional enzyme is preferably operably linked to another such sequence, or to a promoter.
The terms "expression vector" or "expression construct" refer to nucleotide sequences that are capable of effecting expression of a gene in host cells or host organisms compatible with such sequences. These expression vectors typically include at least suitable promoters and optionally, 3' transcription termination signals. Additional factors necessary or helpful in effecting expression may also be present, such as expression enhancer elements. The expression vector will be introduced into a suitable host cell and be able to effect expression of the coding sequence in an in vitro cell culture of the host cell.
As used herein, the term "promoter" refers to a nucleic acid fragment that functions to control the transcription of one or more nucleic acid molecules, usually located upstream with respect to the direction of transcription of the transcription initiation site of the nucleic acid molecule, and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skill in the art to act directly or indirectly to regulate the amount of transcription from the promoter. A "constitutive" promoter is a promoter that is active under most environmental and developmental conditions. An "inducible" promoter is a promoter that is active under environmental or developmental regulation.
Optional further elements that may be present in a nucleic acid construct according to the invention include, but are not limited to, one or more leader sequences, enhancers, integration factors, and/or reporter genes, intron sequences, centromers, telomers and/or matrix attachment (MAR) sequences. A nucleic acid construct according to the invention can be provided in a manner known per se, which generally involves techniques such as restricting and linking nucleic acids/nucleic acid sequences, for which reference is made to the standard handbooks, such as Sambrook and Russel, Molecular Cloning: A Laboratory Manual, 3rd edition, Cold Spring Harbor Laboratory, Cold Spring Harbor Laboratory Press, NY, 2001.
Suitable elements useful for genetic modification of filamentous fungi are standard and known to those in the art, including e.g. promoters for (over-)expression of genes, other regulatory sequences such as terminators, episomal and/or integrating expression constructs and vectors, selectable markers, and methods and genetic constructs for disrupting and/or deleting endogenous fungal genes or parts thereof, described e.g. in Moore, M., Genetic engineering of fungal cells, In Biotechnology Vol. III (eds H. W. Doelle and E. J. Dasilva), EOLSS, Ontario, Canada, 2007; Lubertozzi, D., Keasling, J. D. (2009) 27(1), 53-75; Meyer, V. (2008) Biotechnology Advances, (26), 177-85; Kuck and Hoff (2010) Applied microbiology and biotechnology, 86(1), 51-62; and WO2014/142647.

Accordingly, in a third aspect the invention provides an expression vector comprising a nucleic acid construct comprising a nucleotide sequence encoding a functional enzyme with protocatechuic hydroxylase (Phy) activity, and/or protocatechuate 3,4-dioxygenase (Prc) activity, and/or hydroxyquinol 1,2-dioxygenase (Hqd) activity, preferably encoding a functional enzyme with protocatechuic hydroxylase (Phy) activity. The features of the nucleic acid constructs are preferably those as described herein. Such expression vectors are referred to herein as expression vectors according to the invention. Suitable expression vectors may be selected from any vector which can bring about the expression of the nucleic acid sequence encoding the enzymes of interest upon its introduction to the host cell according to methods known in the art as described herein.
The choice of the expression vector will typically depend on the compatibility of the vector with the cell into which the vector is to be introduced. The expression vectors may be linear or closed circular plasmids. The vector may be an autonomously replicating vector, i.e. a vector which exists as an extra-chromosomal entity, the replication of which is independent of chromosomal replication, e.g., a plasmid, an extra-chromosomal element, a mini-chromosome, or an artificial chromosome. The expression vector may also be an integrative vector, i.e. a vector which integrates into the genome and replicates together with the chromosome(s) into which it has been integrated. The integrative expression vector may integrate at random or at a predetermined target locus in the chromosomes of the host cell. Preferably, the integrative cloning vector comprises a DNA fragment, which is homologous to a DNA sequence in a predetermined target locus in the genome of host cell for targeting the integration of the cloning vector to the predetermined locus. The integrative expression vector may be linearized prior to transformation of the cell. Linearization is preferably performed such that at least one but preferably either end of the cloning vector is flanked by sequences homologous to the target locus. The length of the homologous sequences flanking the target locus may be at least 30 bp, at least 50 bp, at least 0.1 kb, at least 0.2 kb, at least 0.5 kb, at least 1 kb, at least 2 kb. Preferably, the length of the homologous sequences flanking the target locus is at least 0.5 kb, more preferably at least 1kb, even more preferably 2 kb. Preferably, the efficiency of targeted integration into the genome of the host cell, i.e. integration in a predetermined target locus, is increased by augmenting the homologous recombination capability of the host cell, for example by prior disruption of non-homologous end-joining DNA repair machinery. Examples of such methods are discussed in WO2005/095624A2 and WO2005/095624A2.

In some embodiments the invention provides an expression vector comprising a nucleic acid construct comprising a nucleotide sequence that has at least 60%, 65%, 70%, 75%, 80%, 85%, 90%, 95%, 96%, 97%, 98%, 99%, or 100%, preferably at least 60%, more preferably at least 80%, even more preferably at least 90%, most preferably 100% sequence identity with SEQ ID NO: 2 or 10, or wherein the expression vector consists of a polynucleotide that has at least 60%, 65%, 70%, 75%, 80%, 85%, 90%, 95%, 96%, 97%, 98%, 99%, or 100%, preferably at least 60%, more preferably at least 80%, even more preferably at least 90%, most preferably 100% sequence identity with SEQ ID NO: 3 or 13. In preferred embodiments, the expression vector comprises a nucleic acid construct comprising a nucleotide sequence that has at least 90% sequence identity with SEQ ID NO: 2 or 10, or the expression vector consists of a polynucleotide that has at least 90% sequence identity with SEQ ID NO: 3 or 13. In still preferred embodiments, the expression vector comprises a nucleic acid construct comprising a nucleotide sequence that has at least 95% sequence identity with SEQ ID NO: 2 or 10, or the expression vector consists of a polynucleotide that has at least 95% sequence identity with SEQ ID NO: 3 or 13. In more preferred embodiments, the expression vector comprises a nucleic acid construct comprising a nucleotide sequence that has at least 96% sequence identity with SEQ ID NO: 2 or 10, or the expression vector consists of a polynucleotide that has at least 96% sequence identity with SEQ ID NO: 3 or 13. In still more preferred embodiments, the expression vector comprises a nucleic acid construct comprising a nucleotide sequence that has at least 97% sequence identity with SEQ ID NO: 2 or 10, or the expression vector consists of a polynucleotide that has at least 97% sequence identity with SEQ ID NO: 3 or 13. In still more preferred embodiments, the expression vector comprises a nucleic acid construct comprising a nucleotide sequence that has at least 98% sequence identity with SEQ ID NO: 2 or 10, or the expression vector consists of a polynucleotide that has at least 98% sequence identity with SEQ ID NO: 3 or 13. In even more preferred embodiments, the expression vector comprises a nucleic acid construct comprising a nucleotide sequence that has at least 99% sequence identity with SEQ ID NO: 2 or 10, or the expression vector consists of a polynucleotide that has at least 99% sequence identity with SEQ ID NO: 3 or 13. In most preferred embodiments, the expression vector comprises a nucleic acid construct comprising a nucleotide sequence that has 100% sequence identity with SEQ ID NO: 3 or 10, or the expression vector consists of a polynucleotide that has 100% sequence identity with SEQ ID NO: 3 or 13.
In some embodiments the invention provides an expression vector comprising a nucleic acid construct comprising a nucleotide sequence that has at least 60%, 65%, 70%, 75%, 80%, 85%, 90%, 95%, 96%, 97%, 98%, 99%, or 100%, preferably at least 60%, more preferably at least 80%, even more preferably at least 90%, most preferably 100% sequence identity with SEQ ID NO: 5 or 11, or wherein the expression vector consists of a polynucleotide that has at least 60%, 65%, 70%, 75%, 80%, 85%, 90%, 95%, 96%, 97%, 98%, 99%, or 100%, preferably at least 60%, more preferably at least 80%, even more preferably at least 90%, most preferably 100% sequence identity with SEQ ID NO: 6. In preferred embodiments, the expression vector comprises a nucleic acid construct comprising a nucleotide sequence that has at least 90% sequence identity with SEQ ID NO: 5 or 11, or it consists of a polynucleotide that has at least 90% sequence identity with SEQ ID NO: 6. In still preferred embodiments, it comprises a nucleic acid construct comprising a nucleotide sequence that has at least 95% sequence identity with SEQ ID NO: 5 or 11, or the expression vector consists of a polynucleotide that has at least 95% sequence identity with SEQ ID NO: 6. In more preferred embodiments, the expression vector comprises a nucleic acid construct comprising a nucleotide sequence that has at least 96% sequence identity with SEQ ID NO: 5 or 11, or the expression vector consists of a polynucleotide that has at least 96% sequence identity with SEQ ID NO: 6. In still more preferred embodiments, it comprises a nucleic acid construct comprising a nucleotide sequence that has at least 97% sequence identity with SEQ ID NO: 5 or 11, or the expression vector consists of a polynucleotide that has at least 97% sequence identity with SEQ ID NO: 6. In still more preferred embodiments, the expression vector comprises a nucleic acid construct comprising a nucleotide sequence that has at least 98% sequence identity with SEQ ID NO: 5 or 11, or the expression vector consists of a polynucleotide that has at least 98% sequence identity with SEQ ID NO: 6. In even more preferred embodiments, the expression vector comprises a nucleic acid construct comprising a nucleotide sequence that has at least 99% sequence identity with SEQ ID NO: 5 or 11, or the expression vector consists of a polynucleotide that has at least 99% sequence identity with SEQ ID NO: 6. In most preferred embodiments, it comprises a nucleic acid construct comprising a nucleotide sequence that has 100% sequence identity with SEQ ID NO: 5 or 11, or it consists of a polynucleotide that has 100% sequence identity with SEQ ID NO: 6.

In some embodiments the invention provides an expression vector comprising a nucleic acid construct comprising a nucleotide sequence that has at least 60%, 65%, 70%, 75%, 80%, 85%, 90%, 95%, 96%, 97%, 98%, 99%, or 100%, preferably at least 60%, more preferably at least 80%, even more preferably at least 90%, most preferably 100% sequence identity with SEQ ID NO: 8 or 12, or wherein the expression vector consists of a polynucleotide that has at least 60%, 65%, 70%, 75%, 80%, 85%, 90%, 95%, 96%, 97%, 98%, 99%, or 100%, preferably at least 60%, more preferably at least 80%, even more preferably at least 90%, most preferably 100% sequence identity with SEQ ID NO: 9. In preferred embodiments, the expression vector comprises a nucleic acid construct comprising a nucleotide sequence that has at least 90% sequence identity with SEQ ID NO: 8 or 12, or the expression vector consists of a polynucleotide that has at least 90% sequence identity with SEQ ID NO: 9. In still preferred embodiments, the expression vector comprises a nucleic acid construct comprising a nucleotide sequence that has at least 95% sequence identity with SEQ ID NO: 8 or 12, or the expression vector consists of a polynucleotide that has at least 95% sequence identity with SEQ ID NO: 9. In more preferred embodiments, the expression vector comprises a nucleic acid construct comprising a nucleotide sequence that has at least 96% sequence identity with SEQ ID NO: 8 or 12, or the expression vector consists of a polynucleotide that has at least 96% sequence identity with SEQ ID NO: 9. In still more preferred embodiments, the expression vector comprises a nucleic acid construct comprising a nucleotide sequence that has at least 97% sequence identity with SEQ ID NO: 8 or 12, or the expression vector consists of a polynucleotide that has at least 97% sequence identity with SEQ ID NO: 9. In still more preferred embodiments, the expression vector comprises a nucleic acid construct comprising a nucleotide sequence that has at least 98% sequence identity with SEQ ID NO: 8 or 12, or the expression vector consists of a polynucleotide that has at least 98% sequence identity with SEQ ID NO: 9. In even more preferred embodiments, the expression vector comprises a nucleic acid construct comprising a nucleotide sequence that has at least 99% sequence identity with SEQ ID NO: 8 or 12, or the expression vector consists of a polynucleotide that has at least 99% sequence identity with SEQ ID NO: 9. In most preferred embodiments, the expression vector comprises a nucleic acid construct comprising a nucleotide sequence that has 100% sequence identity with SEQ ID NO: 8 or 12, or the expression vector consists of a polynucleotide that has 100% sequence identity with SEQ ID NO: 9.

Alternatively or in combination, isolated nucleotide sequences or nucleic acid constructs comprising nucleotide sequences encoding for the functional enzymes according to the invention may be introduced to the host cells. Said nucleotide sequences and/or nucleic acid constructs may be flanked by sequences homologous to the target locus, similarly to when integrative expression vectors are used. Any nucleotide sequence and/or nucleic acid construct may be contemplated as long as it can bring about the expression of the functional enzymes according to the invention upon its introduction to the host cell according to methods known in the art as previously described herein. Preferably, such a nucleotide sequence and/or nucleic acid construct is integrated into the chromosome(s) of the host cell after transformation of the host cells. In some embodiments, the isolated nucleotide sequences or nucleic acid constructs are introduced to the host cells as repair fragments following CRISPR/Cas-mediated transformation according to methods known in the art, described for example in WO2016/110453.

The nucleic acid constructs and/or expression vectors according to the invention may further comprise a selectable marker. The term "marker" refers herein to a gene encoding a trait or a phenotype which permits the selection of, or the screening for, a cell containing the marker. A marker gene may be an antibiotic resistance gene whereby the appropriate antibiotic can be used to select for transformed cells from among cells that are not transformed. A preferred selection marker is kanamycin and its corresponding resistance gene. Preferably however, a non-antibiotic resistance marker is used, such as an auxotrophic marker (Ura3, Trp1, Leu2). Other selectable markers, particularly useful for use in a filamentous fungal cells are known to the skilled person and described, for example, in handbooks referred to elsewhere herein and may be selected from the group including, but not limited to, AmdS (acetamidase), ArgB (ornithine carbamoyltransferase), Bar (phosphinothricinacetyltransferase), BleA (phleomycin binding), HygB (hygromycin phosphotransferase), Nial (nitrate reductase), PyrG (orotidine-5'-phosphate decarboxylase), SC (sulfate adenyltransferase), and TrpC (anthranilate synthase), as well as equivalents. Preferably, the selectable marker is PyrG from *Aspergillus oryzae* or hph from *Escherichia coli.* A preferred cell according to the invention, e.g. transformed with a nucleic acid construct, is marker gene free. Methods for constructing recombinant marker gene free microbial host cells are known to the skilled person and examples are available in handbooks referred to elsewhere herein. Alternatively, a screenable marker such as Green Fluorescent Protein, LacZ, luciferase, chloramphenicol acetyltransferase, beta-glucuronidase may be incorporated into a nucleic acid construct according to the invention allowing to screen for transformed cells.

Increased expression of a functional enzyme by a host cell, resulting in increased levels of enzyme transcript, enzyme polypeptide, and/or enzyme activity as compared to a corresponding wild type cell lacking the genetic modification, can be achieved by means known to the skilled person which are described, for example, in handbooks referred to elsewhere herein. Examples include replacing native promoters with strong constitutive or inducible promoters to drive gene expression, codon optimization, evolutionary engineering, random or directed mutagenesis, transformation with multiple copies of the relevant nucleotide sequences, nucleic acid constructs and/or expression vectors and the like.
Reduced expression of a functional enzyme by a host cell, resulting in decreased levels of enzyme transcript, enzyme polypeptide, and/or enzyme activity as compared to a corresponding wild type cell lacking the genetic modification, can be achieved by means known to the skilled person which are described, for example, in handbooks referred to elsewhere herein. Examples include replacing native promoters with weak constitutive or inducible promoters to drive gene expression, replacing native genes with heterologous genes, random or directed mutagenesis, fusion of nucleic acid constructs with nucleotide sequences encoding for degradation tags, activating a transcriptional repressor, repressing a repressible promoter, decreasing RNA stability, activating a post-transcriptional inhibitor (for example, expressing a ribozyme or antisense oligonucleotide) or inhibiting translation (for example, via a regulatable RNA) and the like.
Elimination of expression of a functional enzyme refers to embodiments wherein the activity of the functional enzyme has been eliminated and can be the result of a disruption of any step involved in the production of said enzyme including, but not limited to, transcription, post-transcriptional modification, translation, post-translational modification, and secretion. For example, the gene encoding for said enzyme may be inactivated or deleted. Gene inactivation may be achieved by any suitable means as known to a skilled person, with examples being gene deletion, random or directed mutagenesis, PCR or site-directed mutagenesis, for example to disturb the open reading frame, activating a transcriptional repressor, repressing a repressible promoter, activating a post-transcriptional inhibitor (for example, expressing a ribozyme or antisense oligonucleotide) or inhibiting translation (for example, via a regulatable RNA) and the like. Gene deletion or inactivation should also be interpreted as to include embodiments wherein only its regulatory sequences (such as enhancer, promoter and terminator sequences) are deleted or inactivated instead of the complete gene (which includes the open reading frame), and embodiments wherein only the open reading frame is deleted or inactivated instead of the complete gene. Gene deletion may be achieved by any suitable means known to a skilled person including, for example, excising a gene or fragment thereof, and/or its regulatory sequences (such as enhancer, promoter and terminator sequences) using a FLP-FRT or Cre-Lox cassette, homologous recombination or CRIPR-Cas9 activity or through loss or degradation of a plasmid. Preferably, homologous recombination is used for gene deletion. In a preferred embodiment, the deleted nucleotide sequence is replaced by a selectable marker as described herein which is flanked by homologous sequences to the target locus as described herein, more preferably said selectable marker is PyrG or hph, most preferably PyrG from *Aspergillus oryzae* or hph from *Escherichia coli.*
In the context of the invention, elimination of expression should not be interpreted as to exclude embodiments wherein the transcript encoding for the enzyme or the enzyme polypeptide are present in the host cell. For example, an enzyme polypeptide might require a post-translational modification in order to be converted to a functional enzyme and the host cell might lack the capability of performing this post-translation modification while the polypeptide is present or an mRNA transcript may be translated to a polypeptide unable to fold in a way as to result in a functional enzyme.
In a preferred embodiment *Aspergillus niger* cells are transformed. Preferably, said cells are deficient in expression of ATP-dependent DNA helicase II subunit 1 (kusA). Gene deletion is preferably performed using homologous recombination using deletion cassettes comprising homologous sequences of at least 900 bp, preferably at least 1000 bp, upstream and downstream of the target gene, fused to an orotidine 5'-phosphate decarboxylase (pyrG) from *Aspergillus oryzae* or to a hygromycin phosphotranferase (hph) from *Escherichia coli.* For protoplast preparation, young mycelia from overnight culture are harvested, preferably by vacuum filtration, washed with 0.4-0.8 M such as about 0.6 M MgSO4 and dried, preferably between two sheets of paper. The mycelium is then dissolved, preferably in PS buffer (0.1-0.3 M such as about 0.2 M sodium phosphate buffer, 0.6-1 M such as about 0.8 M L-sorbitol, about pH 6) containing VinoTaste^{®} Pro lysing enzyme (about 0.5 g enzyme/g of mycelia) and incubated, preferably in a rotary shaker at an agitation speed of about 100 rpm. When protoplasts are abundantly present, the mixture is filtrated, preferably through glass wool and undigested mycelia debris is removed. The protoplasts are collected, preferably by centrifugation (about 10 min, 1811 ×g, 4 °C), washed, preferably twice with ice-cold SC solution (about 182.2 g L⁻¹ sorbitol, about 7.35 g L⁻¹ CaCl2* 2H₂O), and resuspended, preferably in SC, to a final concentration of about 2 * 10⁷ protoplasts/mL. For transformation, about 200 µL of fresh protoplast suspension, about 20 µL of 0.2-0.6 such as about 0.4 M ATA (aurintricarboxylic acid ammonium salt), about 100 µL of 10-30% such as about 20% PEG-4000 are mixed with about 5 µg of deletion cassette DNA and incubated for about 10 min. After addition of about 1.5 mL 50-70% such as about 60% PEG-4000 the mixture is incubated for about 20 min. Next, about 5 mL of 1-1.4 such as about 1.2 M sorbitol solution is added and the mixture is incubated for about another 10 min. Transformed protoplasts are collected, preferably by centrifugation (about 10 min, 3220 ×g), resuspended in about 1 mL 1-1.4 such as about 1.2 M sorbitol and spread over selective plates using a cell spreader. Growing colonies are observed after about 4 days.

### Polypeptides

In a fourth aspect, the invention provides a polypeptide product expressed from the expression vector according to the third aspect. Features and definitions are provided elsewhere herein. The polypeptide is preferably at least 70% pure, more preferably at least 75%, 80%, 85%, 90%, 95%, 96%, 97%, 98%, 99% pure, most preferably it is substantially pure. Optionally, the polypeptide can be in a composition comprising it, preferably further comprising a buffered solution. Suitable compositions will be known to the skilled person and are discussed in standard handbooks, for example in and Bisswanger H, Practical Enzymology, 2nd edition, Wiley-VCH Verlag GmbH & Co. KgaA, 2012. An example of a suitable buffered solution is a phosphate-buffered saline solution.

In an embodiment, a polypeptide product expressed from an expression vector comprising a nucleic acid construct comprising a nucleotide sequence that has at least 60%, 65%, 70%, 75%, 80%, 85%, 90%, 95%, 96%, 97%, 98%, 99%, or 100%, preferably at least 60%, more preferably at least 80%, even more preferably at least 90%, most preferably 100% sequence identity with SEQ ID NO: 2 or 10, or wherein the expression vector consists of a polynucleotide that has at least 60%, 65%, 70%, 75%, 80%, 85%, 90%, 95%, 96%, 97%, 98%, 99%, or 100%, preferably at least 60%, more preferably at least 80%, even more preferably at least 90%, most preferably 100% sequence identity with SEQ ID NO: 3 or 13 is provided. In preferred embodiments, a polypeptide product expressed from an expression vector comprising a nucleic acid construct comprising a nucleotide sequence that has at least 90% sequence identity with SEQ ID NO: 2 or 10, or wherein the expression vector consists of a polynucleotide that has at least 90% sequence identity with SEQ ID NO: 3 or 13 is provided. In still preferred embodiments, a polypeptide product expressed from an expression vector comprising a nucleic acid construct comprising a nucleotide sequence that has at least 95% sequence identity with SEQ ID NO: 2 or 10, or wherein the expression vector consists of a polynucleotide that has at least 95% sequence identity with SEQ ID NO: 3 or 13 is provided. In more preferred embodiments, a polypeptide product expressed from an expression vector comprising a nucleic acid construct comprising a nucleotide sequence that has at least 96% sequence identity with SEQ ID NO: 2 or 10, or wherein the expression vector consists of a polynucleotide that has at least 96% sequence identity with SEQ ID NO: 3 or 13 is provided. In still more preferred embodiments, a polypeptide product expressed from an expression vector comprising a nucleic acid construct comprising a nucleotide sequence that has at least 97% sequence identity with SEQ ID NO: 2 or 10, or wherein the expression vector consists of a polynucleotide that has at least 97% sequence identity with SEQ ID NO: 3 or 13 is provided. In still more preferred embodiments, a polypeptide product expressed from an expression vector comprising a nucleic acid construct comprising a nucleotide sequence that has at least 98% sequence identity with SEQ ID NO: 2 or 10, or wherein the expression vector consists of a polynucleotide that has at least 98% sequence identity with SEQ ID NO: 3 or 13 is provided. In even more preferred embodiments, a polypeptide product expressed from an expression vector comprising a nucleic acid construct comprising a nucleotide sequence that has at least 99% sequence identity with SEQ ID NO: 2 or 10, or wherein the expression vector consists of a polynucleotide that has at least 99% sequence identity with SEQ ID NO: 3 or 13 is provided. In most preferred embodiments, a polypeptide product expressed from an expression vector comprising a nucleic acid construct comprising a nucleotide sequence that has 100% sequence identity with SEQ ID NO: 3 or 10, or wherein the expression vector consists of a polynucleotide that has 100% sequence identity with SEQ ID NO: 3 or 13 is provided.

### Methods for production of compounds

The enzymes, host cells and lysates of host cells according to the invention are useful for the production of compounds involved in benzoic acid-related pathways including triple-substituted benzenes such as protocatechuic acid and hydroxyquinol. In some embodiments the methods are for production of protocatechuic acid. In some embodiments the methods are for production of hydroxyquinol.
Accordingly, in a fifth aspect, the invention provides a method for the production of a compound involved in a benzoic acid-related metabolic pathway, preferably a triple-substituted benzene, comprising the steps of:
- culturing a cell according the invention, or contacting a lysate of a cell according to the invention with a substrate comprising at least one of protocatechuic aldehyde, benzoic acid, benzaldehyde, benzyl alcohol, p-anisic acid, p-anisaldehyde, p-anisyl alcohol, p-hydroxybenzoic acid, p-hydroxybenzaldehyde, p-hydroxybenzyl alcohol, m-hydroxybenzoic acid, p-coumaric acid, caffeic acid and cinnamyl alcohol, under conditions conducive to the production of said compound, and, optionally,
- isolating and/or purifying said compound from the cell and/or the culture medium and/or the reaction medium.

In an embodiment, the invention provides a method for the production of protocatechuic acid, comprising the steps of:
- culturing a cell according the invention under conditions conducive to the production of protocatechuic acid, preferably in the presence of protocatechuic aldehyde, and, optionally,
- isolating and/or purifying the protocatechuic acid from the cell and/or the culture medium. Cells used in these embodiments preferably have reduced PhyA expression as defined earlier herein. Cells used in these embodiments preferably have reduced PrcA expression as defined earlier herein. Most preferably both PhyA and PrcA are reduced.
In an embodiment, the invention provides a method for the production of hydroxyquinol, comprising the steps of:
- culturing a cell according the invention under conditions conducive to the production of hydroxyquinol, and, optionally,
- isolating and/or purifying the hydroxyquinol from the cell and/or the culture medium. Cells used in these embodiments preferably have PhyA expression as defined earlier herein, more preferably increased PhyA expression. Cells used in these embodiments preferably have reduced PrcA expression as defined earlier herein. Most preferably both PhyA is increased and PrcA is reduced. In an embodiment, the invention provides a method for the production of 3-carboxy-cis,cis-muconic acid, comprising the steps of:
- culturing a cell according the invention under conditions conducive to the production of 3-carboxy-cis,cis-muconic acid, and, optionally,
- isolating and/or purifying the 3-carboxy-cis,cis-muconic acid from the cell and/or the culture medium. Cells used in these embodiments preferably have reduced PhyA expression as defined earlier herein. Cells used in these embodiments preferably have increased PrcA expression as defined earlier herein. Most preferably both PhyA is reduced and PrcA is increased.
In an embodiment, the invention provides a method for the production of maleylacetic acid, comprising the steps of:
- culturing a cell according the invention under conditions conducive to the production of maleylacetic acid, and, optionally,
- isolating and/or purifying the maleylacetic acid from the cell and/or the culture medium.
In an embodiment, the invention provides a method for the production of protocatechuic acid, comprising the steps of:
- contacting a lysate of a cell according to the invention with a substrate comprising at least one of protocatechuic aldehyde, benzoic acid, benzaldehyde, benzyl alcohol, p-anisic acid, p-anisaldehyde, p-anisyl alcohol, p-hydroxybenzoic acid, p-hydroxybenzaldehyde, p-hydroxybenzyl alcohol, m-hydroxybenzoic acid, p-coumaric acid, caffeic acid and cinnamyl alcohol, under conditions conducive to the production of protocatechuic acid, and, optionally,
- isolating and/or purifying the protocatechuic acid from the reaction medium.

In an embodiment, the invention provides a method for the production of hydroxyquinol, comprising the steps of:
- contacting a lysate of a cell according to the invention with a substrate comprising at least one of protocatechuic aldehyde, benzoic acid, benzaldehyde, benzyl alcohol, p-anisic acid, p-anisaldehyde, p-anisyl alcohol, p-hydroxybenzoic acid, p-hydroxybenzaldehyde, p-hydroxybenzyl alcohol, m-hydroxybenzoic acid, p-coumaric acid, caffeic acid and cinnamyl alcohol, under conditions conducive to the production of hydroxyquinol, and, optionally,
- isolating and/or purifying the hydroxyquinol from the reaction medium.

The features of this aspect are preferably as described elsewhere herein. This method may hereinafter alternately be referred to as a process according to the invention. Suitable cell culturing methods for use in a process according to the invention are known to the skilled person and are discussed, for example, in van't Riet, K. and Tramper, J., 1st edition, Basic Bioreactor Design, CRC Press, NY, 1991. Such methods include, but are not limited to, submerged fermentation in liquid media, surface fermentation on liquid media and solid-state fermentations. Cell culturing may, for example, be performed by cultivation in micro-titer plates, shake-flasks, small-scale benchtop bioreactors, medium-scale bioreactors and/or large-scale bioreactors in a laboratory and/or an industrial setting. Suitable cell culturing modes include, but are not limited to, continuous, batch and/or fed-batch fermentation as well as their combinations. In an embodiment, cell culturing is performed using continuous fermentation. In a preferred embodiment, cell culturing is performed using batch fermentation. In a more preferred embodiment, cell culturing is performed using fed batch fermentation.
In the context of the invention, "culture medium", hereinafter alternately referred to as "growth medium", can be interpreted to encompass cases wherein the cultured cells are absent as well as cases wherein the cultured cells are present in the culture medium. "Culture broth" refers to the culture medium wherein the cultured cells are present. "Culture supernatant" refers to the culture medium wherein the cultured cells are absent.
Cell culturing as part of the process of the invention can be performed under conditions conducive to the production of the relevant compounds, which are known to the skilled person. Such conditions depend not only on the chemical composition of the culture medium but also on other process parameters including culture duration, temperature, O₂ levels in the culture broth and/or headspace, CO₂ levels in the culture broth and/or headspace, pH, agitation speed, hydrostatic pressure and the like. Cell culturing can take place using a culture medium comprising suitable nutrients, such as carbon and nitrogen sources and additional compounds such as inorganic salts and vitamins, using procedures known in the art (see, e. g. Bennett, W. and Lasure, L., 1st edition, More Gene Manipulations in Fungi, Academic Press, CA, 1991). Suitable growth media are available from commercial suppliers or may be prepared using published compositions (e.g. in catalogues of the Centraalbureau Voor Schimmelcultures collection (CBS)). An example of a suitable growth medium is a complete medium, comprising about 6 g/L NaNO₃, 1.5g g/L KH₂PO₄, 0.5 g/L KCI, 0.5 g/L MgSO₄*7H₂O, 0.2 mL/L trace elements (stock solution containing: 10 g/L ethylenediamine tetra-acetic acid, 4.4 g/L ZnSO₄*7H₂O, 1.47 g/L CaCl₂.2H₂O, 1.01 g/L MnCl₂*4H₂O, 1.0 g/L FeSO₄*7H₂O, 0.22 g/L (NH₄)₆MO₇O₂₄*4H₂O, 0.315 g/L CuSO₂*5H₂O, 0.32g/L COCl₂*6H₂O), 0.2% (mass/vol) tryptone, 0.1% (mass/vol) yeast extract, 0.1% (mass/vol) casamino acids, 0.05% (mass/vol) yeast RNA, and a suitable carbon source, preferably glucose, fructose or an aromatic compound, more preferably an aromatic compound.
The exact composition of the growth medium and the values of culture process parameters are not critical features of the invention. Any growth medium composition may be contemplated, as long as it allows for growth of the host cell and production of a compound involved in benzoic acid-related pathways including, but not limited to, triple-substituted benzenes such as protocatechuic acid and hydroxyquinol. The growth medium will typically comprise a carbon source to be used for the growth of the cultured cell and a substrate to be converted to the relevant compound. The substrate may be the same carbon source as the one used for the growth of the cell and/or may be a separate carbon source which is additionally present in the growth medium, preferably it is the same carbon source. Examples of suitable carbon sources known in the art include simple sugars such as glucose, maltose, sucrose, xylose, arabinose, complex sugars such as maltodextrins, hydrolysed starch, starch, molasses, and second-generation feedstocks such as (agricultural) biomass, commercial organic matter, building and demolition waste, municipal solid waste, virgin biomass such as waste paper and garden waste, or non-virgin biomass, as described elsewhere herein,
Carbon sources such as organic acids, aldehydes, ketones, esters and alcohols may also be contemplated. Preferred carbon sources that may be contemplated are protocatechuic aldehyde, benzoic acid, benzaldehyde, benzyl alcohol, p-anisic acid, p-anisaldehyde, p-anisyl alcohol, p-hydroxybenzoic acid, p-hydroxybenzaldehyde, p-hydroxybenzyl alcohol, m-hydroxybenzoic acid, p-coumaric acid, caffeic acid, cinnamic acid and cinnamyl alcohol, preferably at least one of benzaldehyde, p-hydroxybenzaldehyde and 3,4-dihydroxybenzaldehyde. The skilled person understands that said sources may be added externally to the growth media or may already be present. Said sources may be added or may be already present in a mixture. The use of growth media comprising combinations of multiple different carbon sources may also be contemplated in the process of the invention. Such media could, as a non-limiting example, combine more oxidized carbon sources such as organic acids with more reduced carbon sources such as alcohols. Examples of suitable nitrogen sources known in the art include soy bean meal, corn steep liquor, yeast extract, whey protein, egg protein, casein hydrolysate, urea, ammonia, ammonium salts and nitrate salts. Examples of additional suitable compounds known in the art include phosphate, sulphate, metals such as magnesium, trace elements and vitamins. The exact growth medium requirements will vary based on the host cell, e.g. between yeasts, bacteria and filamentous fungi, and the targeted compound to be produced; said requirements will be known to the skilled person. Accordingly, the growth medium may be a complete (rich) medium or a minimal medium. The growth medium may be a complex medium or a defined medium. In a preferred embodiment, the culture medium is a defined medium, more preferably it is a minimal medium, most preferably it is a minimal medium comprising about 6 g/L NaNO₃, 1.5g g/L KH₂PO₄, 0.5 g/L KCI, 0.5 g/L MgSO₄*7H₂O, 0.2 mL/L trace elements (stock solution containing: 10 g/L ethylenediamine tetra-acetic acid, 4.4 g/L ZnSO₄*7H₂O, 1.47 g/L CaCl₂.2H₂O, 1.01 g/L MnCl₂*4H₂O, 1.0 g/L FeSO₄*7H₂O, 0.22 g/L (NH₄)6MO₇O₂₄*4H₂O, 0.315 g/L CuSO₂*5H₂O, 0.32 g/L COCl₂*6H₂O), and a suitable carbon source, preferably glucose, fructose or an aromatic compound, more preferably an aromatic compound.
Similar to the composition of the growth medium, process parameters can be assigned any value, as long as it allows for growth of the host cell and production of a compound involved in benzoic acid-related pathways including, but not limited to, triple-substituted benzenes such as protocatechuic acid and hydroxyquinol. Typically, said values will differ based on the host cell that is being cultured and will be known to the skilled person.
In a preferred embodiment the process according to the invention is an oxygen-limited or aerobic process, meaning that cell culturing is performed under oxygen-limited or aerobic conditions, more preferably the process is oxygen-limited. Oxygen-limited conditions, also known as a micro-aerobic conditions, are culture conditions in which the oxygen consumption is limited by the availability of oxygen. The degree of oxygen limitation is determined by the amount and composition of the ingoing gas flow as well as the actual mixing/mass transfer properties of the fermentation equipment used. Preferably, under oxygen-limited conditions in a liquid culture, the rate of oxygen consumption is at least about 5.5, more preferably at least about 6 and even more preferably at least about 7 mmol/L/h. Aerobic conditions are culture conditions in which the oxygen consumption is not limited by the availability of oxygen.
Cell culturing is performed at a temperature value that is optimal for the cell. When yeasts or fungal host cells are cultured, the fermentation process is preferably performed at a temperature range of 20-42 °C, more preferably at a temperature range of 22-38 °C, even more preferably at a temperature range of 25-35 °C, most preferably at a temperature range of 28-32 °C. In some most preferred embodiments, a temperature value of about 30 °C is used.
Cell culturing is performed at a pH value that is optimal for the cell. In some embodiments, the culture pH value is about pH 2.5, about pH 3.0, about pH 3.5, about pH 4.0, about pH 4.5, about pH 5, about pH 5.5, about pH 6, about pH 6.5, about pH 7, about pH 7.5, about pH 8.0, about pH 8.5, about pH 9. In preferred embodiments, the pH range is from about pH 3.0 to about pH 9, more preferably from about pH 3.5 to pH 6.5, most preferably from about 5.5 to about 6.3. In some most preferred embodiments, the pH is about 6, such as in the range 5.8 to 6.
As a non-limiting example, a temperature of about 30 °C, an agitation speed of about 250 rpm and a pH value of about 6 can be used as process parameters when shake-flask cultivation is performed.
Cell culturing may also be performed by implementation of a multiple step, preferably a two-step, culture method. For example, a production step of a triple-substituted benzene may be preceded by a biomass growth step, wherein only limited production or no production is taking place. The different steps may be carried out using different culture modes and/or different growth media and/or different culture process parameter values, depending on the goal of each step and/or the cultured cell. The biomass during the production step may or may not be actively growing.
Accordingly, in a preferred embodiment, *Aspergillus niger* cells according to the invention are cultured. The starting cells can be fresh or originate from frozen samples. Starting cells can originate from mycelia, protoplasts or spores, preferably from spores. The starting cells can originate from cultures grown on rich medium or minimal medium. In a more preferred embodiment, *Aspergillus niger* spores are inoculated, preferably in shake flasks containing a minimal medium, more preferably a minimal medium as described in the Examples, with about 2% fructose, preferably at a concentration of about 2 x 10⁸ spores/mL and incubated for about 16h in a rotary shaker at a temperature of about 30 °C and agitation speed of about 250 rpm. Freshly germinated mycelia are harvested, preferably on Miracloth (supplied by Merck KGaA, Darmstadt, Germany), and washed, preferably with a minimal medium, more preferably with a minimal medium as described in the Examples. Equal portions of mycelia are transferred, preferably to shake flasks containing about 10 mL of a minimal medium, more preferably a minimal medium as described in the Examples, and about 2 mM of an aromatic compound, preferably selected from benzoic acid, cinnamic acid, ferulic acid, protocatechuic aldehyde, caffeic acid, p-coumaric acid, p-hydroxybenzoic acid, p-hydroxybenzaldehyde, protocatechuic acid and vanillic acid. The cultures are incubated in a rotary shaker, preferably for about 24 hours at a temperature of about 30 °C and an agitation speed of about 250 rpm.
In the context of the process of the invention, the produced compound may optionally be isolated and/or purified from the cell and/or the culture medium and/or the culture headspace. The relevant downstream processing technology that may be suitable for use is not a critical feature of the invention and will depend on whether the produced compound is accumulated within the cultured cells or excreted. Said processing technology and the associated choice will be known to the skilled person and is discussed, for example, in Wesselingh, J.A and Krijgsman, J., 1st edition, Downstream Processing in Biotechnology, Delft Academic Press, NL, 2013. In a non-limiting example of a recovery process, the biomass is separated from the culture medium using e.g. centrifugation or filtration. If the produced compound is accumulated within the cells, it can then be isolated and/or purified from the biomass. If it is excreted, it can be recovered from the cell-free medium or, if the biomass separation step is skipped, directly from the culture broth. Isolation and/or purification may be performed according to any conventional isolation or purification methodology known in the art including, but not limited to, alteration of pH, solvent extraction, dialysis, cell filtration and ultrafiltration, concentration, lyophilisation and the like. In a preferred embodiment, the produced compound is isolated and/or purified from the culture medium. This may be realized continuously with the production process or subsequently to it.
In an embodiment, a method is provided, wherein the method is for the production of protocatechuic acid, wherein the method comprises culturing a host cell comprising a genetic modification that reduces or eliminates the expression of a functional enzyme with protocatechuic hydroxylase (Phy) activity as defined herein, as compared to a corresponding wild type cell lacking the genetic modification, under conditions conducive to the production of protocatechuic acid, and, optionally, isolating and/or purifying the protocatechuic acid from the cell and/or the culture medium.
In an embodiment, a method is provided, wherein the method is forthe production of hydroxyquinol, wherein the method comprises culturing a host cell comprising a genetic modification that increases the expression of a functional enzyme with protocatechuic hydroxylase (Phy) activity as defined herein, as compared to a corresponding wild type cell lacking the genetic modification, under conditions conducive to the production of hydroxyquinol, and, optionally, isolating and/or purifying the hydroxyquinol from the cell and/or the culture medium.

In an embodiment, a method is provided, wherein the method is for the production of protocatechuic acid, wherein the method comprises culturing a host cell comprising a genetic modification that reduces or eliminates the expression of a functional enzyme with protocatechuate 3,4-dioxygenase (Prc) activity as defined herein, as compared to a corresponding wild type cell lacking the genetic modification, under conditions conducive to the production of protocatechuic acid, and, optionally, isolating and/or purifying the protocatechuic acid from the cell and/or the culture medium.
In an embodiment, a method is provided, wherein the method is for the production of 3-carboxy-*cis*,*cis*-muconic acid, wherein the method comprises culturing a host cell comprising a genetic modification that increases the expression of a functional enzyme with protocatechuate 3,4-dioxygenase (Prc) activity as defined herein, as compared to a corresponding wild type cell lacking the genetic modification, under conditions conducive to the production of 3-carboxy-*cis*,*cis*-muconic acid, and, optionally, isolating and/or purifying the 3-carboxy-*cis*,*cis*-muconic acid from the cell and/or the culture medium.
In an embodiment, a method is provided, wherein the method is forthe production of hydroxyquinol, wherein the method comprises culturing a host cell comprising a genetic modification that reduces or eliminates the expression of a functional enzyme with hydroxyquinol 1,2-dioxygenase (Hqd) activity as defined herein, as compared to a corresponding wild type cell lacking the genetic modification, under conditions conducive to the production of hydroxyquinol, and, optionally, isolating and/or purifying the hydroxyquinol from the cell and/or the culture medium.
In an embodiment, a method is provided, wherein the method is for the production of maleylacetic acid, wherein the method comprises culturing a host cell comprising a genetic modification that increases the expression of a functional enzyme with hydroxyquinol 1,2-dioxygenase (Hqd) activity as defined herein, as compared to a corresponding wild type cell lacking the genetic modification, under conditions conducive to the production of maleylacetic acid, and, optionally, isolating and/or purifying the maleylacetic acid from the cell and/or the culture medium.

In an embodiment, a method is provided, wherein the method is for the production of protocatechuic acid, wherein the method comprises culturing a host cell comprising a genetic modification that reduces or eliminates the expression of a functional enzyme with protocatechuic hydroxylase (Phy) activity and a further genetic modification that reduces or eliminates the expression of a functional enzyme with protocatechuate 3,4-dioxygenase (Prc) activity as defined herein, as compared to a corresponding wild type cell lacking the genetic modifications, under conditions conducive to the production of protocatechuic acid, and, optionally, isolating and/or purifying the protocatechuic acid from the cell and/or the culture medium, wherein the functional enzyme with protocatechuate 3,4-dioxygenase (Prc) activity has at least 60%, preferably at least 80%, more preferably at least 90%, most preferably 100% sequence identity with SEQ ID NO: 4, or is encoded by a polynucleotide which has at least 60%, preferably at least 80%, more preferably at least 90%, most preferably 100% sequence identity with SEQ ID NO: 5. Preferably, the functional enzyme with protocatechuate 3,4-dioxygenase (Prc) activity is PrcA from *Aspergillus niger,* more preferably from *Aspergillus niger* CBS 120.49 or a derivative thereof. Preferably, said production and/or substrate conversion is obtained after 24 hours of culture.

In an embodiment, a method is provided, wherein the method is for the production of protocatechuic acid, wherein the method comprises culturing a filamentous fungal host cell, preferably an *Aspergillus niger* cell, wherein the host cell comprises a genetic modification that reduces or eliminates the expression of a functional enzyme with protocatechuic hydroxylase (Phy) activity as defined herein and further comprises a genetic modification that reduces or eliminates the expression of a functional enzyme with protocatechuate 3,4-dioxygenase (PrcA) activity, as compared to a corresponding wild type cell lacking the genetic modifications, wherein the host cell is capable of converting at least 40% of a substrate as defined herein into protocatechuic acid, under conditions conducive to the production of protocatechuic acid, and, optionally, isolating and/or purifying the protocatechuic acid from the cell and/orthe culture medium. Preferably, said substrate conversion is obtained after 24 hours of culture.
In an embodiment, a method is provided, wherein the method is forthe production of hydroxyquinol, wherein the method comprises culturing a host cell comprising a genetic modification that reduces or eliminates the expression of a functional enzyme with protocatechuate 3,4-dioxygenase (Prc) activity and a further genetic modification that increases the expression of a functional enzyme with protocatechuic hydroxylase (Phy) activity as defined herein, as compared to a corresponding wild type cell lacking the genetic modifications, under conditions conducive to the production of hydroxyquinol, and, optionally, isolating and/or purifying the hydroxyquinol from the cell and/or the culture medium. Optionally and preferably, the cultured cell further comprises a genetic modification that reduces or eliminates the expression of a functional enzyme with hydroxyquinol 1,2-dioxygenase (Hqd) activity as defined earlier herein.

In an embodiment, a method is provided, wherein the method is for the production of 3-carboxy-cis,cis-muconic acid, wherein the method comprises culturing a host cell comprising a genetic modification that reduces or eliminates the expression of a functional enzyme with protocatechuic hydroxylase (Phy) activity and a further genetic modification that increases the expression of a functional enzyme with protocatechuate 3,4-dioxygenase (Prc) activity as defined herein, as compared to a corresponding wild type cell lacking the genetic modifications, under conditions conducive to the production of 3-carboxy-cis,cis-muconic acid, and, optionally, isolating and/or purifying the 3-carboxy-cis,cis-muconic acid from the cell and/or the culture medium.

In an embodiment, a method is provided, wherein the method is for the production of maleylacetic acid, wherein the method comprises culturing a host cell comprising a genetic modification that increases the expression of a functional enzyme with protocatechuic hydroxylase (Phy) activity and a further genetic modification that increases the expression of a functional enzyme with hydroxyquinol 1,2-dioxygenase (Hqd) activity as defined herein, as compared to a corresponding wild type cell lacking the genetic modifications, under conditions conducive to the production of maleylacetic acid, and, optionally, isolating and/or purifying the maleylacetic acid from the cell and/or the culture medium.

In a preferred embodiment, the cultured host cells used in the method of the invention are immobilized. Immobilization of cells may be achieved by any means known to the skill person as discussed in standard handbooks such as Guisan, J.M., Bolivar, J.M., López-Gallego, F., Rocha-Martin, J. (Eds.), Immobilization of Enzymes and Cells: Methods and Protocols, Springer US, USA, 2020. Typically, the host cells can be immobilized to a semi-solid or solid support by three different methods. The first method involves polymerizing or solidifying a spore- or cell-containing solution. Examples of polymerizable or solidifyable solutions include alginate, A-carrageenan, chitosan, polyacrylamide, polyacrylamide-hydrazide, agarose, polypropylene, polyethylene glycol, dimethyl acrylate, polystyrene divinyle benzene, polyvinyl benzene, polyvinyl alcohol, epoxy carrier, cellulose, cellulose acetate, photocrosslinkable resin, prepolymers, urethane, and gelatin. The second method involves cell adsorption onto a support. Examples of such supports include bone char, cork, clay, resin, sand porous alumina beads, porous brick, porous silica, celite, orwood chips. The host cells can colonize the support and form a biofilm. The third method involves the covalent coupling of the host cells to a support using chemical agents like glutaraldehyde, o-dianisidine (U.S. Pat. No. 3,983,000), polymeric isocyanates (U.S. Pat. No. 4,071,409), silanes (U.S. Pat. Nos. 3,519,538 and 3,652,761), hydroxyethyl acrylate, transition metal-activated supports, cyanuric chloride, sodium periodate, toluene, and the like. Cultured host cells can be immobilized in any phase of their growth, for example after a desired cell density in the culture has been reached. Suitable culture modes and/or different culture process parameter values will be known to the skilled person and are discussed in standard handbooks, such as Colin R. Phillips C.R., Poon Y. C., Immobilization of Cells: In Biotechnology Monographs book series (Biotechnology, volume 5), Springer, Berlin, Germany, 1988; Tampion J., Tampion M. D., Immobilized Cells: Principles and Applications, Cambridge University Press, UK, 1987. Preferably, immobilized cells are cultured in packed bed bioreactors, also known as plug-flow bioreactors, or expanded (fluidized) bed bioreactors. Suitable growth media and/or product isolation and/or purification methods are further discussed elsewhere herein.

The compounds produced by a cell according to the invention and by a method according to the invention have specific advantageous properties, such as being free of trace impurities that remain after chemical synthesis and being "natural-like". Accordingly, in a preferred embodiment there is provided for triple-substituted-benzene, preferably selected from protocatechuic acid and hydroxyquinol, obtainable by a process according to the invention. In another preferred embodiment, there is provided for 3-carboxy-cis,cis-muconic acid obtainable by a process according to the invention. In still another preferred embodiment, there is provided for maleylacetic acid obtainable by a process according to the invention. A compound obtained using a process according to the invention can conveniently be used in a product. Accordingly, there is provided for a pharmaceutical composition, a flavour composition, a fragrance composition, a cosmetic composition, or a food composition comprising a substituted benzene obtainable by a process according to the invention.

### General information

Unless stated otherwise, all technical and scientific terms used herein have the same meaning as customarily and ordinarily understood by a person of ordinary skill in the art to which this invention belongs, and read in view of this disclosure.

"Sequence identity" or "identity" in the context of amino acid- or nucleic acid-sequence is herein defined as a relationship between two or more amino acid (peptide, polypeptide, or protein) sequences or two or more nucleic acid (nucleotide, polynucleotide) sequences, as determined by comparing the sequences. In the art, "identity" also means the degree of sequence relatedness between amino acid or nucleotide sequences, as the case may be, as determined by the match between strings of such sequences. Sequence identity is preferably assessed over the entire length of the shorter of the two sequences under comparison.

Within the present invention, sequence identity with a particular sequence preferably means sequence identity over the entire length of said particular polypeptide or polynucleotide sequence.

Preferred methods to determine identity are designed to give the largest match between the sequences tested. Methods to determine identity and similarity are codified in publicly available computer programs. Preferred computer program methods to determine identity and similarity between two sequences include e.g. the GCG program package (Devereux, J., et al., Nucleic Acids Research 12 (1): 387 (1984)), BestFit, BLASTP, BLASTN, and FASTA (Altschul, S. F. et al., J. Mol. Biol. 215:403-410 (1990). The BLAST X program is publicly available from NCBI and other sources (BLAST Manual, Altschul, S., et al., NCBI NLM NIH Bethesda, MD 20894; Altschul, S., et al., J. Mol. Biol. 215:403-410 (1990). The well-known Smith Waterman algorithm may also be used to determine identity.

Preferred parameters for polypeptide sequence comparison include the following: Algorithm: Needleman and Wunsch, J. Mol. Biol. 48:443-453 (1970); Comparison matrix: BLOSUM62 from Hentikoff and Hentikoff, Proc. Natl. Acad. Sci. USA. 89:10915-10919 (1992); Gap Penalty: 12; and Gap Length Penalty: 4. A program useful with these parameters is publicly available as the "Ogap" program from Genetics Computer Group, located in Madison, WI. The aforementioned parameters are the default parameters for amino acid comparisons (along with no penalty for end gaps).

Preferred parameters for nucleic acid comparison include the following: Algorithm: Needleman and Wunsch, J. Mol. Biol. 48:443-453 (1970); Comparison matrix: matches=+10, mismatch=0; Gap Penalty: 50; Gap Length Penalty: 3. Available as the Gap program from Genetics Computer Group, located in Madison, Wis. Given above are the default parameters for nucleic acid comparisons.

Moderate conditions are herein defined as conditions that allow a nucleic acid sequences of at least 50 nucleotides, preferably of about 200 or more nucleotides, to hybridize at a temperature of about 45° C in a solution comprising about 1 M salt, preferably 6×SSC or any other solution having a comparable ionic strength, and washing at room temperature in a solution comprising about 1 M salt, preferably 6×SSC or any other solution having a comparable ionic strength. Preferably, the hybridization is performed overnight, i.e. at least for 10 hours, and preferably washing is performed for at least one hour with at least two changes of the washing solution. These conditions will usually allow the specific hybridization of sequences having up to 50% sequence identity. The person skilled in the art will be able to modify these hybridization conditions in order to specifically identify sequences varying in identity between 50% and 90%.

In this document and in its claims, the verb "to comprise" and its conjugations is used in its non-limiting sense to mean that items following the word are included, but items not specifically mentioned are not excluded. In addition, the verb "to consist" may be replaced by "to essentially consist of" meaning that a composition, medium and/or substrate as described herein may comprise additional component(s) than the ones specifically identified, said additional component(s) not altering the unique characteristic of the invention.

In addition, reference to an element by the indefinite article "a" or "an" or the definite article "the" does not exclude the possibility that more than one of the element is present, unless the context clearly requires that there be one and only one of the elements. The indefinite article "a" or "an" and the definite article 'the" thus usually mean "at least one".

The word "about" or "approximately" when used in association with a numerical value (e.g. about 10) preferably means that the value may be the given value (of 10) more or less 10% of the value. Each nucleotide sequence or amino acid sequence described herein by virtue of its identity percentage with a given nucleotide sequence or amino acid sequence respectively has in a further preferred embodiment an identity of at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% with the given nucleotide or amino acid sequence, respectively.

All patent and literature references cited in the present specification are hereby incorporated by reference in their entirety.

### Examples

### Example 1 - Production of recombinant PhyA

A full-length *phyA* was synthesized based on the reference *Aspergillus niger* sequence (NRRL3_4569) in pET23b (also known as pET-23a(+) 3.7kb) containing a C-terminal hexa His-tag (Genscript Biotech, Leiden, the Netherlands). The pET23b-p*hyA* plasmid was used to transform the *E. coli* protein production strain BL21 (DE3) (New England Biolabs, Ipswich, MA). *E. coli* BL-21 DE3 pET23b-*phyA* was grown overnight in LB medium with 50 µg/mL ampicillin at 37°C, 200 rpm. After incubation, 400 µL of the culture was transferred to a 1 L flask containing 400 mL LB medium with 50 µg/mL ampicillin and incubated at 37°C, 200 rpm until the OD600 of 0.4-0.8 was reached. Then, isopropyl-p-D-thiogalactoside (IPTG) was added, with a final concentration of 100 µM, to the culture and further incubated for 24 hours at 16°C, 200 rpm. The culture was centrifuged at 3.200 × g, 4°C for 10 min. The supernatant was discarded, the pellet was dissolved in 20 mL BugBuster Protein Extraction Reagent (Novagen), containing 1 KU Lysozyme/ml (Sigma-Aldrich), 25 U Benzonase^{®} Nuclease and complete^{™}, EDTA-free Protease Inhibitor Cocktail (Roche) and was incubated for 20 min at 4°C, shaking. The cell debris was centrifuged at 4°C and the supernatant containing the soluble fraction of proteins was frozen for further use.

### Example 2 - Conversion of protocatechuic acid to hydroxyquinol by PhyA

To confirm the enzymatic activity of PhyA, *E. coli* strains that produce PhyA were created. Cell free extracts of three independent recombinant strains (PhyA.1, PhyA.2 and PhyA.3) and a strain harboring the pET-23 plasmid without insert was collected and tested for activity on protocatechuic acid. The reaction mixture contained 100 µM Mcllvaine buffer, pH 7.0, (Mcllvaine, 1921, J Biol Chem 49: 183-186) 100 µM protocatechuic acid and 40 µL cell free extract in a total volume of 1 mL. Reactions were incubated for 1 h at 30 °C and were stopped by incubation at 80 °C for 10 minutes. All reactions were analyzed with HPLC using a Dionex ICS-5000+ chromatography system, equipped with an Acclaim MixedMode WAX-1 LC Column (3×150 mm) and a UV detector (225, 250 or 280 nm), said system being supplied by Thermo Scientific, with the chromatographic separation being carried out according to manufacturer's recommendation using isocratic elution (30°C; flow rate: 0.25 mL/min) with 25 mM potassium monophosphate, 0.8 mM pyrophosphate, pH 6.0, in 50% acetonitrile, as described in Dilokpimol et al. 2017.

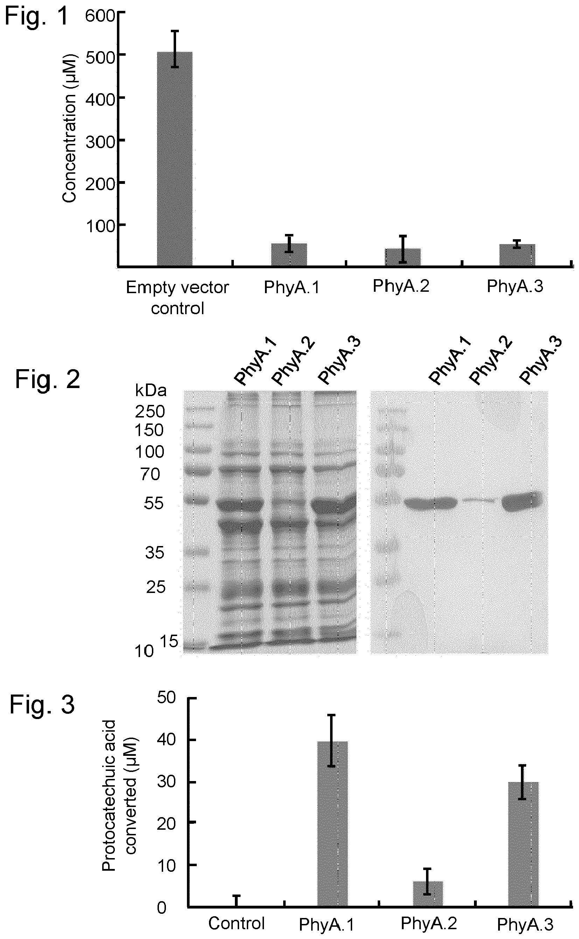

The protocatechuic acid concentration of the reactions containing PhyA was significantly reduced compared to the empty vector control (Figure 1). To further investigate the enzymatic activity of PhyA, it was isolated and purified from three *E. coli PhyA* producing strains using immobilized metal affinity chromatography. The soluble fraction of proteins was loaded to a HisTrap FF 1 mL column coupled with the ÄKTA start system (GE Healthcare Life Sciences, Uppsala, Sweden). The column was equilibrated with a buffer containing 20 mM HEPES, 0.4 M NaCl, and 20 mM imidazole, pH 7.5, at a flow rate of 1 mL/min. After washing with the equilibrating buffer, the protein was eluted with 5 mL of 20 mM HEPES, 0.4 M NaCl, and 400 mM imidazole, pH 7.5. Fractions containing the enzymes, confirmed by SDS-PAGE, were pooled, concentrated, and buffer-exchanged to 20 mM HEPES, pH 7.0. All purification steps were performed at 4°C.

SDS-PAGE and Western blotting using a mouse monoclonal anti-Histidine Tag antibody (Bio-Rad) raised against the Histidine-tag (Figure 2) was used to visualize the purified fraction. The estimated molecular mass of PhyA-His, 49.3 kDa, corresponded with the expected mass. The purified PhyA-His of the three strains were tested for enzymatic activity on protocatechuic acid (Figure 3). Activity assays towards 100 µM protocatechuic acid were performed in 200 µL reaction mixtures that contained 5 µl purified PhyA, 1 mM NADH and Mcllvaine buffer, pH 7.0. Reactions were incubated for 1 h at 30 °C and were stopped by incubation at 80 °C for 10 minutes. The results confirm the expected activity.

### Example 3 - Construction of genetically modified Aspergillus niger strains

*A. niger* strains used in this example are shown in Table 1. All strains generated herein have been deposited in the Centraalbureau Voor Schimmelcultures collection (CBS) of the Westerdijk Fungal Biodiversity Institute, The Netherlands. Their unique identification numbers are shown in Table 1.

**Table 1. Strains used in this example.**

| **Strain** | **CBS number** | **Genotype** | **Reference** |
|---|---|---|---|
| N593 *ΔkusA* | CBS 138852 | *cspA1, pyrG,* Δ*kusA::amdS* | Meyer et al., 2007 |
| Reference | CBS 145984 | *cspA1, pyrG,* Δ*kusA*::amdS, Δ*pyrG*::*pyrG* | This example |
| Δ*prcA* | CBS 145165 | *cspA1, pyrG,* Δ*kusA::amdS,* Δ*prcA::hph* | Lubbers et al., 2019 |
| Δ*prcA*Δ*pyrG* | CBS 146349 | *cspA1, pyrG,* Δ*kusA::amdS,* Δ*prcA::hph,* Δ*pyrG::pyrG* | This example |
| Δ*hqdA* | CBS 145839 | *cspA1, pyrG,* Δ*kusA::amdS,* Δ*hqdA::pyrG* | Lubbers et al., 2019 |
| Δ*phyA* | CBS 146351 | *cspA1, pyrG,* Δ*kusA::amdS,* Δ*phyA::pyrG* | This example |
| Δ*prcA*Δ*hqdA* | CBS 145840 | *cspA1*, *pyrG,* Δ*kusA::amdS,* Δ*prcA::hph,* Δ*hqdA::pyrG* | Lubbers et al., 2019 |
| Δ*phyA*Δ*prcA* | CBS 146352 | *cspA1*, *pyrG,* Δ*kusA::*amdS, Δ*prcA::hph,* Δ*phyA::pyrG* | This example |

All deletion strains were made through protoplast-mediated transformation of *A. niger* N593 Δ*kusA* by homologous recombination using deletion cassettes containing 900-1000 bp upstream and downstream of the target gene fused to an orotidine 5'-phosphate decarboxylase (*pyrG*) from *Aspergillus oryzae* RIB40 or *hph* from *Escherichia coli.* Δ*prcA*Δ*pyrG* was obtained by replacing the endogenous *pyrG* (NRRL3_3466) of Δ*prcA* with the gene deletion cassette containing *pyrG* from *A. oryzae.*

The protoplast-mediated transformation and purification of the selected transformants was performed as previously described (Kowalczyk et al. 2017). For protoplast preparation, young mycelia from overnight culture were harvested by vacuum filtration, washed with 0.6 M MgSO4 and dried between two sheets of paper. The mycelium was then dissolved in PS buffer (0.2 M sodium phosphate buffer, 0.8 M L-sorbitol, pH6) containing VinoTaste^{®} Pro lysing enzyme (0.5 g enzyme/g of mycelia) and incubated in a rotary shaker at an agitation speed of 100 rpm. When protoplasts were abundantly present, the mixture was filtrated through glass wool and undigested mycelia debris was removed. The protoplasts were collected by centrifugation (10 min, 1811x g, 4 °C), washed twice with ice-cold SC solution (182.2 g L⁻¹ sorbitol, 7.35 g L⁻¹ CaCl2* 2H₂O) and resuspended in SC to a final concentration of 2 * 107 protoplasts/mL. For transformation, 200 µL of fresh protoplast suspension, 20 µL of 0.4 M ATA (aurintricarboxylic acid ammonium salt), 100 µL of 20% PEG-4000 was mixed with 5 µg of deletion cassette DNA and incubated for 10 min. After addition of 1.5 mL 60% PEG-4000 the mixture was incubated for 20 min. Next, 5 mL of 1.2 M sorbitol solution was added and the mixture was incubated for another 10 min. Transformed protoplasts were collected by centrifugation (10 min, 3220 ×g), resuspended in 1 mL 1.2 M sorbitol and were spread evenly over selective plates using a cell spreader. Growing colonies were observed after 4 days. Genomic DNA of two independent transformants per strain was isolated using standard phenol/chloroform extraction. Homologous integrations of the deletion cassette were verified by Southern blotting.

### Example 4 - Double deletion of phyA and prcA results in reduced growth on protocatechuic acid

Spores were obtained by growing the fungi on complete media (CM, 2% (mass/vol) fructose, 6 g/L NaNO₃, 1.5g g/L KH₂PO₄, 0.5 g/L KCI, 0.5 g/L MgSO₄*7H₂O, 0.2 mL/L trace elements (stock solution containing: 50 g/L ethylenediamine tetra-acetic acid, 22 g/L ZnSO₄*7H₂O, 5.54 g/L CaCl₂, 5.06 g/L MnCl₂*4H₂O, 4.99 g/L FeSO₄*7H₂O, 1.10 g/L (NH₄)₆MO₇O₂₄*4H₂O, 1.57 g/L CuSO₄*5H₂O, 1.61 g/L COCl₂*6H₂O), 0.2% (mass/vol) tryptone, 0.1% (mass/vol) yeast extract, 0.1% (mass/vol) casamino acids, 0.05% (mass/vol) yeast RNA) agar (1.5% w/v) plates at 30 °C for four days and were harvested with 10 mL *N*-(2-acetamido)-2-aminoethanesulfonic acid (ACES) buffer. Minimal medium (MM, 6 g/L NaNO₃, 1.5g g/L KH₂PO₄, 0.5 g/L KCI, 0.5 g/L MgSO₄*7H₂O, 0.2 mL/L trace elements (stock solution containing: 10 g/L ethylenediamine tetra-acetic acid, 4.4 g/L ZnSO₄*7H₂O, 1.47 g/L CaCl₂.2H₂O, 1.01 g/L MnCl₂*4H₂O, 1.0 g/L FeSO₄*7H₂O, 0.22 g/L (NH₄)₆MO₇O₂₄*4H₂O, 0.315 g/L CuSO₂*5H₂O, 0.32 g/L COCl₂*6H₂O)) agar (1.5% w/v) plates for growth profile experiments were supplemented with aromatic compounds as sole carbon source, adjusted to pH 6.0, and were inoculated with 2 µL droplets containing 10³ freshly isolated spores. Due to toxicity of the aromatic compounds different concentrations were used for the growth profiles, i.e. 2 mM for benzoic acid, cinnamic acid, ferulic acid and protocatechuic aldehyde and 5 mM for caffeic acid, p-coumaric acid, p-hydroxybenzoic acid, p-hydroxybenzaldehyde, protocatechuic acid and vanillic acid. All aromatic compounds and chemicals were purchased from Sigma Aldrich. Agar plates were incubated at 30 °C for 7 days.

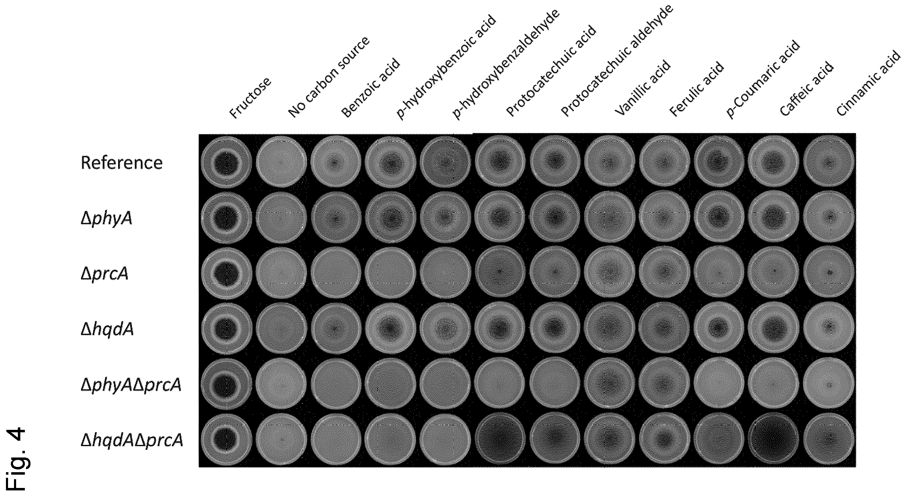

No phenotypes were observed when *ΔphyA* or *ΔhqdA* were grown on the tested aromatic compounds (Figure 4). As observed previously, phenotypes were observed when *prcA* was deleted together with *hqdA* (Lubbers et al., 2019). Here, this was the case for deletion of *phyA* and growth reduction was observed when both *phyA* and *prcA* were deleted compared to *ΔprcA.* In addition, accumulation of hydroxy-1,4-benzoquinone was observed in *ΔprcAΔhqdA* on protocatechuic acid but not by *ΔphyAΔprcA* indicating that the hydroxylation of protocatechuic acid to hydroxyquinol was blocked. Accumulation of hydroxy-1,4-benzoquinone was also observed in cinnamic acid, caffeic acid, p-coumaric acid and protocatechuic aldehyde indicating that these compounds were converted to protocatechuic acid.

### Example 5 - Accumulation of protocatechuic acid by A. niger ΔphyAΔprcA

An accumulation test was performed to verify if the *ΔphyAΔprcA* mutant can be used as a cell factory that accumulates protocatechuic acid from benzoates and cinnamic acids. Ferulic acid, vanillin and veratric acid were selected as negative controls. In addition, bitter almond oil, which mainly consists of benzaldehyde (Remaud et al., 1997) and a mixture of benzaldehyde, benzoic acid, p-hydroxybenzoic acid, p-coumaric acid and caffeic acid were tested.

Pre-cultures of A. *niger* reference and Δ*phyA*Δ*prcA* were made in 1 L flasks containing 200 ml MM with 2% D-fructose and inoculated with 2 x 10⁸ freshly harvested spores. The pre-cultures were incubated at 30 °C, 250 rpm for 16 h. Mycelia was harvested on Miracloth (Sigma-Aldrich) and washed with MM, after which equal portions of mycelia were transferred to 50 mL flasks containing 10 mL MM supplemented with 2 mM aromatic compound. The cultures were incubated at 30 °C, 250 rpm and the supernatant were collected after 24 h. Supernatant samples were diluted 20 times with acetonitrile and analyzed with HPLC. The reduction of aromatic compounds or formation of the products were monitored by HPLC using the same system as described in Example 2.

After 24 hours, all starting substrates were not detected in the supernatant of both strains. Accumulation of protocatechuic acid was observed in the supernatant of Δ*phyA*Δ*prcA* grown in protocatechuic aldehyde, benzoic acid, benzaldehyde, benzyl alcohol, *p*-anisic acid, *p-*anisaldehyde, *p*-hydroxybenzoic acid, *p*-hydroxybenzaldehyde, *p*-hydroxybenzyl alcohol, m-hydroxybenzoic acid, *p*-coumaric acid, caffeic acid, cinnamic acid and cinnamyl alcohol (Table 2). Δ*phyA*Δ*prcA* grown in the aromatic compound mixture resulted in the accumulation of protocatechuic acid. No protocatechuic acid was observed when grown on anethole, cinnamaldehyde, cinnamic acid, ferulic acid, *p*-cresol, vanillin or veratric acid. No accumulated compounds were detected in the supernatant of the reference strain indicating that all aromatic compounds were utilized. These results demonstrate that a *ΔphyAΔprcA* strain is able to accumulate protocatechuic acid when grown on a variety of substrates.

**Table 2. Accumulation of protocatechuic acid by ΔphyAΔprcA. The protocatechuic acid concentrations were determined from the average of biological triplicates. 2 mM was used as starting substrate.**

| | **Reference** | **Δ*phyA*Δ*prcA*** | | |
|---|---|---|---|---|
| **Substrate** | **Compound detected** | **Compound detected** | **Concentration (mM)** | **Conversion rate (%)** |
| Protocatechuic acid | - | Protocatechuic acid | 1.68 ± 0.03 | **84.0** |
| Protocatechuic aldehyde | - | Protocatechuic acid | 1.95 ± 0.11 | 97.5 |
| Benzoic acid | - | Protocatechuic acid | 1.60 ± 0.02 | 80.0 |
| Benzaldehyde | - | Protocatechuic acid | 1.95 ± 0.10 | 97.5 |
| Benzyl alcohol | - | Protocatechuic acid | 1.78 ± 0.15 | 88.9 |
| *p*-anisic acid | - | Protocatechuic acid | 1.66 ± 0.11 | 83.0 |
| *p*-anisaldehyde | - | Protocatechuic acid | 2.09 ± 0.02 | ≥99.0 |
| *p*-anisyl alcohol | - | Protocatechuic acid | 1.63 ± 0.11 | 81.5 |
| *p*-hydroxybenzoic acid | - | Protocatechuic acid | 1.81 ± 0.05 | 90.5 |
| *p*-hydroxybenzaldehyde | - | Protocatechuic acid | 2.10 ± 0.06 | ≥99.0 |
| *p*-hydroxybenzyl alcohol | - | Protocatechuic acid | 1.90 ± 0.02 | 95.0 |
| *m*-hydroxybenzoic acid | - | Protocatechuic acid | 1.69 ± 0.20 | 84.5 |
| *p*-coumaric acid | - | Protocatechuic acid | 1.82 ± 0.05 | 91.0 |
| Caffeic acid | - | Protocatechuic acid | 2.10 ± 0.09 | ≥99.0 |
| Cinnamyl alcohol | - | Protocatechuic acid | 0.83 ± 0.03 | 41.5 |
| Cinnamaldehyde | - | - | - | - |
| Cinnamic acid | - | Protocatechuic acid | 0.28 ± 0.03 | 13.7 |
| Ferulic acid | - | - | - | - |
| Vanillin | - | - | - | - |
| Veratric acid | - | - | - | - |
| *p*-cresol | - | - | - | - |
| Anethole | - | - | - | - |
| Bitter almond oil^ | - | Protocatechuic acid | 1.63 ± 0.04 | 81.5 |
| Mixture* | - | Protocatechuic acid | 10.32 ± 0.31 | ≥99.0 |

| | | | | |
|---|---|---|---|---|
| ^ Bitter almond oil mainly consists of benzaldehyde (96-98%)⁴⁰⁻⁴³, the molecular weight of benzaldehyde was used to obtain approximal 2 mM. * The mixture consisted of 2 mM benzaldehyde, 2 mM benzoic acid, 2 mM p-hydroxybenzoic acid, 2 mM p-coumaric acid and 2 mM caffeic acid. | | | | |

### References

1. Hasunuma, T.; et al., Bioresour. Technol. 2013, 135, 513-522
2. Kawai, Y.; et al., Microb. Cell Fact. 2013, 12 (1), 1-9
3. Lubbers, R. J. M., et al., ACS Sustain. Chem. Eng. 2019, 7 (23), 19081-19089
4. Boschloo, J. G et al., Appl. Microbiol. Biotechnol. 1990, 34, 225-228
5. van Gorcom, R. F. M. et al., Mol. Gen. Genet. 1990, 223 (2), 192-197
6. Faber, B. W et al., Arch. Biochem. Biophys. 2001, 394 (2), 245-254
7. Guo, X et al., Biochem. Eng. J. 2020, 156 (October 2019), 107518
8. Okai, N. et al.,. Appl. Microbiol. Biotechnol. 2016, 100 (1), 135-145
9. Williams, K. M. et al.; Int. J. Mol. Sci. 2012, 13 (3), 3765-3772
10. Weber, C.; et al., Appl. Environ. Microbiol. 2012, 78 (23), 8421-8430
11. Omori, T.; Hatakeyama, K.; Kodama, T. Appl. Microbiol. Biotechnol. 1988, 29 (5), 497-500
12. Meyer, V.; et al., J. Biotechnol. 2007, 128 (4), 770-775
13. Lubbers, R. J. M.; Dilokpimol, A.; Visser, J.; Hilden, K. S.; Mäkelä, M. R.; de Vries, R. P. Appl. Enviorn. Microbiol. 2020
14. Remaud, G.; et al., Agric. Food Chem. 1997, 45 (10), 4042-4048

**Table 3 - brief overview of sequences provided in the sequence listing**

| **SEQ ID NO** | **Description** | **Sequence** |
|---|---|---|
| 1 | PhyA enzyme | *See sequence listing* |
| 2 | PhyAnucleic acid | *See sequence listing* |
| 3 | PhyA vector (pET23b-PhyA) | *See sequence listing* |
| 4 | prcA enzyme | *See sequence listing* |
| 5 | prcA nucleic acid | *See sequence listing* |
| 6 | prcA vector (Pan7.1-PrcA) | *See sequence listing* |
| 7 | HqdA enzyme | *See sequence listing* |
| 8 | HqdA nucleic acid | *See sequence listing* |
| 9 | HqdA vector (Pet28b-HqdA) | *See sequence listing* |
| 10 | PhyA nucleic acid (codon opt) | *See sequence listing* |
| 11 | prcA nucleic acid (codon opt) | *See sequence listing* |
| 12 | HqdA nucleic acid (codon opt) | *See sequence listing* |
| 13 | PhyA vector (with codon opt sequence of PhyA) | *See sequence listing* |
| 14 | deletion cassette CBS 145984 | *See sequence listing* |
| 15 | deletion cassette CBS 146349 | *See sequence listing* |
| 16 | deletion cassette CBS 146351 | *See sequence listing* |
| 17 | deletion cassette CBS 146352 | *See sequence listing* |
| 18 | pyrG-5'F | CGAACTACTTTCGGCATCTTCTAG |
| 19 | pyrG-5'R-pyrg | GCACTTACCTTCGCATTTTCTGGTATATT CGTAAATCCAGAGACGTGCG |
| 20 | pyrG-3'F-pyrg | CCTGTGTTGGTTCTCAGGAACTGCGAAT ATGGATACGGAAGCGGAAACG |
| 21 | pyrG-3'R | CCGCCATGTTAGATCTTGGC |
| 22 | pyrG Forward verification | CGCAAGTTCATTGACATCGGC |
| 23 | pyrG Reverse verification | GGAAGAAATGTTCACACCAGTCG |
| 24 | pyrG-5'F | CGAACTACTTTCGGCATCTTCTAG |
| 25 | pyrG-5'R-pyrg | GCACTTACCTTCGCATTTTCTGGTATATT CGTAAATCCAGAGACGTGCG |
| 26 | pyrG-3'F-pyrg | CCTGTGTTGGTTCTCAGGAACTGCGAAT ATGGATACGGAAGCGGAAACG |
| 27 | pyrG-3'R | CCGCCATGTTAGATCTTGGC |
| 28 | pyrG Forward verification | CGCAAGTTCATTGACATCGGC |
| 29 | pyrG Reverse verification | GGAAGAAATGTTCACACCAGTCG |
| 30 | prcA-5'F | GTCAGCGCCAATCAGTAACC |
| 31 | prcA-5'R-hph | GAAAGAGTCACCGGTCACTGTACAGAGC TCGTGTGGTAAAAGGGGAGAAAGGTAG |
| 32 | prcA-3'F-hph | TGTAAGCGCCCACTCCACATCTCCACTC GAGAAGACTTGCCATGATTTGGTGATAC |
| 33 | prcA-3'R | CTGGCAAGAATGAAATGAGTGAGAG |
| 34 | prcA Forward verification | CTGATCAGACACATCCACGACTTC |
| 35 | prcA Reverse verification | GAGGGGTAGATCTGGTTGATGAC |
| 36 | phyA-5'F | CTGGGTGCCATTGGCAGTAG |
| 37 | phyA-5'R-pyrg | GCACTTACCTTCGCATTTTCTGGTATATT CGGAGTTTGCGCCATGATGATAG |
| 38 | phyA-3'F-pyrg | CCTGTGTTGGTTCTCAGGAACTGCGAAT ATCGAGCTCTTTGCTTCTGTGTAAGC |
| 39 | phyA-3'R | CTTCGATTTGCCACCGTCACC |
| 40 | phyA check F | CACTAGCGTTAGAGGTATGTGC |
| 41 | phyA check R | GTGGATCATGATACGGTAGGC |
| 42 | phyA-5'F | CTGGGTGCCATTGGCAGTAG |
| 43 | phyA-5'R-pyrg | GCACTTACCTTCGCATTTTCTGGTATATT CGGAGTTTGCGCCATGATGATAG |
| 44 | phyA-3'F-pyrg | CCTGTGTTGGTTCTCAGGAACTGCGAAT ATCGAGCTCTTTGCTTCTGTGTAAGC |
| 45 | phyA-3'R | CTTCGATTTGCCACCGTCACC |
| 46 | phyA check F | CACTAGCGTTAGAGGTATGTGC |
| 47 | phyA check R | GTGGATCATGATACGGTAGGC |
| 48 | prcA-5'F | GTCAGCGCCAATCAGTAACC |
| 49 | prcA-5'R-hph | GAAAGAGTCACCGGTCACTGTACAGAGC TCGTGTGGTAAAAGGGGAGAAAGGTAG |
| 50 | prcA-3'F-hph | TGTAAGCGCCCACTCCACATCTCCACTC GAGAAGACTTGCCATGATTTGGTGATAC |
| 51 | prcA-3'R | CTGGCAAGAATGAAATGAGTGAGAG |
| 52 | prcA Forward deletion verification | CTGATCAGACACATCCACGACTTC |
| 53 | prcA Reverse deletion verification | GAGGGGTAGATCTGGTTGATGAC |
| 54 | Deletion cassette prcA | *See sequence listing* |
| 55 | Deletion cassette hqdA | *See sequence listing* |
| 56 | hqdA Forward verification | CGACAACATCACCGAGAACG |
| 57 | hqdA reverse verification | GTGGGTATCTTTGACGGTGC |
| 58 | hqdA-5'F | GACGATGATCCGGATGCCAA |
| 59 | hqdA-5'R-pyrg | GCACTTACCTTCGCATTTTCTGGTATATT GATTAGTGGTGAGGGGTGAGAAG |
| 60 | hqdA-3'F-pyrg | CCTGTGTTGGTTCTCAGGAACTGCGAAT ATGACATTGGTGTTGGCTTCTGC |
| 61 | hqdA-3'R | CCTACTACCTGGACACCGTAC |

## Claims

1. Use of a functional enzyme with protocatechuic hydroxylase (Phy) activity for the production of hydroxyquinol.

2. The use according to claim 1, wherein the enzyme is PhyA from *Aspergillus niger,* preferably from *Aspergillus niger* CBS 120.49 or a derivative thereof.

3. The use according to claim 1 or 2, wherein the PhyA has at least 60%, preferably at least 80%, more preferably at least 90%, most preferably 100% sequence identity with SEQ ID NO: 1, or is encoded by a polynucleotide which has at least 60%, preferably at least 80%, more preferably at least 90%, most preferably 100% sequence identity with SEQ ID NO: 2.

4. A host cell comprising a genetic modification that reduces or eliminates the expression of a functional enzyme with protocatechuic hydroxylase (Phy) activity as defined in any one of claims 1-3, as compared to a corresponding wild type cell lacking the genetic modification.

5. The host cell according to claim 4, wherein the host cell is a microbial cell, preferably a fungal cell, more preferably a filamentous fungal cell.

6. The host cell according to claim 4 or 5, wherein the host cell further comprises a genetic modification that reduces or eliminates the expression of a functional enzyme with protocatechuate 3,4-dioxygenase (Prc) activity as compared to a corresponding wild type cell lacking the genetic modification, preferably wherein the functional enzyme with Prc activity is from *Aspergillus niger,* more preferably from *Aspergillus niger* CBS 120.49, wherein the functional enzyme with Prc activity has at least 60%, preferably at least 80%, more preferably at least 90%, most preferably 100% sequence identity with SEQ ID NO: 4, or is encoded by a polynucleotide which has at least 60%, preferably at least 80%, more preferably at least 90%, most preferably 100% sequence identity with SEQ ID NO: 5.

7. The host cell according to any one of claims 4-6, capable of producing at least 0.8 mM of protocatechuic acid, more preferably capable of producing at least 1.5 mM of protocatechuic acid, or capable of converting at least 40% of a substrate into protocatechuic acid, preferably capable of converting at least 80% of a substrate into protocatechuic acid.

8. The host cell according to claim 7, wherein the substrate comprises at least one of protocatechuic aldehyde, benzoic acid, benzaldehyde, benzyl alcohol, *p*-anisic acid, *p-*anisaldehyde, *p*-anisyl alcohol, *p*-hydroxybenzoic acid, *p*-hydroxybenzaldehyde, *p-*hydroxybenzyl alcohol, *m*-hydroxybenzoic acid, *p*-coumaric acid, caffeic acid, and cinnamyl alcohol.

9. A host cell comprising a genetic modification that increases the expression of a functional enzyme with protocatechuic hydroxylase (Phy) activity as defined in any one of claims 1-3, as compared to a corresponding wild type cell lacking the genetic modification.

10. The host cell according to any one of claims 4-8, wherein the host cell is a filamentous fungal cell, preferably *Aspergillus niger,* wherein the host cell further comprises a genetic modification that reduces or eliminates the expression of a functional enzyme with protocatechuate 3,4-dioxygenase (PrcA) activity as compared to a corresponding wild type cell lacking the genetic modification, and wherein the host cell is capable of converting at least 40% of a substrate into protocatechuic acid.

11. A lysate of a host cell according to any one of claims 4-10.

12. An expression vector comprising a nucleic acid construct comprising a nucleotide sequence that has at least 60%, 65%, 70%, 75%, 80%, 85%, 90%, 95%, 96%, 97%, 98%, 99%, or 100% sequence identity with SEQ ID NO: 2 or 10, or wherein the expression vector consists of a polynucleotide that has at least 60%, 65%, 70%, 75%, 80%, 85%, 90%, 95%, 96%, 97%, 98%, 99%, or 100% sequence identity with SEQ ID NO: 3 or 13.

13. A polypeptide product expressed from the expression vector according to claim 12, wherein the polypeptide preferably has at least 80% sequence identity with SEQ ID NO: 1.

14. A method for the production of protocatechuic acid, comprising the steps of:
- culturing a cell according to any one of claims 4-10 under conditions conducive to the production of protocatechuic acid, and, optionally,
- isolating and/or purifying the protocatechuic acid from the cell and/or the culture medium.

15. A method for the production of protocatechuic acid, comprising the steps of:
- contacting a lysate as defined in claim 11 with a substrate as defined in claim 8 under conditions conducive to the production of protocatechuic acid, and, optionally,
- isolating and/or purifying the protocatechuic acid from the reaction medium.


SEQUENCE LISTING

<110>  Koninklijke Nederlandse Akademie van Wetenschappen

<120>  New enzymes and methods for benzoic acid-related metabolic
       pathways

<130>  P6095870EP

<160>  61

<170>  PatentIn version 3.5

<210>  1
<211>  444
<212>  PRT
<213>  Aspergillus niger

<400>  1

Met Ala Gln Thr Pro Gln Asp Leu Lys Ile Ala Ile Leu Gly Ala Gly
1               5                   10                  15

Met Gly Gly Leu Thr Cys Ala Leu Ala Leu Ala Lys Glu Gly Phe Lys
            20                  25                  30

His Ile Asp Val Tyr Glu Asn Ala His Asp Leu Gly Phe Val Gly Ala
        35                  40                  45

Gly Ile Gln Leu Ala Pro Asn Met Ala Arg Val Leu Asp Arg Leu Gly
    50                  55                  60

Val Trp Lys Pro Ile Glu Ala Glu Ala Val Asn Ile Thr Ser Thr Ser
65                  70                  75                  80

Val Arg Val Gly Ala Ser Asp Ser Glu Leu Ala His Val Pro Leu Glu
                85                  90                  95

Tyr Ile His Asp Thr Tyr Gly Tyr Pro His Met Val Gly His Arg Ser
            100                 105                 110

Ser Leu Ala Gly His Leu Tyr Gln Gly Cys Leu Gln His Pro Asp Gln
        115                 120                 125

Ile His Phe His Phe Ala Thr Thr Ala Ser Asp Thr Thr Phe Gly Pro
    130                 135                 140

Lys Ser Ser Phe Thr Ala Thr Pro Arg Asp Ala Pro Glu Lys Ala Tyr
145                 150                 155                 160

Arg Val Glu Ala Asp Val Leu Leu Ala Cys Asp Gly Ile Lys Ser Gln
                165                 170                 175

Ala Arg Val Ala Met Leu Asp Leu Leu Asn Ile Lys Ala Ser Val Lys
        180                 185                 190

Asp Thr His Gln Ala Ala Tyr Arg Ile Met Ile His Lys Asp Gln Ile
    195                 200                 205

Lys Asp Asp Pro Glu Leu Leu Glu Leu Met Asn Gly Asn Arg Val Thr
    210                 215                 220

Arg Trp Ile Gly Glu Lys Arg His Ile Ile Ala Tyr Pro Val Ser Asn
225                 230                 235                 240

Asn Thr Ile Tyr Asn Leu Ser Thr Thr Gln Pro Asp Thr His Phe Ala
                245                 250                 255

Ala Ala Thr Asn Ala Thr Tyr Thr Thr Arg Gly Asp Lys Ser Val Met
        260                 265                 270

Leu Gly Val Phe Gly Asp Phe Cys Pro Met Val Gln Arg Met Leu Asn
    275                 280                 285

Tyr Val Pro Glu Gly Glu Val Cys Glu Trp Lys Leu Arg Val His Glu
    290                 295                 300

Pro Leu Pro Thr Trp Val His Glu Thr Val Ala Leu Val Gly Asp Ala
305                 310                 315                 320

Cys His Pro Thr Leu Pro His Leu Ala Gln Gly Ala Ala Gln Ala Ile
                325                 330                 335

Glu Asp Gly Gly Val Ile Gly Ala Val Leu Ser Lys Leu Pro Asp Thr
        340                 345                 350

Thr Pro Ala Ser Ile His Lys Ala Leu Lys Val Tyr Glu Lys Val Arg
    355                 360                 365

Lys Asp Arg Ala Tyr Ala Ile Val Glu Leu Ala Ala Ala Ser Gly Arg
    370                 375                 380

Ala Leu His Leu Gly Glu Gly Ala Ala Lys Glu Glu Arg Asp Arg Gln
385                 390                 395                 400

Phe Ala Ala Leu Arg Gln Gly Lys Gly Pro Val Pro Asp Lys Trp Ala
                405                 410                 415

Asp Ala Asp Val Gln Lys Glu Leu Tyr Gly Val Asp Ala Thr Lys Ile
        420                 425                 430

Ala Leu Glu Gln Phe Asp Glu Leu Phe Ala Ser Val
        435                 440

<210>  2
<211>  1335
<212>  DNA
<213>  Aspergillus niger

<400>  2
atggcgcaaa ctccgcaaga ccttaagatt gccattttgg gtgctggcat gggtggcctg      60

acctgcgctt tggctctggc aaaggaaggt ttcaagcaca tcgacgttta tgagaatgca     120

cacgatctgg gcttcgttgg agctggtatc caactggccc cgaacatggc ccgcgtgctg     180

gatcgcctgg gcgtctggaa gcccattgag gccgaagctg ttaacatcac ttccactagc     240

gttagagtcg gtgcctcgga ctcagaattg gcccacgttc cattggaata tatccacgat     300

acctacggat atcctcacat ggttggccac cggtcttccc tcgccggcca cctgtaccaa     360

ggttgcctgc agcaccctga ccagatccac ttccacttcg ccactaccgc cagcgacacc     420

accttcggcc ccaagtcctc cttcactgcc accccccgcg acgcccccga gaaggcctac     480

cgcgtcgagg ccgatgtcct cctcgcctgt gatggcatca agtcccaggc ccgtgtggcc     540

atgctcgatc tcctcaacat caaggccagc gtcaaggaca cccaccaggc cgcctaccgt     600

atcatgatcc acaaggacca gatcaaggac gacccggagc tcctggagct catgaacggc     660

aaccgcgtga ctcgctggat cggcgagaag cgccatatca tcgcctaccc cgtctccaac     720

aacaccatct acaacctctc caccacccaa cccgacactc acttcgccgc cgccaccaat     780

gccacctaca ccacccgcgg tgacaagtcc gtcatgctgg gcgtctttgg cgacttctgc     840

cccatggtcc agcgtatgct gaactacgtc cccgagggtg aagtctgcga gtggaagctg     900

cgtgtccatg agcccctccc cacctgggtg cacgagaccg tcgccctcgt cggtgatgcc     960

tgccacccta ccctgcccca tctcgcccag ggtgccgccc aggccatcga agacggtggt    1020

gtcattggtg ccgtgctctc caagctcccc gataccaccc cggcttccat ccacaaggcc    1080

ctcaaggttt acgagaaggt gcgcaaggac cgcgcctacg ctatcgtcga actggccgcc    1140

gcttccggtc gtgctctgca cctgggtgaa ggtgccgcca aggaggagcg cgatcgccaa    1200

ttcgctgctc tccgccaggg caagggccct gtgcccgaca agtgggcgga tgccgatgtc    1260

cagaaggagc tgtatggcgt tgatgcgact aagatcgcgc tggagcaatt cgacgagctc    1320

tttgcttctg tgtaa                                                     1335

<210>  3
<211>  6744
<212>  DNA
<213>  Artificial Sequence

<220>
<223>  synthetic oligonucleotide


<220>
<221>  misc_feature
<222>  (3593)..(3593)
<223>  n is a, c, g, or t

<220>
<221>  misc_feature
<222>  (3801)..(3801)
<223>  n is a, c, g, or t

<220>
<221>  misc_feature
<222>  (3892)..(3892)
<223>  n is a, c, g, or t

<400>  3
gaattccctt gtatctctac acacaggctc aaatcaataa gaagaacggt tcgtcttttt      60

cgtttatatc ttgcatcgtc ccaaagctat tggcgggata ttctgtttgc agttggctga     120

cttgaagtaa tctctgcaga tctttcgaca ctgaaatacg tcgagcctgc tccgcttgga     180

agcggcgagg agcctcgtcc tgtcacaact accaacatgg agtacgataa gggccagttc     240

cgccagctca ttaagagcca gttcatgggc gttggcatga tggccgtcat gcatctgtac     300

ttcaagtaca ccaacgctct tctgatccag tcgatcatcc gctgaaggcg ctttcgaatc     360

tggttaagat ccacgtcttc gggaagccag cgactggtga cctccagcgt ccctttaagg     420

ctgccaacag ctttctcagc cagggccagc ccaagaccga caaggcctcc ctccagaacg     480

ccgagaagaa ctggaggggt ggtgtcaagg aggagtaagc tccttattga agtcggagga     540

cggagcggtg tcaagaggat attcttcgac tctgtattat agataagatg atgaggaatt     600

ggaggtagca tagcttcatt tggatttgct ttccaggctg agactctagc ttggagcata     660

gagggtcctt tggctttcaa tattctcaag tatctcgagt ttgaacttat tccctgtgaa     720

ccttttattc accaatgagc attggaatga acatgaatct gaggactgca atcgccatga     780

ggttttcgaa atacatccgg atgtcgaagg cttggggcac ctgcgttggt tgaatttaga     840

acgtggcact attgatcatc cgatagctct gcaaagggcg ttgcacaatg caagtcaaac     900

gttgctagca gttccaggtg gaatgttatg atgagcattg tattaaatca ggagatatag     960

catgatctct agttagctca ccacaaaagt cagacggcgt aaccaaaagt cacacaacac    1020

aagctgtaag gatttcggca cggctacgga agacggagaa gccaccttca gtggactcga    1080

gtaccattta attctatttg tgtttgatcg agacctaata cagcccctac aacgaccatc    1140

aaagtcgtat agctaccagt gaggaagtgg actcaaatcg acttcagcaa catctcctgg    1200

ataaacttta agcctaaact atacagaata agataggtgg agagcttata ccgagctccc    1260

aaatctgtcc agatcatggt tgaccggtgc ctggatcttc ctatagaatc atccttattc 1320
gttgacctag ctgattctgg agtgacccag agggtcatga cttgagccta aaatccgccg 1380
cctccaccat ttgtagaaaa atgtgacgaa ctcgtgagct ctgtacagtg accggtgact 1440
ctttctggca tgcggagaga cggacggacg cagagagaag ggctgagtaa taagccactg 1500
gccagacagc tctggcggct ctgaggtgca gtggatgatt attaatccgg gaccggccgc 1560
ccctccgccc cgaagtggaa aggctggtgt gcccctcgtt gaccaagaat ctattgcatc 1620
atcggagaat atggagcttc atcgaatcac cggcagtaag cgaaggagaa tgtgaagcca 1680
ggggtgtata gccgtcggcg aaatagcatg ccattaacct aggtacagaa gtccaattgc 1740
ttccgatctg gtaaaagatt cacgagatag taccttctcc gaagtaggta gagcgagtac 1800
ccggcgcgta agctccctaa ttggcccatc cggcatctgt agggcgtcca aatatcgtgc 1860
ctctcctgct ttgcccggtg tatgaaaccg gaaaggccgc tcaggagctg gccagcggcg 1920
cagaccggga acacaagctg gcagtcgacc catccggtgc tctgcactcg acctgctgag 1980
gtccctcagt ccctggtagg cagctttgcc ccgtctgtcc gcccggtgtg tcggcggggt 2040
tgacaaggtc gttgcgtcag tccaacattt gttgccatat tttcctgctc tccccaccag 2100
ctgctctttt cttttctctt tcttttccca tcttcagtat attcatcttc ccatccaaga 2160
acctttattt cccctaagta agtactttgc tacatccata ctccatcctt cccatccctt 2220
attcctttga acctttcagt tcgagctttc ccacttcatc gcagcttgac taacagctac 2280
cccgcttgag cagacatcac catgtctacc aaccgccgct tcgaccctaa cttcactccc 2340
tatgtggtca actccatggg acccaagacc cccgagcgtg ctcgtgtggt ccttggtgcc 2400
ctgatcagac acatccacga cttcgctcgt gaggttgagc ttacctccgc tgagtggatg 2460
cttggtgttg agttcatcaa ctccattgga aagatcagca ctcccatccg taacgagtgc 2520
caccgtatct gcgatgttat cggtctggaa tcgtaagtcc acacaacagc gatcgcgcca 2580
aactcaaaaa ctaaccttta tctccgcaca gtctcgtcga tgagatcgcc aacaagatcg 2640
tcaccgagga cggtgtctcc cccacctcca acgtcattct cggccccttc tggtctccca 2700
acgctccctt ccgtgagctt ggcgacagca tcatccagga ccccaacccc aacggaaagg 2760
tcacctacat gcacggtgtc ctgaaggaca tggagactgg cgctcccatc gtcggtgccg 2820
tcctcgatat ctggcaggcc tccgccaacg gccagtacga cttccaggac cccaaccaga 2880
gtgagaacaa ccttcgcggc aagttccgct ccaacgagaa gggcgagttc aactggtact 2940
gctaccaccc cactccttac tctctcccca ccgatggtcc cgccggtgtc ctcctcaacc 3000
tcatggaccg ctctcccatg cgtcctgccc acatccacct gatgatcacc caccccgact 3060
acgccaccgt catcaaccag atctacccct ccgacgaccc tcatctcgac atcgactccg 3120
tcttcgccgt caaggacgac ctggttgtcg acttcaagcc caagaccgat gaccccaagg 3180

cccagctcga cctggagtac aacgtcacca tggctctgaa gaagcaccac cccaacccca 3240

actccgctcc cccggtctct tcctttgagc gtttcaacaa ggccagcaag actcaggaga 3300

agttgcacca ccaccaccac cactagggat ccacttaacg ttactgaaat catcaaacag 3360

cttgacgaat ctggatataa gatcgttggt gtcgatgtca gctccggagt tgagacaaat 3420

ggtgttcagg atctcgataa gatacgttca tttgtccaag cagcaaagag tgccttctag 3480

tgatttaata gctccatgtc aacaagaata aaacgcgttt tcgggtttac ctcttccaga 3540

tacagctcat ctgcaatgca ttaatgcatt gactgcaacc tagtaacgcc ttncaggctc 3600

cggcgaagag aagaatagct tagcagagct attttcattt tcgggagacg agatcaagca 3660

gatcaacggt cgtcaagaga cctacgagac tgaggaatcc gctcttggct ccacgcgact 3720

atatatttgt ctctaattgt actttgacat gctcctcttc tttactctga tagcttgact 3780

atgaaaattc cgtcaccagc ncctgggttc gcaaagataa ttgcatgttt cttccttgaa 3840

ctctcaagcc tacaggacac acattcatcg taggtataaa cctcgaaatc anttcctact 3900

aagatggtat acaatagtaa ccatgcatgg ttgcctagtg aatgctccgt aacacccaat 3960

acgccggccg aaactttttt acaactctcc tatgagtcgt ttacccagaa tgcacaggta 4020

cacttgttta gaggtaatcc ttctttctag aagtcctcgt gtactgtgta agcgcccact 4080

ccacatctcc actcgacctg caggcatgca agcttggcac tggccgtcgt tttacaacgt 4140

cgtgactggg aaaaccctgg cgttacccaa cttaatcgcc ttgcagcaca tccccctttc 4200

gccagctggc gtaatagcga agaggcccgc accgatcgcc cttcccaaca gttgcgcagc 4260

ctgaatggcg aatggcgcct gatgcggtat tttctcctta cgcatctgtg cggtatttca 4320

caccgcatat ggtgcactct cagtacaatc tgctctgatg ccgcatagtt aagccagccc 4380

cgacacccgc caacacccgc tgacgcgccc tgacgggctt gtctgctccc ggcatccgct 4440

tacagacaag ctgtgaccgt ctccgggagc tgcatgtgtc agaggttttc accgtcatca 4500

ccgaaacgcg cgagacgaaa gggcctcgtg atacgcctat ttttataggt taatgtcatg 4560

ataataatgg tttcttagac gtcaggtggc acttttcggg gaaatgtgcg cggaacccct 4620

atttgtttat ttttctaaat acattcaaat atgtatccgc tcatgagaca ataaccctga 4680

taaatgcttc aataatattg aaaaaggaag agtatgagta ttcaacattt ccgtgtcgcc 4740

cttattccct tttttgcggc attttgcctt cctgtttttg ctcacccaga aacgctggtg 4800

aaagtaaaag atgctgaaga tcagttgggt gcacgagtgg gttacatcga actggatctc 4860

aacagcggta agatccttga gagttttcgc cccgaagaac gttttccaat gatgagcact 4920

tttaaagttc tgctatgtgg cgcggtatta tcccgtattg acgccgggca agagcaactc 4980

ggtcgccgca tacactattc tcagaatgac ttggttgagt actcaccagt cacagaaaag 5040

catcttacgg atggcatgac agtaagagaa ttatgcagtg ctgccataac catgagtgat 5100

aacactgcgg ccaacttact tctgacaacg atcggaggac cgaaggagct aaccgctttt 5160

ttgcacaaca tgggggatca tgtaactcgc cttgatcgtt gggaaccgga gctgaatgaa 5220

gccataccaa acgacgagcg tgacaccacg atgcctgtag caatggcaac aacgttgcgc 5280

aaactattaa ctggcgaact acttactcta gcttcccggc aacaattaat agactggatg 5340

gaggcggata aagttgcagg accacttctg cgctcggccc ttccggctgg ctggtttatt 5400

gctgataaat ctggagccgg tgagcgtggg tctcgcggta tcattgcagc actggggcca 5460

gatggtaagc cctcccgtat cgtagttatc tacacgacgg ggagtcaggc aactatggat 5520

gaacgaaata gacagatcgc tgagataggt gcctcactga ttaagcattg gtaactgtca 5580

gaccaagttt actcatatat actttagatt gatttaaaac ttcattttta atttaaaagg 5640

atctaggtga agatcctttt tgataatctc atgaccaaaa tcccttaacg tgagttttcg 5700

ttccactgag cgtcagaccc cgtagaaaag atcaaaggat cttcttgaga tccttttttt 5760

ctgcgcgtaa tctgctgctt gcaaacaaaa aaaccaccgc taccagcggt ggtttgtttg 5820

ccggatcaag agctaccaac tctttttccg aaggtaactg gcttcagcag agcgcagata 5880

ccaaatactg tccttctagt gtagccgtag ttaggccacc acttcaagaa ctctgtagca 5940

ccgcctacat acctcgctct gctaatcctg ttaccagtgg ctgctgccag tggcgataag 6000

tcgtgtctta ccgggttgga ctcaagacga tagttaccgg ataaggcgca gcggtcgggc 6060

tgaacggggg gttcgtgcac acagcccagc ttggagcgaa cgacctacac cgaactgaga 6120

tacctacagc gtgagctatg agaaagcgcc acgcttcccg aagggagaaa ggcggacagg 6180

tatccggtaa gcggcagggt cggaacagga gagcgcacga gggagcttcc agggggaaac 6240

gcctggtatc tttatagtcc tgtcgggttt cgccacctct gacttgagcg tcgatttttg 6300

tgatgctcgt caggggggcg gagcctatgg aaaaacgcca gcaacgcggc ctttttacgg 6360

ttcctggcct tttgctggcc ttttgctcac atgttctttc ctgcgttatc ccctgattct 6420

gtggataacc gtattaccgc ctttgagtga gctgataccg ctcgccgcag ccgaacgacc 6480

gagcgcagcg agtcagtgag cgaggaagcg gaagagcgcc caatacgcaa accgcctctc 6540

cccgcgcgtt ggccgattca ttaatgcagc tggcacgaca ggtttcccga ctggaaagcg 6600

ggcagtgagc gcaacgcaat taatgtgagt tagctcactc attaggcacc ccaggcttta 6660

cactttatgc ttccggctcg tatgttgtgt ggaattgtga gcggataaca atttcacaca 6720

ggaaacagct atgaccatga ttac 6744

<210> 4
<211> 315
<212> PRT
<213> Aspergillus niger

<400>  4

Met Ser Thr Asn Arg Arg Phe Asp Pro Asn Phe Thr Pro Tyr Val Val
1               5                   10                  15

Asn Ser Met Gly Pro Lys Thr Pro Glu Arg Ala Arg Val Val Leu Gly
            20                  25                  30

Ala Leu Ile Arg His Ile His Asp Phe Ala Arg Glu Val Glu Leu Thr
        35                  40                  45

Ser Ala Glu Trp Met Leu Gly Val Glu Phe Ile Asn Ser Ile Gly Lys
    50                  55                  60

Ile Ser Thr Pro Ile Arg Asn Glu Cys His Arg Ile Cys Asp Val Ile
65                  70                  75                  80

Gly Leu Glu Ser Leu Val Asp Glu Ile Ala Asn Lys Ile Val Thr Glu
                85                  90                  95

Asp Gly Val Ser Pro Thr Ser Asn Val Ile Leu Gly Pro Phe Trp Ser
            100                 105                 110

Pro Asn Ala Pro Phe Arg Glu Leu Gly Asp Ser Ile Ile Gln Asp Pro
        115                 120                 125

Asn Pro Asn Gly Lys Val Thr Tyr Met His Gly Val Leu Lys Asp Met
    130                 135                 140

Glu Thr Gly Ala Pro Ile Val Gly Ala Val Leu Asp Ile Trp Gln Ala
145                 150                 155                 160

Ser Ala Asn Gly Gln Tyr Asp Phe Gln Asp Pro Asn Gln Ser Glu Asn
                165                 170                 175

Asn Leu Arg Gly Lys Phe Arg Ser Asn Glu Lys Gly Glu Phe Asn Trp
            180                 185                 190

Tyr Cys Tyr His Pro Thr Pro Tyr Ser Leu Pro Thr Asp Gly Pro Ala
        195                 200                 205

Gly Val Leu Leu Asn Leu Met Asp Arg Ser Pro Met Arg Pro Ala His
    210                 215                 220

Ile His Leu Met Ile Thr His Pro Asp Tyr Ala Thr Val Ile Asn Gln
225                 230                 235                 240

Ile Tyr Pro Ser Asp Asp Pro His Leu Asp Ile Asp Ser Val Phe Ala
                245                 250                 255

Val Lys Asp Asp Leu Val Val Asp Phe Lys Pro Lys Thr Asp Asp Pro
            260                 265                 270

Lys Ala Gln Leu Asp Leu Glu Tyr Asn Val Thr Met Ala Leu Lys Lys
        275                 280                 285

His His Pro Asn Pro Asn Ser Ala Pro Pro Val Ser Ser Phe Glu Arg
    290                 295                 300

Phe Asn Lys Ala Ser Lys Thr Gln Glu Lys Leu
305                 310                 315

<210>  5
<211>  948
<212>  DNA
<213>  Aspergillus niger

<400>  5
atgtctacca accgccgctt cgaccctaac ttcactccct atgtggtcaa ctccatggga      60

cccaagaccc ccgagcgtgc tcgtgtggtc cttggtgccc tgatcagaca catccacgac     120

ttcgctcgtg aggttgagct tacctccgct gagtggatgc ttggtgttga gttcatcaac     180

tccattggaa agatcagcac tcccatccgt aacgagtgcc accgtatctg cgatgttatc     240

ggtctggaat ctctcgtcga tgagatcgcc aacaagatcg tcaccgagga cggtgtctcc     300

cccacctcca acgtcattct cggccccttc tggtctccca acgctccctt ccgtgagctt     360

ggcgacagca tcatccagga ccccaacccc aacggaaagg tcacctacat gcacggtgtc     420

ctgaaggaca tggagactgg cgctcccatc gtcggtgccg tcctcgatat ctggcaggcc     480

tccgccaacg gccagtacga cttccaggac cccaaccaga gtgagaacaa ccttcgcggc     540

aagttccgct ccaacgagaa gggcgagttc aactggtact gctaccaccc cactccttac     600

tctctcccca ccgatggtcc cgccggtgtc ctcctcaacc tcatggaccg ctctcccatg     660

cgtcctgccc acatccacct gatgatcacc caccccgact acgccaccgt catcaaccag     720

atctacccct ccgacgaccc tcatctcgac atcgactccg tcttcgccgt caaggacgac     780

ctggttgtcg acttcaagcc caagaccgat gaccccaagg cccagctcga cctggagtac     840

aacgtcacca tggctctgaa gaagcaccac cccaacccca actccgctcc cccggtctct     900

tcctttgagc gtttcaacaa ggccagcaag actcaggaga agttgtaa                  948

<210>  6
<211>  6744
<212>  DNA
<213>  Artificial Sequence

<220>
<223>  synthetic oligonucleotide

<220>
<221>  misc_feature
<222>  (3593)..(3593)
<223>  n is a, c, g, or t

<220>
<221>  misc_feature
<222>  (3801)..(3801)
<223>  n is a, c, g, or t

<220>
<221>  misc_feature
<222>  (3892)..(3892)
<223>  n is a, c, g, or t

<400>  6
gaattccctt gtatctctac acacaggctc aaatcaataa gaagaacggt tcgtcttttt      60

cgtttatatc ttgcatcgtc ccaaagctat tggcgggata ttctgtttgc agttggctga     120

cttgaagtaa tctctgcaga tctttcgaca ctgaaatacg tcgagcctgc tccgcttgga     180

agcggcgagg agcctcgtcc tgtcacaact accaacatgg agtacgataa gggccagttc     240

cgccagctca ttaagagcca gttcatgggc gttggcatga tggccgtcat gcatctgtac     300

ttcaagtaca ccaacgctct tctgatccag tcgatcatcc gctgaaggcg ctttcgaatc     360

tggttaagat ccacgtcttc gggaagccag cgactggtga cctccagcgt ccctttaagg     420

ctgccaacag ctttctcagc cagggccagc ccaagaccga caaggcctcc ctccagaacg     480

ccgagaagaa ctggaggggt ggtgtcaagg aggagtaagc tccttattga agtcggagga     540

cggagcggtg tcaagaggat attcttcgac tctgtattat agataagatg atgaggaatt     600

ggaggtagca tagcttcatt tggatttgct ttccaggctg agactctagc ttggagcata     660

gagggtcctt tggctttcaa tattctcaag tatctcgagt ttgaacttat tccctgtgaa     720

ccttttattc accaatgagc attggaatga acatgaatct gaggactgca atcgccatga     780

ggttttcgaa atacatccgg atgtcgaagg cttggggcac ctgcgttggt tgaatttaga     840

acgtggcact attgatcatc cgatagctct gcaaagggcg ttgcacaatg caagtcaaac     900

gttgctagca gttccaggtg gaatgttatg atgagcattg tattaaatca ggagatatag     960

catgatctct agttagctca ccacaaaagt cagacggcgt aaccaaaagt cacacaacac    1020

aagctgtaag gatttcggca cggctacgga agacggagaa gccaccttca gtggactcga    1080

gtaccattta attctatttg tgtttgatcg agacctaata cagcccctac aacgaccatc    1140

aaagtcgtat agctaccagt gaggaagtgg actcaaatcg acttcagcaa catctcctgg    1200

ataaacttta agcctaaact atacagaata agataggtgg agagcttata ccgagctccc    1260

aaatctgtcc agatcatggt tgaccggtgc ctggatcttc ctatagaatc atccttattc 1320
gttgacctag ctgattctgg agtgacccag agggtcatga cttgagccta aaatccgccg 1380
cctccaccat ttgtagaaaa atgtgacgaa ctcgtgagct ctgtacagtg accggtgact 1440
ctttctggca tgcggagaga cggacggacg cagagagaag ggctgagtaa taagccactg 1500
gccagacagc tctggcggct ctgaggtgca gtggatgatt attaatccgg gaccggccgc 1560
ccctccgccc cgaagtggaa aggctggtgt gcccctcgtt gaccaagaat ctattgcatc 1620
atcggagaat atggagcttc atcgaatcac cggcagtaag cgaaggagaa tgtgaagcca 1680
ggggtgtata gccgtcggcg aaatagcatg ccattaacct aggtacagaa gtccaattgc 1740
ttccgatctg gtaaaagatt cacgagatag taccttctcc gaagtaggta gagcgagtac 1800
ccggcgcgta agctccctaa ttggcccatc cggcatctgt agggcgtcca aatatcgtgc 1860
ctctcctgct ttgcccggtg tatgaaaccg gaaaggccgc tcaggagctg gccagcggcg 1920
cagaccggga acacaagctg gcagtcgacc catccggtgc tctgcactcg acctgctgag 1980
gtccctcagt ccctggtagg cagctttgcc ccgtctgtcc gcccggtgtg tcggcggggt 2040
tgacaaggtc gttgcgtcag tccaacattt gttgccatat tttcctgctc tccccaccag 2100
ctgctctttt cttttctctt tcttttccca tcttcagtat attcatcttc ccatccaaga 2160
acctttattt cccctaagta agtactttgc tacatccata ctccatcctt cccatccctt 2220
attcctttga acctttcagt tcgagctttc ccacttcatc gcagcttgac taacagctac 2280
cccgcttgag cagacatcac catgtctacc aaccgccgct tcgaccctaa cttcactccc 2340
tatgtggtca actccatggg acccaagacc cccgagcgtg ctcgtgtggt ccttggtgcc 2400
ctgatcagac acatccacga cttcgctcgt gaggttgagc ttacctccgc tgagtggatg 2460
cttggtgttg agttcatcaa ctccattgga aagatcagca ctcccatccg taacgagtgc 2520
caccgtatct gcgatgttat cggtctggaa tcgtaagtcc acacaacagc gatcgcgcca 2580
aactcaaaaa ctaaccttta tctccgcaca gtctcgtcga tgagatcgcc aacaagatcg 2640
tcaccgagga cggtgtctcc cccacctcca acgtcattct cggccccttc tggtctccca 2700
acgctccctt ccgtgagctt ggcgacagca tcatccagga ccccaacccc aacggaaagg 2760
tcacctacat gcacggtgtc ctgaaggaca tggagactgg cgctcccatc gtcggtgccg 2820
tcctcgatat ctggcaggcc tccgccaacg gccagtacga cttccaggac cccaaccaga 2880
gtgagaacaa ccttcgcggc aagttccgct ccaacgagaa gggcgagttc aactggtact 2940
gctaccaccc cactccttac tctctcccca ccgatggtcc cgccggtgtc ctcctcaacc 3000
tcatggaccg ctctcccatg cgtcctgccc acatccacct gatgatcacc caccccgact 3060
acgccaccgt catcaaccag atctacccct ccgacgaccc tcatctcgac atcgactccg 3120
tcttcgccgt caaggacgac ctggttgtcg acttcaagcc caagaccgat gaccccaagg 3180

cccagctcga cctggagtac aacgtcacca tggctctgaa gaagcaccac cccaacccca 3240

actccgctcc cccggtctct tcctttgagc gtttcaacaa ggccagcaag actcaggaga 3300

agttgcacca ccaccaccac cactagggat ccacttaacg ttactgaaat catcaaacag 3360

cttgacgaat ctggatataa gatcgttggt gtcgatgtca gctccggagt tgagacaaat 3420

ggtgttcagg atctcgataa gatacgttca tttgtccaag cagcaaagag tgccttctag 3480

tgatttaata gctccatgtc aacaagaata aaacgcgttt tcgggtttac ctcttccaga 3540

tacagctcat ctgcaatgca ttaatgcatt gactgcaacc tagtaacgcc ttncaggctc 3600

cggcgaagag aagaatagct tagcagagct attttcattt tcgggagacg agatcaagca 3660

gatcaacggt cgtcaagaga cctacgagac tgaggaatcc gctcttggct ccacgcgact 3720

atatatttgt ctctaattgt actttgacat gctcctcttc tttactctga tagcttgact 3780

atgaaaattc cgtcaccagc ncctgggttc gcaaagataa ttgcatgttt cttccttgaa 3840

ctctcaagcc tacaggacac acattcatcg taggtataaa cctcgaaatc anttcctact 3900

aagatggtat acaatagtaa ccatgcatgg ttgcctagtg aatgctccgt aacacccaat 3960

acgccggccg aaactttttt acaactctcc tatgagtcgt ttacccagaa tgcacaggta 4020

cacttgttta gaggtaatcc ttctttctag aagtcctcgt gtactgtgta agcgcccact 4080

ccacatctcc actcgacctg caggcatgca agcttggcac tggccgtcgt tttacaacgt 4140

cgtgactggg aaaaccctgg cgttacccaa cttaatcgcc ttgcagcaca tccccctttc 4200

gccagctggc gtaatagcga agaggcccgc accgatcgcc cttcccaaca gttgcgcagc 4260

ctgaatggcg aatggcgcct gatgcggtat tttctcctta cgcatctgtg cggtatttca 4320

caccgcatat ggtgcactct cagtacaatc tgctctgatg ccgcatagtt aagccagccc 4380

cgacacccgc caacacccgc tgacgcgccc tgacgggctt gtctgctccc ggcatccgct 4440

tacagacaag ctgtgaccgt ctccgggagc tgcatgtgtc agaggttttc accgtcatca 4500

ccgaaacgcg cgagacgaaa gggcctcgtg atacgcctat ttttataggt taatgtcatg 4560

ataataatgg tttcttagac gtcaggtggc acttttcggg gaaatgtgcg cggaacccct 4620

atttgtttat ttttctaaat acattcaaat atgtatccgc tcatgagaca ataaccctga 4680

taaatgcttc aataatattg aaaaaggaag agtatgagta ttcaacattt ccgtgtcgcc 4740

cttattccct tttttgcggc attttgcctt cctgtttttg ctcacccaga aacgctggtg 4800

aaagtaaaag atgctgaaga tcagttgggt gcacgagtgg gttacatcga actggatctc 4860

aacagcggta agatccttga gagttttcgc cccgaagaac gttttccaat gatgagcact 4920

tttaaagttc tgctatgtgg cgcggtatta tcccgtattg acgccgggca agagcaactc 4980

ggtcgccgca tacactattc tcagaatgac ttggttgagt actcaccagt cacagaaaag 5040

catcttacgg atggcatgac agtaagagaa ttatgcagtg ctgccataac catgagtgat 5100

aacactgcgg ccaacttact tctgacaacg atcggaggac cgaaggagct aaccgctttt 5160

ttgcacaaca tgggggatca tgtaactcgc cttgatcgtt gggaaccgga gctgaatgaa 5220

gccataccaa acgacgagcg tgacaccacg atgcctgtag caatggcaac aacgttgcgc 5280

aaactattaa ctggcgaact acttactcta gcttcccggc aacaattaat agactggatg 5340

gaggcggata aagttgcagg accacttctg cgctcggccc ttccggctgg ctggtttatt 5400

gctgataaat ctggagccgg tgagcgtggg tctcgcggta tcattgcagc actggggcca 5460

gatggtaagc cctcccgtat cgtagttatc tacacgacgg ggagtcaggc aactatggat 5520

gaacgaaata gacagatcgc tgagataggt gcctcactga ttaagcattg gtaactgtca 5580

gaccaagttt actcatatat actttagatt gatttaaaac ttcattttta atttaaaagg 5640

atctaggtga agatcctttt tgataatctc atgaccaaaa tcccttaacg tgagttttcg 5700

ttccactgag cgtcagaccc cgtagaaaag atcaaaggat cttcttgaga tccttttttt 5760

ctgcgcgtaa tctgctgctt gcaaacaaaa aaaccaccgc taccagcggt ggtttgtttg 5820

ccggatcaag agctaccaac tctttttccg aaggtaactg gcttcagcag agcgcagata 5880

ccaaatactg tccttctagt gtagccgtag ttaggccacc acttcaagaa ctctgtagca 5940

ccgcctacat acctcgctct gctaatcctg ttaccagtgg ctgctgccag tggcgataag 6000

tcgtgtctta ccgggttgga ctcaagacga tagttaccgg ataaggcgca gcggtcgggc 6060

tgaacggggg gttcgtgcac acagcccagc ttggagcgaa cgacctacac cgaactgaga 6120

tacctacagc gtgagctatg agaaagcgcc acgcttcccg aagggagaaa ggcggacagg 6180

tatccggtaa gcggcagggt cggaacagga gagcgcacga gggagcttcc agggggaaac 6240

gcctggtatc tttatagtcc tgtcgggttt cgccacctct gacttgagcg tcgatttttg 6300

tgatgctcgt caggggggcg gagcctatgg aaaaacgcca gcaacgcggc ctttttacgg 6360

ttcctggcct tttgctggcc ttttgctcac atgttctttc ctgcgttatc ccctgattct 6420

gtggataacc gtattaccgc ctttgagtga gctgataccg ctcgccgcag ccgaacgacc 6480

gagcgcagcg agtcagtgag cgaggaagcg gaagagcgcc caatacgcaa accgcctctc 6540

cccgcgcgtt ggccgattca ttaatgcagc tggcacgaca ggtttcccga ctggaaagcg 6600

ggcagtgagc gcaacgcaat taatgtgagt tagctcactc attaggcacc ccaggcttta 6660

cactttatgc ttccggctcg tatgttgtgt ggaattgtga gcggataaca atttcacaca 6720

ggaaacagct atgaccatga ttac 6744

<210> 7
<211> 329
<212> PRT
<213> Aspergillus niger

<400>  7

Met Asp Pro Ser Lys Val Lys Ile Pro Pro Met Lys Asp Leu Thr Val
1               5                   10                  15

Asp Asn Ile Thr Glu Asn Val Ile Arg Ile Asn Ser Leu Cys Gln Asp
            20                  25                  30

Glu Arg Leu Lys Tyr Val Leu Glu Arg Leu Val Thr His Leu His Asp
        35                  40                  45

Phe Ala Arg Glu Thr Arg Leu Ser Thr Asp Glu Trp Met Thr Gly Leu
    50                  55                  60

Arg Phe Leu Thr Glu Val Gly Lys Ile Cys Ser Asp Val Arg Gln Glu
65                  70                  75                  80

Tyr Ile Leu Leu Ser Asp Ile Leu Gly Leu Ser Ile Leu Val Asp Ser
                85                  90                  95

Ile Asp His Pro Lys Pro Pro Asn Ser Thr Glu Gly Thr Val Leu Gly
            100                 105                 110

Pro Phe His Thr His Asp Ala Glu Pro Leu Thr Pro Gly Ala Ser Ile
        115                 120                 125

Ser His Asp Pro Ala Gly Glu Pro Leu Leu Val Val Cys Thr Val Lys
    130                 135                 140

Asp Thr His Gly Asn Pro Val Ser Asp Val Lys Ile Asp Ile Trp Glu
145                 150                 155                 160

Thr Asp Ser Thr Gly His Tyr Asp Val Gln Tyr Ala Gly Arg Asp Gly
                165                 170                 175

Pro Asp Gly Arg Cys Ile Met Thr Ser Asp Lys Glu Gly Met Phe Trp
            180                 185                 190

Phe Lys Ala Ile Thr Pro Val Pro Tyr Pro Ile Pro His Asp Gly Pro
        195                 200                 205

Val Gly Lys Leu Leu Lys Leu Leu Gly Arg His Pro Tyr Arg Pro Ser
    210                 215                 220

His Met His Phe Met Phe Glu Lys Gly Gly Phe Asp His Leu Ile Thr
225                 230                 235                 240

Ala Leu Tyr Leu Arg Asn Asp Pro Tyr Glu Thr Ser Asp Ala Val Phe
                245                 250                 255

Gly Val Lys Asp Ser Leu Val Val Asp Ile Gly Lys Ala Gly Pro Glu
            260                 265                 270

Tyr Ala Ala Lys Tyr Gly Val Ser Glu Asp His Ala Leu Leu Thr Tyr
        275                 280                 285

Asp Phe Val Leu Val Ser Asp Glu Glu Thr Ser Glu Leu Arg Ala Arg
    290                 295                 300

Asn Ser Lys Glu Ala Leu Asp Lys Leu Gly Arg Lys Val Lys Ile Val
305                 310                 315                 320

Asn Gly Leu Pro Val Pro Asp Leu Asp
                325

<210>  8
<211>  990
<212>  DNA
<213>  Aspergillus niger

<400>  8
atggatccca gcaaagtcaa aattcccccc atgaaagacc tcacggtcga caacatcacc      60

gagaacgtca ttcgcatcaa ctccctctgc caagacgagc gactgaaata cgttctcgag     120

cggctcgtca cccatctcca cgatttcgct agagagactc gtctcagcac ggatgaatgg     180

atgactggct tgagattcct cacggaagta ggcaagattt gttctgatgt tagacaggaa     240

tacattctac taagcgacat cctcggcctc tccatcctcg tcgactccat cgaccacccc     300

aaacccccca acagcaccga aggcaccgtc ctgggcccct tccacacgca cgacgccgag     360

cccctcaccc caggcgccag catctcccat gaccccgccg gcgagcccct cctcgtcgtc     420

tgcaccgtca aagataccca cggaaacccc gtctccgacg tgaagatcga tatctgggag     480

acggattcca ccggccatta tgatgtgcag tatgccggac gggatgggcc ggatggaaga     540

tgtatcatga cgtctgacaa ggagggcatg ttctggttca aggccattac cccggttccg     600

taccccattc cgcatgatgg gccggtgggg aagctgttga agctgttggg aagacatccc     660

tatcgcccga gtcatatgca ttttatgttt gagaagggag ggtttgatca tttgattacg     720

gcgctgtatc tccgcaatga tccgtatgag acctccgatg cggtcttcgg agtgaaggat     780

tcgctggtgg tggatattgg taaggccggg ccggagtatg cggccaagta tggggtttcc     840

gaggatcatg cgctgctgac gtatgatttc gtgctggtga gtgatgagga gacgagtgag     900

ttgcgggcga ggaattccaa ggaggcgttg gataagttgg ggaggaaggt gaagattgtt     960

aatgggttgc cggtgccgga tttggattga                                      990

<210>  9
<211>  6269
<212>  DNA
<213>  Artificial Sequence

<220>
<223>  synthetic oligonucleotide

<400>  9
tggcgaatgg gacgcgccct gtagcggcgc attaagcgcg gcgggtgtgg tggttacgcg      60

cagcgtgacc gctacacttg ccagcgccct agcgcccgct cctttcgctt tcttcccttc     120

ctttctcgcc acgttcgccg gctttccccg tcaagctcta aatcgggggc tccctttagg     180

gttccgattt agtgctttac ggcacctcga ccccaaaaaa cttgattagg gtgatggttc     240

acgtagtggg ccatcgccct gatagacggt ttttcgccct ttgacgttgg agtccacgtt     300

ctttaatagt ggactcttgt tccaaactgg aacaacactc aaccctatct cggtctattc     360

ttttgattta taagggattt tgccgatttc ggcctattgg ttaaaaaatg agctgattta     420

acaaaaattt aacgcgaatt ttaacaaaat attaacgttt acaatttcag gtggcacttt     480

tcggggaaat gtgcgcggaa cccctatttg tttatttttc taaatacatt caaatatgta     540

tccgctcatg aattaattct tagaaaaact catcgagcat caaatgaaac tgcaatttat     600

tcatatcagg attatcaata ccatattttt gaaaaagccg tttctgtaat gaaggagaaa     660

actcaccgag gcagttccat aggatggcaa gatcctggta tcggtctgcg attccgactc     720

gtccaacatc aatacaacct attaatttcc cctcgtcaaa aataaggtta tcaagtgaga     780

aatcaccatg agtgacgact gaatccggtg agaatggcaa aagtttatgc atttctttcc     840

agacttgttc aacaggccag ccattacgct cgtcatcaaa atcactcgca tcaaccaaac     900

cgttattcat tcgtgattgc gcctgagcga gacgaaatac gcgatcgctg ttaaaaggac     960

aattacaaac aggaatcgaa tgcaaccggc gcaggaacac tgccagcgca tcaacaatat    1020

tttcacctga atcaggatat tcttctaata cctggaatgc tgttttcccg gggatcgcag    1080

tggtgagtaa ccatgcatca tcaggagtac ggataaaatg cttgatggtc ggaagaggca    1140

taaattccgt cagccagttt agtctgacca tctcatctgt aacatcattg gcaacgctac    1200

ctttgccatg tttcagaaac aactctggcg catcgggctt cccatacaat cgatagattg    1260

tcgcacctga ttgcccgaca ttatcgcgag cccatttata cccatataaa tcagcatcca    1320

tgttggaatt taatcgcggc ctagagcaag acgtttcccg ttgaatatgg ctcataacac    1380

cccttgtatt actgtttatg taagcagaca gttttattgt tcatgaccaa aatcccttaa    1440

cgtgagtttt cgttccactg agcgtcagac cccgtagaaa agatcaaagg atcttcttga    1500

gatccttttt ttctgcgcgt aatctgctgc ttgcaaacaa aaaaaccacc gctaccagcg    1560

gtggtttgtt tgccggatca agagctacca actctttttc cgaaggtaac tggcttcagc 1620

agagcgcaga taccaaatac tgtccttcta gtgtagccgt agttaggcca ccacttcaag 1680

aactctgtag caccgcctac atacctcgct ctgctaatcc tgttaccagt ggctgctgcc 1740

agtggcgata agtcgtgtct taccgggttg gactcaagac gatagttacc ggataaggcg 1800

cagcggtcgg gctgaacggg gggttcgtgc acacagccca gcttggagcg aacgacctac 1860

accgaactga gatacctaca gcgtgagcta tgagaaagcg ccacgcttcc cgaagggaga 1920

aaggcggaca ggtatccggt aagcggcagg gtcggaacag gagagcgcac gagggagctt 1980

ccagggggaa acgcctggta tctttatagt cctgtcgggt ttcgccacct ctgacttgag 2040

cgtcgatttt tgtgatgctc gtcagggggg cggagcctat ggaaaaacgc cagcaacgcg 2100

gcctttttac ggttcctggc cttttgctgg ccttttgctc acatgttctt tcctgcgtta 2160

tcccctgatt ctgtggataa ccgtattacc gcctttgagt gagctgatac cgctcgccgc 2220

agccgaacga ccgagcgcag cgagtcagtg agcgaggaag cggaagagcg cctgatgcgg 2280

tattttctcc ttacgcatct gtgcggtatt tcacaccgca tatatggtgc actctcagta 2340

caatctgctc tgatgccgca tagttaagcc agtatacact ccgctatcgc tacgtgactg 2400

ggtcatggct gcgccccgac acccgccaac acccgctgac gcgccctgac gggcttgtct 2460

gctcccggca tccgcttaca gacaagctgt gaccgtctcc gggagctgca tgtgtcagag 2520

gttttcaccg tcatcaccga aacgcgcgag gcagctgcgg taaagctcat cagcgtggtc 2580

gtgaagcgat tcacagatgt ctgcctgttc atccgcgtcc agctcgttga gtttctccag 2640

aagcgttaat gtctggcttc tgataaagcg ggccatgtta agggcggttt tttcctgttt 2700

ggtcactgat gcctccgtgt aagggggatt tctgttcatg ggggtaatga taccgatgaa 2760

acgagagagg atgctcacga tacgggttac tgatgatgaa catgcccggt tactggaacg 2820

ttgtgagggt aaacaactgg cggtatggat gcggcgggac cagagaaaaa tcactcaggg 2880

tcaatgccag cgcttcgtta atacagatgt aggtgttcca cagggtagcc agcagcatcc 2940

tgcgatgcag atccggaaca taatggtgca gggcgctgac ttccgcgttt ccagacttta 3000

cgaaacacgg aaaccgaaga ccattcatgt tgttgctcag gtcgcagacg ttttgcagca 3060

gcagtcgctt cacgttcgct cgcgtatcgg tgattcattc tgctaaccag taaggcaacc 3120

ccgccagcct agccgggtcc tcaacgacag gagcacgatc atgcgcaccc gtggggccgc 3180

catgccggcg ataatggcct gcttctcgcc gaaacgtttg gtggcgggac cagtgacgaa 3240

ggcttgagcg agggcgtgca agattccgaa taccgcaagc gacaggccga tcatcgtcgc 3300

gctccagcga aagcggtcct cgccgaaaat gacccagagc gctgccggca cctgtcctac 3360

gagttgcatg ataaagaaga cagtcataag tgcggcgacg atagtcatgc cccgcgccca 3420

ccggaaggag ctgactgggt tgaaggctct caagggcatc ggtcgagatc ccggtgccta 3480

atgagtgagc taacttacat taattgcgtt gcgctcactg cccgctttcc agtcgggaaa 3540

cctgtcgtgc cagctgcatt aatgaatcgg ccaacgcgcg gggagaggcg gtttgcgtat 3600

tgggcgccag ggtggttttt cttttcacca gtgagacggg caacagctga ttgcccttca 3660

ccgcctggcc ctgagagagt tgcagcaagc ggtccacgct ggtttgcccc agcaggcgaa 3720

aatcctgttt gatggtggtt aacggcggga tataacatga gctgtcttcg gtatcgtcgt 3780

atcccactac cgagatatcc gcaccaacgc gcagcccgga ctcggtaatg gcgcgcattg 3840

cgcccagcgc catctgatcg ttggcaacca gcatcgcagt gggaacgatg ccctcattca 3900

gcatttgcat ggtttgttga aaaccggaca tggcactcca gtcgccttcc cgttccgcta 3960

tcggctgaat ttgattgcga gtgagatatt tatgccagcc agccagacgc agacgcgccg 4020

agacagaact taatgggccc gctaacagcg cgatttgctg gtgacccaat gcgaccagat 4080

gctccacgcc cagtcgcgta ccgtcttcat gggagaaaat aatactgttg atgggtgtct 4140

ggtcagagac atcaagaaat aacgccggaa cattagtgca ggcagcttcc acagcaatgg 4200

catcctggtc atccagcgga tagttaatga tcagcccact gacgcgttgc gcgagaagat 4260

tgtgcaccgc cgctttacag gcttcgacgc cgcttcgttc taccatcgac accaccacgc 4320

tggcacccag ttgatcggcg cgagatttaa tcgccgcgac aatttgcgac ggcgcgtgca 4380

gggccagact ggaggtggca acgccaatca gcaacgactg tttgcccgcc agttgttgtg 4440

ccacgcggtt gggaatgtaa ttcagctccg ccatcgccgc ttccactttt tcccgcgttt 4500

tcgcagaaac gtggctggcc tggttcacca cgcgggaaac ggtctgataa gagacaccgg 4560

catactctgc gacatcgtat aacgttactg gtttcacatt caccaccctg aattgactct 4620

cttccgggcg ctatcatgcc ataccgcgaa aggttttgcg ccattcgatg gtgtccggga 4680

tctcgacgct ctcccttatg cgactcctgc attaggaagc agcccagtag taggttgagg 4740

ccgttgagca ccgccgccgc aaggaatggt gcatgcaagg agatggcgcc caacagtccc 4800

ccggccacgg ggcctgccac catacccacg ccgaaacaag cgctcatgag cccgaagtgg 4860

cgagcccgat cttccccatc ggtgatgtcg gcgatatagg cgccagcaac cgcacctgtg 4920

gcgccggtga tgccggccac gatgcgtccg gcgtagagga tcgagatctc gatcccgcga 4980

aattaatacg actcactata ggggaattgt gagcggataa caattcccct ctagaaataa 5040

ttttgtttaa ctttaagaag gagatatacc atgggcagca gccatcatca tcatcatcac 5100

agcagcggcc tggtgccgcg cggcagccat atggctagcg atcccagcaa agtcaaaatt 5160

ccccccatga aagacctcac ggtcgacaac atcaccgaga acgtcattcg catcaactcc 5220

ctctgccaag acgagcgact gaaatacgtt ctcgagcggc tcgtcaccca tctccacgat 5280

ttcgctagag agactcgtct cagcacggat gaatggatga ctggcttgag attcctcacg 5340

gaagtaggca agatttgttc tgatgttaga caggaataca ttctactaag cgacatcctc 5400

ggcctctcca tcctcgtcga ctccatcgac caccccaaac cccccaacag caccgaaggc 5460

accgtcctgg gccccttcca cacgcacgac gccgagcccc tcaccccagg cgccagcatc 5520

tcccatgacc ccgccggcga gcccctcctc gtcgtctgca ccgtcaaaga tacccacgga 5580

aaccccgtct ccgacgtgaa gatcgatatc tgggagacgg attccaccgg ccattatgat 5640

gtgcagtatg ccggacggga tgggccggat ggaagatgta tcatgacgtc tgacaaggag 5700

ggcatgttct ggttcaaggc cattaccccg gttccgtacc ccattccgca tgatgggccg 5760

gtggggaagc tgttgaagct gttgggaaga catccctatc gcccgagtca tatgcatttt 5820

atgtttgaga agggagggtt tgatcatttg attacggcgc tgtatctccg caatgatccg 5880

tatgagacct ccgatgcggt cttcggagtg aaggattcgc tggtggtgga tattggtaag 5940

gccgggccgg agtatgcggc caagtatggg gtttccgagg atcatgcgct gctgacgtat 6000

gatttcgtgc tggtgagtga tgaggagacg agtgagttgc gggcgaggaa ttccaaggag 6060

gcgttggata agttggggag gaaggtgaag attgttaatg ggttgccggt gccggatttg 6120

gattgaccac tgagatccgg ctgctaacaa agcccgaaag gaagctgagt tggctgctgc 6180

caccgctgag caataactag cataacccct tggggcctct aaacgggtct tgaggggttt 6240

tttgctgaaa ggaggaacta tatccggat 6269

<210> 10
<211> 1335
<212> DNA
<213> Artificial Sequence

<220>
<223> synthetic oligonucleotide

<400> 10
atggcgcaaa ccccgcaaga cctgaagatt gcgattctgg gtgcgggcat gggtggcctg 60

acctgcgcgc tggcgctggc gaaggagggt ttcaagcaca tcgacgtgta cgagaacgcg 120

cacgatctgg gctttgttgg tgcgggcatt caactggcgc cgaacatggc gcgtgtgctg 180

gaccgtctgg gtgtttggaa accgatcgag gcggaagcgg tgaacattac cagcaccagc 240

gtgcgtgttg gtgcgagcga cagcgagctg gcgcatgtgc cgctggaata catccacgat 300

acctacggtt atccgcacat ggttggtcat cgtagcagcc tggcgggtca cctgtatcag 360

ggctgcctgc aacacccgga ccagattcac ttccactttg cgaccaccgc gagcgatacc 420

accttcggtc cgaagagcag cttcaccgcg accccgcgtg acgcgccgga gaaagcgtac 480

cgtgtggaag cggacgttct gctggcgtgc gatggcatca agagccaagc gcgtgtggcg 540

atgctggacc tgctgaacat taaggcgagc gttaaagata cccaccaagc ggcgtatcgt 600

atcatgattc acaaggacca gatcaaagac gatccggagc tgctggaact gatgaacggt 660

aaccgtgtga cccgttggat cggcgaaaaa cgtcacatca ttgcgtaccc ggttagcaac 720

aacaccattt ataacctgag caccacccaa ccggataccc acttcgctgc ggcgaccaac 780

gcgacctaca ccacccgtgg tgacaagagc gtgatgctgg gtgttttcgg cgatttttgc 840

ccgatggtgc agcgtatgct gaactatgtg ccggagggtg aagtttgcga gtggaaactg 900

cgtgtgcacg aaccgctgcc gacctgggtt cacgaaaccg tggcgctggt tggcgatgcg 960

tgccacccga ccctgccgca cctggcgcag ggtgcggcgc aggcgattga agatggtggc 1020

gtgattggcg cggttctgag caagctgccg gataccaccc cggcgagcat ccacaaggcg 1080

ctgaaagtgt acgagaaggt tcgtaaagat cgtgcgtatg cgattgttga actggctgcg 1140

gcgagcggtc gtgcgctgca tctgggcgag ggtgcggcga aagaggagcg tgaccgtcaa 1200

tttgcggcgc tgcgtcaggg taaaggtccg gtgccggata aatgggcgga cgcggatgtt 1260

caaaaagaac tgtatggcgt tgatgcgacc aagattgcgc tggagcagtt tgatgagctg 1320

tttgcttctg tgtaa 1335

<210> 11
<211> 948
<212> DNA
<213> Artificial Sequence

<220>
<223> synthetic oligonucleotide

<400> 11
atgtccacca accgccgctt cgaccccaac ttcaccccct acgtcgtcaa ctccatgggt 60

cccaagaccc ccgagcgcgc ccgcgtcgtc ctcggtgccc tcatccgcca catccacgac 120

ttcgcccgcg aggtcgagct cacctccgcc gagtggatgc tcggtgtcga gttcatcaac 180

tccatcggta agatctccac ccccatccgc aacgagtgcc accgcatctg cgacgtcatc 240

ggtctcgagt ccctcgtcga cgagatcgcc aacaagatcg tcaccgagga cggtgtctcc 300

cccacctcca acgtcatcct cggtcccttc tggtccccca acgccccctt ccgcgagctc 360

ggtgactcca tcatccagga ccccaacccc aacggtaagg tcacctacat gcacggtgtc 420

ctcaaggaca tggagaccgg tgcccccatc gtcggtgccg tcctcgacat ctggcaggcc 480

tccgccaacg gtcagtacga cttccaggac cccaaccagt ccgagaacaa cctccgcggt 540

aagttccgct ccaacgagaa gggtgagttc aactggtact gctaccaccc caccccctac 600

tccctcccca ccgacggtcc cgccggtgtc ctcctcaacc tcatggaccg ctcccccatg 660

cgccccgccc acatccacct catgatcacc caccccgact acgccaccgt catcaaccag 720

atctacccct ccgacgaccc ccacctcgac atcgactccg tcttcgccgt caaggacgac 780

ctcgtcgtcg acttcaagcc caagaccgac gaccccaagg cccagctcga cctcgagtac 840

aacgtcacca tggccctcaa gaagcaccac cccaacccca actccgcccc ccccgtctcc 900

tccttcgagc gcttcaacaa ggcctccaag acccaggaga agctctaa 948

<210> 12
<211> 990
<212> DNA
<213> Artificial Sequence

<220>
<223> synthetic oligonucleotide

<400> 12
atggacccct ccaaggtcaa gatccccccc atgaaggacc tcaccgtcga caacatcacc 60

gagaacgtca tccgcatcaa ctccctctgc caggacgagc gcctcaagta cgtcctcgag 120

cgcctcgtca cccacctcca cgacttcgcc cgcgagaccc gcctctccac cgacgagtgg 180

atgaccggtc tccgcttcct caccgaggtc ggtaagatct gctccgacgt ccgccaggag 240

tacatcctcc tctccgacat cctcggtctc tccatcctcg tcgactccat cgaccacccc 300

aagcccccca actccaccga gggtaccgtc ctcggtccct tccacaccca cgacgccgag 360

cccctcaccc ccggtgcctc catctcccac gaccccgccg gtgagcccct cctcgtcgtc 420

tgcaccgtca aggacaccca cggtaacccc gtctccgacg tcaagatcga catctgggag 480

accgactcca ccggtcacta cgacgtccag tacgccggtc gcgacggtcc cgacggtcgc 540

tgcatcatga cctccgacaa ggagggtatg ttctggttca aggccatcac ccccgtcccc 600

taccccatcc cccacgacgg tcccgtcggt aagctcctca agctcctcgg tcgccacccc 660

taccgcccct cccacatgca cttcatgttc gagaagggtg gtttcgacca cctcatcacc 720

gccctctacc tccgcaacga cccctacgag acctccgacg ccgtcttcgg tgtcaaggac 780

tccctcgtcg tcgacatcgg taaggccggt cccgagtacg ccgccaagta cggtgtctcc 840

gaggaccacg ccctcctcac ctacgacttc gtcctcgtct ccgacgagga gacctccgag 900

ctccgcgccc gcaactccaa ggaggccctc gacaagctcg gtcgcaaggt caagatcgtc 960

aacggtctcc ccgtccccga cctcgactaa 990

<210> 13
<211> 6744
<212> DNA
<213> Artificial Sequence

<220>
<223> synthetic oligonucleotide

<220>
<221> misc_feature
<222> (3593)..(3593)
<223> n is a, c, g, or t

<220>
<221>  misc_feature
<222>  (3801)..(3801)
<223>  n is a, c, g, or t

<220>
<221>  misc_feature
<222>  (3892)..(3892)
<223>  n is a, c, g, or t

<400>  13
gaattccctt gtatctctac acacaggctc aaatcaataa gaagaacggt tcgtcttttt      60

cgtttatatc ttgcatcgtc ccaaagctat tggcgggata ttctgtttgc agttggctga     120

cttgaagtaa tctctgcaga tctttcgaca ctgaaatacg tcgagcctgc tccgcttgga     180

agcggcgagg agcctcgtcc tgtcacaact accaacatgg agtacgataa gggccagttc     240

cgccagctca ttaagagcca gttcatgggc gttggcatga tggccgtcat gcatctgtac     300

ttcaagtaca ccaacgctct tctgatccag tcgatcatcc gctgaaggcg ctttcgaatc     360

tggttaagat ccacgtcttc gggaagccag cgactggtga cctccagcgt ccctttaagg     420

ctgccaacag ctttctcagc cagggccagc ccaagaccga caaggcctcc ctccagaacg     480

ccgagaagaa ctggaggggt ggtgtcaagg aggagtaagc tccttattga agtcggagga     540

cggagcggtg tcaagaggat attcttcgac tctgtattat agataagatg atgaggaatt     600

ggaggtagca tagcttcatt tggatttgct ttccaggctg agactctagc ttggagcata     660

gagggtcctt tggctttcaa tattctcaag tatctcgagt ttgaacttat tccctgtgaa     720

ccttttattc accaatgagc attggaatga acatgaatct gaggactgca atcgccatga     780

ggttttcgaa atacatccgg atgtcgaagg cttggggcac ctgcgttggt tgaatttaga     840

acgtggcact attgatcatc cgatagctct gcaaagggcg ttgcacaatg caagtcaaac     900

gttgctagca gttccaggtg gaatgttatg atgagcattg tattaaatca ggagatatag     960

catgatctct agttagctca ccacaaaagt cagacggcgt aaccaaaagt cacacaacac    1020

aagctgtaag gatttcggca cggctacgga agacggagaa gccaccttca gtggactcga    1080

gtaccattta attctatttg tgtttgatcg agacctaata cagcccctac aacgaccatc    1140

aaagtcgtat agctaccagt gaggaagtgg actcaaatcg acttcagcaa catctcctgg    1200

ataaacttta agcctaaact atacagaata agataggtgg agagcttata ccgagctccc    1260

aaatctgtcc agatcatggt tgaccggtgc ctggatcttc ctatagaatc atccttattc    1320

gttgacctag ctgattctgg agtgacccag agggtcatga cttgagccta aaatccgccg    1380

cctccaccat ttgtagaaaa atgtgacgaa ctcgtgagct ctgtacagtg accggtgact    1440

ctttctggca tgcggagaga cggacggacg cagagagaag ggctgagtaa taagccactg    1500

gccagacagc tctggcggct ctgaggtgca gtggatgatt attaatccgg gaccggccgc    1560

ccctccgccc cgaagtggaa aggctggtgt gcccctcgtt gaccaagaat ctattgcatc 1620

atcggagaat atggagcttc atcgaatcac cggcagtaag cgaaggagaa tgtgaagcca 1680

ggggtgtata gccgtcggcg aaatagcatg ccattaacct aggtacagaa gtccaattgc 1740

ttccgatctg gtaaaagatt cacgagatag taccttctcc gaagtaggta gagcgagtac 1800

ccggcgcgta agctccctaa ttggcccatc cggcatctgt agggcgtcca aatatcgtgc 1860

ctctcctgct ttgcccggtg tatgaaaccg gaaaggccgc tcaggagctg gccagcggcg 1920

cagaccggga acacaagctg gcagtcgacc catccggtgc tctgcactcg acctgctgag 1980

gtccctcagt ccctggtagg cagctttgcc ccgtctgtcc gcccggtgtg tcggcggggt 2040

tgacaaggtc gttgcgtcag tccaacattt gttgccatat tttcctgctc tccccaccag 2100

ctgctctttt cttttctctt tcttttccca tcttcagtat attcatcttc ccatccaaga 2160

acctttattt cccctaagta agtactttgc tacatccata ctccatcctt cccatccctt 2220

attcctttga acctttcagt tcgagctttc ccacttcatc gcagcttgac taacagctac 2280

cccgcttgag cagacatcac catgtctacc aaccgccgct tcgaccctaa cttcactccc 2340

tatgtggtca actccatggg acccaagacc cccgagcgtg ctcgtgtggt ccttggtgcc 2400

ctgatcagac acatccacga cttcgctcgt gaggttgagc ttacctccgc tgagtggatg 2460

cttggtgttg agttcatcaa ctccattgga aagatcagca ctcccatccg taacgagtgc 2520

caccgtatct gcgatgttat cggtctggaa tcgtaagtcc acacaacagc gatcgcgcca 2580

aactcaaaaa ctaaccttta tctccgcaca gtctcgtcga tgagatcgcc aacaagatcg 2640

tcaccgagga cggtgtctcc cccacctcca acgtcattct cggccccttc tggtctccca 2700

acgctccctt ccgtgagctt ggcgacagca tcatccagga ccccaacccc aacggaaagg 2760

tcacctacat gcacggtgtc ctgaaggaca tggagactgg cgctcccatc gtcggtgccg 2820

tcctcgatat ctggcaggcc tccgccaacg gccagtacga cttccaggac cccaaccaga 2880

gtgagaacaa ccttcgcggc aagttccgct ccaacgagaa gggcgagttc aactggtact 2940

gctaccaccc cactccttac tctctcccca ccgatggtcc cgccggtgtc ctcctcaacc 3000

tcatggaccg ctctcccatg cgtcctgccc acatccacct gatgatcacc caccccgact 3060

acgccaccgt catcaaccag atctacccct ccgacgaccc tcatctcgac atcgactccg 3120

tcttcgccgt caaggacgac ctggttgtcg acttcaagcc caagaccgat gaccccaagg 3180

cccagctcga cctggagtac aacgtcacca tggctctgaa gaagcaccac cccaacccca 3240

actccgctcc cccggtctct tcctttgagc gtttcaacaa ggccagcaag actcaggaga 3300

agttgcacca ccaccaccac cactagggat ccacttaacg ttactgaaat catcaaacag 3360

cttgacgaat ctggatataa gatcgttggt gtcgatgtca gctccggagt tgagacaaat 3420

ggtgttcagg atctcgataa gatacgttca tttgtccaag cagcaaagag tgccttctag 3480

tgatttaata gctccatgtc aacaagaata aaacgcgttt tcgggtttac ctcttccaga 3540

tacagctcat ctgcaatgca ttaatgcatt gactgcaacc tagtaacgcc ttncaggctc 3600

cggcgaagag aagaatagct tagcagagct attttcattt tcgggagacg agatcaagca 3660

gatcaacggt cgtcaagaga cctacgagac tgaggaatcc gctcttggct ccacgcgact 3720

atatatttgt ctctaattgt actttgacat gctcctcttc tttactctga tagcttgact 3780

atgaaaattc cgtcaccagc ncctgggttc gcaaagataa ttgcatgttt cttccttgaa 3840

ctctcaagcc tacaggacac acattcatcg taggtataaa cctcgaaatc anttcctact 3900

aagatggtat acaatagtaa ccatgcatgg ttgcctagtg aatgctccgt aacacccaat 3960

acgccggccg aaactttttt acaactctcc tatgagtcgt ttacccagaa tgcacaggta 4020

cacttgttta gaggtaatcc ttctttctag aagtcctcgt gtactgtgta agcgcccact 4080

ccacatctcc actcgacctg caggcatgca agcttggcac tggccgtcgt tttacaacgt 4140

cgtgactggg aaaaccctgg cgttacccaa cttaatcgcc ttgcagcaca tccccctttc 4200

gccagctggc gtaatagcga agaggcccgc accgatcgcc cttcccaaca gttgcgcagc 4260

ctgaatggcg aatggcgcct gatgcggtat tttctcctta cgcatctgtg cggtatttca 4320

caccgcatat ggtgcactct cagtacaatc tgctctgatg ccgcatagtt aagccagccc 4380

cgacacccgc caacacccgc tgacgcgccc tgacgggctt gtctgctccc ggcatccgct 4440

tacagacaag ctgtgaccgt ctccgggagc tgcatgtgtc agaggttttc accgtcatca 4500

ccgaaacgcg cgagacgaaa gggcctcgtg atacgcctat ttttataggt taatgtcatg 4560

ataataatgg tttcttagac gtcaggtggc acttttcggg gaaatgtgcg cggaacccct 4620

atttgtttat ttttctaaat acattcaaat atgtatccgc tcatgagaca ataaccctga 4680

taaatgcttc aataatattg aaaaaggaag agtatgagta ttcaacattt ccgtgtcgcc 4740

cttattccct tttttgcggc attttgcctt cctgtttttg ctcacccaga aacgctggtg 4800

aaagtaaaag atgctgaaga tcagttgggt gcacgagtgg gttacatcga actggatctc 4860

aacagcggta agatccttga gagttttcgc cccgaagaac gttttccaat gatgagcact 4920

tttaaagttc tgctatgtgg cgcggtatta tcccgtattg acgccgggca agagcaactc 4980

ggtcgccgca tacactattc tcagaatgac ttggttgagt actcaccagt cacagaaaag 5040

catcttacgg atggcatgac agtaagagaa ttatgcagtg ctgccataac catgagtgat 5100

aacactgcgg ccaacttact tctgacaacg atcggaggac cgaaggagct aaccgctttt 5160

ttgcacaaca tgggggatca tgtaactcgc cttgatcgtt gggaaccgga gctgaatgaa 5220

gccataccaa acgacgagcg tgacaccacg atgcctgtag caatggcaac aacgttgcgc 5280

aaactattaa ctggcgaact acttactcta gcttcccggc aacaattaat agactggatg 5340

gaggcggata aagttgcagg accacttctg cgctcggccc ttccggctgg ctggtttatt 5400

gctgataaat ctggagccgg tgagcgtggg tctcgcggta tcattgcagc actggggcca 5460

gatggtaagc cctcccgtat cgtagttatc tacacgacgg ggagtcaggc aactatggat 5520

gaacgaaata gacagatcgc tgagataggt gcctcactga ttaagcattg gtaactgtca 5580

gaccaagttt actcatatat actttagatt gatttaaaac ttcattttta atttaaaagg 5640

atctaggtga agatcctttt tgataatctc atgaccaaaa tcccttaacg tgagttttcg 5700

ttccactgag cgtcagaccc cgtagaaaag atcaaaggat cttcttgaga tccttttttt 5760

ctgcgcgtaa tctgctgctt gcaaacaaaa aaaccaccgc taccagcggt ggtttgtttg 5820

ccggatcaag agctaccaac tctttttccg aaggtaactg gcttcagcag agcgcagata 5880

ccaaatactg tccttctagt gtagccgtag ttaggccacc acttcaagaa ctctgtagca 5940

ccgcctacat acctcgctct gctaatcctg ttaccagtgg ctgctgccag tggcgataag 6000

tcgtgtctta ccgggttgga ctcaagacga tagttaccgg ataaggcgca gcggtcgggc 6060

tgaacggggg gttcgtgcac acagcccagc ttggagcgaa cgacctacac cgaactgaga 6120

tacctacagc gtgagctatg agaaagcgcc acgcttcccg aagggagaaa ggcggacagg 6180

tatccggtaa gcggcagggt cggaacagga gagcgcacga gggagcttcc agggggaaac 6240

gcctggtatc tttatagtcc tgtcgggttt cgccacctct gacttgagcg tcgatttttg 6300

tgatgctcgt caggggggcg gagcctatgg aaaaacgcca gcaacgcggc ctttttacgg 6360

ttcctggcct tttgctggcc ttttgctcac atgttctttc ctgcgttatc ccctgattct 6420

gtggataacc gtattaccgc ctttgagtga gctgataccg ctcgccgcag ccgaacgacc 6480

gagcgcagcg agtcagtgag cgaggaagcg gaagagcgcc caatacgcaa accgcctctc 6540

cccgcgcgtt ggccgattca ttaatgcagc tggcacgaca ggtttcccga ctggaaagcg 6600

ggcagtgagc gcaacgcaat taatgtgagt tagctcactc attaggcacc ccaggcttta 6660

cactttatgc ttccggctcg tatgttgtgt ggaattgtga gcggataaca atttcacaca 6720

ggaaacagct atgaccatga ttac 6744

<210> 14
<211> 3239
<212> DNA
<213> Artificial Sequence

<220>
<223> synthetic oligonucleotide

<400> 14
cgaactactt tcggcatctt ctagacttcc taataccgcc tagtcatagc agattcaagc 60

tgagaacacc acaagtaaat atcacccatc atgcttaccc tgaccgtccc tgaaaactac 120

gggtatgtgc caattctaca attccttgca gacaatgcca ttctccccat gaagtctgat 180

gctaactatc ctgcagctct gtcattgccg tcgctctggg tgccatcccc gtcctgagct 240

tcgtccatgg cgccgtcgtg tctcgtctcc gcaaggaagc tgattgcccc taccctcact 300

gctatgcgac cgtagagcag tgcaagacca acgtaagcca acctcacaca aacaggattc 360

ctcgagctaa catacattcc gaaccgtgca gcccaaggcc gagcagttca actgcgctca 420

gcgcgctcat gccaacttcc ttgagaactc cagccaaact atgctcttcc tcctggtagc 480

tggactgaag tacccccagt tggcgactgg cctcggaagc atctgggtcc tcggtcgctc 540

actgttcctt tacggatatg tgtactccgg caagccgcgg ggtcgcggtc gtttgtacgg 600

cagcttctac ttgcttgcac agggagctct ctggggcttg acgtcttttg gagttgcgag 660

ggagttgatt tcctacttct aagtttggac tgaatccgtg gtgtgattga ggtgattggc 720

gatgtttggc tataccagct atatgtaata atctctactg tatactacta ttcaacgcat 780

tttactatgc gtgctgctag ggtcggcaat gacaatggca atctgactga cgtggtctat 840

ttctccatgt gcagcaggga atacgagctc caatggacct cgggagtggc acagtcaatg 900

gcaaggaaac tccgcctttg caggtgtggc tgaaccccac gggtcggagg cggagcaatc 960

cacccccgat gtggctggtg cgtggagggg ctcgcgatga ttttactgag cttgcttttc 1020

ttgtcgacat tgaacattgt ccttggtctt ccttcagatt taagggtcag tcactgctac 1080

atttctcagt agtatccgcg cacgtctctg gatttacgaa tcagggtcca ccagtcgaaa 1140

cttcgaacta ctctcattat acaatcctct ttccattccc gcattaaccc ctccatcaac 1200

accatgtcct ccaagtcgca attgacctac actgcccgtg ccagcaagca tcccaatgct 1260

ctggcgaaga ggctgttcga gattgccgag gccaagaaga ccaatgtgac tgtctcggct 1320

gacgttacca ccactaagga gctactagat cttgctgacc gtaggccgac ccgctactct 1380

gcctgattat gctgcatgca aacttattaa cggtgatacc ggactgcagg tctcggtccc 1440

tacattgccg tgatcaaaac ccacatcgat atcctctctg atttcagcaa cgagaccatt 1500

gagggactta aggctctcgc gcagaagcac aactttctca tcttcgagga ccgcaagttc 1560

attgacatcg gcaacacggt ccagaagcaa taccacggcg gtaccctccg tatctcggaa 1620

tgggcccaca tcatcaactg cagcattctc cctggtgagg gtatcgtcga ggctctcgct 1680

cagacggcgt ctgcaccgga cttcgcctac ggccccgaac gcggtctgtt gatcttggca 1740

gagatgacct ctaagggctc cttggctacc ggccagtaca ctacttcctc ggtcgattat 1800

gcccggaaat acaagaactt cgttatggga ttcgtgtcga cgcgcgcgtt gggtgaggtg 1860

cagtcggaag tcagctctcc ttcggatgag gaggactttg tggtcttcac gactggtgtg 1920

aacatttctt ccaaggagga taagcttggt cagcagtacc agacgcccgg atcggctatc 1980

ggccggggtg ctgacttcat tatcgcgggt cgcggtatct acgccgcgcc ggatccggtg 2040

caggctgcgc aacagtatca gaaggagggg tgggaagcct acctggcccg tgtcggcgga 2100

aactaatact ataaaaggag gatcgaagtt ctgatggtta tgaatgatat agaaatgcaa 2160

cttgccgcaa cggatacgga agcggaaacg gaccaatgtc gagcacgggt agtcagactg 2220

cggcatcgga tgtccaaacg gtattgatcc tgcaggctac tatggtgtgg cacaaggatc 2280

aatgcggtac gacgatttga tgcagataag caggctgcga agtagtaact cttgcgtaga 2340

gaaaatggcg acgggtggct gataagggcg gtgataagct taattgtcat cgcagataag 2400

cactgctgtc ttgcatccaa gtcagcgtca gcagaaatac gggacttccg aaagtatatg 2460

gcaaaattaa agaacttgac tctccagcaa tgttttgccc tgaccgtcgc taaaacgtta 2520

ctacccctat acccgtctgt ttgtcccagc ccgaggcatt aggtctgact gacagcacgg 2580

cgccatgcgg gcttgggacg ccatgtccgt cgcgtgataa gggttgatcc atgcagctac 2640

tatccttcca tcgttccatt cccatccttg tcctatctcc atccttgaaa ctttactagt 2700

ttagttggat gctcgagctt gctctcggct actccgtcca atggataaga ccccgatgcc 2760

ggtcctcatt ggtctccagc tggtatcgcc ccaaccttcg tgtgatcgcc tctctgcttc 2820

ccctcatcat cattactaac tagtacatcc aaaagccatc ccagtgcttc ccctcaccct 2880

tgcccaagac attccaagtg ggccttcggc tggaaaacat ggacccattg gttccatcga 2940

taagctagct cctcgtccgt taccccagat tgataccaga taacattgac cagcggctta 3000

tcaccgaggt ctgcgggtga gaccccccct gcgacaagtt agataaaaga aactcgcctc 3060

attgtgcttc cgatggggtc ggatgacgag ccttcggaaa gagctggcgc ctctttaaag 3120

gggacagctg tcgccaagtt gtgaaattct ccgataacta ctaacaatct ctcccttcct 3180

tcccgctact gtggtcacca aatcaactct cttttctcgg ccaagatcta acatggcgg 3239

<210> 15
<211> 3239
<212> DNA
<213> Artificial Sequence

<220>
<223> synthetic oligonucleotide

<400> 15
cgaactactt tcggcatctt ctagacttcc taataccgcc tagtcatagc agattcaagc 60

tgagaacacc acaagtaaat atcacccatc atgcttaccc tgaccgtccc tgaaaactac 120

gggtatgtgc caattctaca attccttgca gacaatgcca ttctccccat gaagtctgat 180

gctaactatc ctgcagctct gtcattgccg tcgctctggg tgccatcccc gtcctgagct 240

tcgtccatgg cgccgtcgtg tctcgtctcc gcaaggaagc tgattgcccc taccctcact 300

gctatgcgac cgtagagcag tgcaagacca acgtaagcca acctcacaca aacaggattc 360

ctcgagctaa catacattcc gaaccgtgca gcccaaggcc gagcagttca actgcgctca 420

gcgcgctcat gccaacttcc ttgagaactc cagccaaact atgctcttcc tcctggtagc 480

tggactgaag tacccccagt tggcgactgg cctcggaagc atctgggtcc tcggtcgctc 540

actgttcctt tacggatatg tgtactccgg caagccgcgg ggtcgcggtc gtttgtacgg 600

cagcttctac ttgcttgcac agggagctct ctggggcttg acgtcttttg gagttgcgag 660

ggagttgatt tcctacttct aagtttggac tgaatccgtg gtgtgattga ggtgattggc 720

gatgtttggc tataccagct atatgtaata atctctactg tatactacta ttcaacgcat 780

tttactatgc gtgctgctag ggtcggcaat gacaatggca atctgactga cgtggtctat 840

ttctccatgt gcagcaggga atacgagctc caatggacct cgggagtggc acagtcaatg 900

gcaaggaaac tccgcctttg caggtgtggc tgaaccccac gggtcggagg cggagcaatc 960

cacccccgat gtggctggtg cgtggagggg ctcgcgatga ttttactgag cttgcttttc 1020

ttgtcgacat tgaacattgt ccttggtctt ccttcagatt taagggtcag tcactgctac 1080

atttctcagt agtatccgcg cacgtctctg gatttacgaa tcagggtcca ccagtcgaaa 1140

cttcgaacta ctctcattat acaatcctct ttccattccc gcattaaccc ctccatcaac 1200

accatgtcct ccaagtcgca attgacctac actgcccgtg ccagcaagca tcccaatgct 1260

ctggcgaaga ggctgttcga gattgccgag gccaagaaga ccaatgtgac tgtctcggct 1320

gacgttacca ccactaagga gctactagat cttgctgacc gtaggccgac ccgctactct 1380

gcctgattat gctgcatgca aacttattaa cggtgatacc ggactgcagg tctcggtccc 1440

tacattgccg tgatcaaaac ccacatcgat atcctctctg atttcagcaa cgagaccatt 1500

gagggactta aggctctcgc gcagaagcac aactttctca tcttcgagga ccgcaagttc 1560

attgacatcg gcaacacggt ccagaagcaa taccacggcg gtaccctccg tatctcggaa 1620

tgggcccaca tcatcaactg cagcattctc cctggtgagg gtatcgtcga ggctctcgct 1680

cagacggcgt ctgcaccgga cttcgcctac ggccccgaac gcggtctgtt gatcttggca 1740

gagatgacct ctaagggctc cttggctacc ggccagtaca ctacttcctc ggtcgattat 1800

gcccggaaat acaagaactt cgttatggga ttcgtgtcga cgcgcgcgtt gggtgaggtg 1860

cagtcggaag tcagctctcc ttcggatgag gaggactttg tggtcttcac gactggtgtg 1920

aacatttctt ccaagggaga taagcttggt cagcagtacc agacgcccgg atcggctatc 1980

ggccggggtg ctgacttcat tatcgcgggt cgcggtatct acgccgcgcc ggatccggtg 2040

caggctgcgc aacagtatca gaaggagggg tgggaagcct acctggcccg tgtcggcgga 2100

aactaatact ataaaaggag gatcgaagtt ctgatggtta tgaatgatat agaaatgcaa 2160

cttgccgcaa cggatacgga agcggaaacg gaccaatgtc gagcacgggt agtcagactg 2220

cggcatcgga tgtccaaacg gtattgatcc tgcaggctac tatggtgtgg cacaaggatc 2280

aatgcggtac gacgatttga tgcagataag caggctgcga agtagtaact cttgcgtaga 2340

gaaaatggcg acgggtggct gataagggcg gtgataagct taattgtcat cgcagataag 2400

cactgctgtc ttgcatccaa gtcagcgtca gcagaaatac gggacttccg aaagtatatg 2460

gcaaaattaa agaacttgac tctccagcaa tgttttgccc tgaccgtcgc taaaacgtta 2520

ctacccctat acccgtctgt ttgtcccagc ccgaggcatt aggtctgact gacagcacgg 2580

cgccatgcgg gcttgggacg ccatgtccgt cgcgtgataa gggttgatcc atgcagctac 2640

tatccttcca tcgttccatt cccatccttg tcctatctcc atccttgaaa ctttactagt 2700

ttagttggat gctcgagctt gctctcggct actccgtcca atggataaga ccccgatgcc 2760

ggtcctcatt ggtctccagc tggtatcgcc ccaaccttcg tgtgatcgcc tctctgcttc 2820

ccctcatcat cattactaac tagtacatcc aaaagccatc ccagtgcttc ccctcaccct 2880

tgcccaagac attccaagtg ggccttcggc tggaaaacat ggacccattg gttccatcga 2940

taagctagct cctcgtccgt taccccagat tgataccaga taacattgac cagcggctta 3000

tcaccgaggt ctgcgggtga gaccccccct gcgacaagtt agataaaaga aactcgcctc 3060

attgtgcttc cgatggggtc ggatgacgag ccttcggaaa gagctggcgc ctctttaaag 3120

gggacagctg tcgccaagtt gtgaaattct ccgataacta ctaacaatct ctcccttcct 3180

tcccgctact gtggtcacca aatcaactct cttttctcgg ccaagatcta acatggcgg 3239

<210> 16
<211> 3822
<212> DNA
<213> Artificial Sequence

<220>
<223> synthetic oligonucleotide

<400> 16
ctgggtgcca ttggcagtag cttgaagcgt cgccagaact gggactacaa ccctgacgcc 60

aaaaatccca acggactcct cgagaagctg ggaccagtga ttgactgcat cttggtgatt 120

accggcgagg acgccaagga ccttatcgtt aagctgagca agcccgtggc tgagctgttc 180

gagaaactgg gactgactgg cctgggcaag agcgttggca aggtcatcaa gacggtcggc 240

gatgcgggcg acttggttgc ggatctgggt ccggtcagcg agagtttgct gacggtagtt 300

ggcgatggtg gcagcaagct ggtggtccag ttggctcctg atgttgcgga cttggtgaag 360

ggcctgggac tccccaaact tggtgatgcc gtgggtgaag ttctcggagc tgtgggtgag 420

aacttgtaag aagtctcgac tatttcccct ctttttcttt tcctctgtcc tatgaatggg 480

tgatggaatt gcattgcatt acattttctg ggatgcgatg gtgttgaggc tacttactag 540

tactatatac tatacaagat gtaattagtg taattgttaa tgatacctaa tcaagatatt 600

ttcagttgac ttcaagtctg ccatttccac gattctatct atagtaataa tgtcttccat 660

atcatccatc cactcttccc cttcatttcc ccatattctt atcccccttc catttatccg 720

ccgattcctc tccgaggcaa ccttcctttt cccccctctt ctctctctct ctctctctct 780

ccataggcgt acgtaatccg cattccacag cctctcattg ctttgccccg gatgtaaatt 840

gattcgcccc acggggttgg actgccgatg tggagaagcg agccgccaga cttggcaagg 900

caaaccgtca tccaactgga gattactggc agtctggcac agcgtgcgtg tgagggagag 960

agggagagag agtttcccgg ggggaaacac agagtttcgc ctcccgcgtc gttccctcca 1020

gcaatgaaat catggatgat tgtgggacgt ggggaggcca cccccccccc cccccccccc 1080

cgcgggtaag attattcccg ccgtgccatt tccgattgcc ggtcagagtt cactatttaa 1140

tcctccagtt ttctctactt tttctggctc tgttcagctt caaaggcttt tcaatttgtt 1200

tctcatagac acatctcctc tctgattctc cttcgttctt atactatact atcatcatgg 1260

cgcaaactcc gcaagacctt aagattgcca ttttgggtgc tggtacgtac atcctttttt 1320

tttcttcccc ctccctataa tagggtttag tataggagta tggcagaaaa ctaacgggtg 1380

cgtggatgta taggcatggg tggcctgacc tgcgctttgg ctctggcaaa ggaaggtttc 1440

aagcacatcg acgtttatga gaatgcacac gatctgggct tcgttggagc tggtatccaa 1500

ctggccccga acatggcccg cgtgctggat cgcctgggcg tctggaagcc cattgaggcc 1560

gaagctgtta acatcacttc cactagcgtt agaggtatgt gctatattcc ctgctggagg 1620

attaaaagat ctacatgtct atctatatga aacaccgaga tcaaacgcta ataactccga 1680

acagtcggtg cctcggactc agaattggcc cacgttccat tggaatatat ccacgatacc 1740

tacggatatc ctcacatggt tggccaccgg tcttccctcg ccggccacct gtaccaaggt 1800

tgcctgcagc accctgacca gatccacttc cacttcgcca ctaccgccag cgacaccacc 1860

ttcggcccca agtcctcctt cactgccacc ccccgcgacg cccccgagaa ggcctaccgc 1920

gtcgaggccg atgtcctcct cgcctgtgat ggcatcaagt cccaggcccg tgtggccatg 1980

ctcgatctcc tcaacatcaa ggccagcgtc aaggacaccc accaggccgc ctaccgtatc 2040

atgatccaca aggaccagat caaggacgac ccggagctcc tggagctcat gaacggcaac 2100

cgcgtgactc gctggatcgg cgagaagcgc catatcatcg cctaccccgt ctccaacaac 2160

accatctaca acctctccac cacccaaccc gacactcact tcgccgccgc caccaatgcc 2220

acctacacca cccgcggtga caagtccgtc atgctgggcg tctttggcga cttctgcccc 2280

atggtccagc gtatgctgaa ctacgtcccc gagggtgaag tctgcgagtg gaagctgcgt 2340

gtccatgagc ccctccccac ctgggtgcac gagaccgtcg ccctcgtcgg tgatgcctgc 2400

caccctaccc tgccccatct cgcccagggt gccgcccagg ccatcgaaga cggtggtgtc 2460

attggtgccg tgctctccaa gctccccgat accaccccgg cttccatcca caaggccctc 2520

aaggtttacg agaaggtgcg caaggaccgc gcctacgcta tcgtcgaact ggccgccgct 2580

tccggtcgtg ctctgcacct gggtgaaggt gccgccaagg aggagcgcga tcgccaattc 2640

gctgctctcc gccagggcaa gggccctgtg cccgacaagt gggcggatgc cgatgtccag 2700

aaggagctgt atggcgttga tgcgactaag atcgcgctgg agcaattcga cgagctcttt 2760

gcttctgtgt aagctttggc ctttatacct ttttcgttta atttttcctt ttcccattca 2820

ttgcctgcac gattgaaccc gatgtatatt gtatatagag ctgattggtt gtattgatga 2880

ttgaaggtcg ggtcgtgagg caaagctggc ttgattgatt gattgattga ttgatcggcc 2940

ggaattagtg ttcttttatc tagcattgca gccccttgca tatacaatga tgatgttatg 3000

attgttatac agagttattt tctttgttcc tagatatact ccttttcgta gatgcatcca 3060

ttgtgtgaat gtatacaccc gtagttgtac caggacggaa gtggttggtt gcatagttag 3120

tctcgtttgt tagtgtcatg cgctggaatt taccccggat tttcagcccc tcctttagga 3180

atgaaaatcc taccaaatcc aaatcaaacc cgaggggttg gaccccttgg tccttgcact 3240

ccggccaggc aacaacaatt gtattggttt acccctcgag tttgtatggg agatacacga 3300

ttctagcgct gcacttccaa gacaaatcag tatctggggt ctacaatttt ccccccttcc 3360

ccaccattgc ggggatgcta atttgaaaag aatttatccc agatacacgt gagtaactgg 3420

ccattagtaa ttatgtgatg tcttatgtag tctacatact agagcacaaa ttttggtaca 3480

aattatggtc cccgataata tctgaaatcg ggatttgaat cagacgtgcg ggattagcta 3540

ggatccattt ctgccttgat ttgatagatc caaagaatag gtatcggaca aaagggtttc 3600

gacccaaatt tggagaatgg aaaaatctac actagcgacg ttctcgcatg ttagtggttt 3660

tacttagatc gaaatgtatt cacggaagca aagtccatga taatgaactc tatgtacttg 3720

gcatagccag cttatgcaca agcctcggca gcaaaaagta acctggtcta agctatgatt 3780

taaccttctt atcagattgt tggtgacggt ggcaaatcga ag 3822

<210> 17
<211> 3822
<212> DNA
<213> Artificial Sequence

<220>
<223> synthetic oligonucleotide

<400> 17
ctgggtgcca ttggcagtag cttgaagcgt cgccagaact gggactacaa ccctgacgcc 60

aaaaatccca acggactcct cgagaagctg ggaccagtga ttgactgcat cttggtgatt 120

accggcgagg acgccaagga ccttatcgtt aagctgagca agcccgtggc tgagctgttc 180

gagaaactgg gactgactgg cctgggcaag agcgttggca aggtcatcaa gacggtcggc 240

gatgcgggcg acttggttgc ggatctgggt ccggtcagcg agagtttgct gacggtagtt 300

ggcgatggtg gcagcaagct ggtggtccag ttggctcctg atgttgcgga cttggtgaag 360

ggcctgggac tccccaaact tggtgatgcc gtgggtgaag ttctcggagc tgtgggtgag 420

aacttgtaag aagtctcgac tatttcccct ctttttcttt tcctctgtcc tatgaatggg 480

tgatggaatt gcattgcatt acattttctg ggatgcgatg gtgttgaggc tacttactag 540

tactatatac tatacaagat gtaattagtg taattgttaa tgatacctaa tcaagatatt 600

ttcagttgac ttcaagtctg ccatttccac gattctatct atagtaataa tgtcttccat 660

atcatccatc cactcttccc cttcatttcc ccatattctt atcccccttc catttatccg 720

ccgattcctc tccgaggcaa ccttcctttt cccccctctt ctctctctct ctctctctct 780

ccataggcgt acgtaatccg cattccacag cctctcattg ctttgccccg gatgtaaatt 840

gattcgcccc acggggttgg actgccgatg tggagaagcg agccgccaga cttggcaagg 900

caaaccgtca tccaactgga gattactggc agtctggcac agcgtgcgtg tgagggagag 960

agggagagag agtttcccgg ggggaaacac agagtttcgc ctcccgcgtc gttccctcca 1020

gcaatgaaat catggatgat tgtgggacgt ggggaggcca cccccccccc cccccccccc 1080

cgcgggtaag attattcccg ccgtgccatt tccgattgcc ggtcagagtt cactatttaa 1140

tcctccagtt ttctctactt tttctggctc tgttcagctt caaaggcttt tcaatttgtt 1200

tctcatagac acatctcctc tctgattctc cttcgttctt atactatact atcatcatgg 1260

cgcaaactcc gcaagacctt aagattgcca ttttgggtgc tggtacgtac atcctttttt 1320

tttcttcccc ctccctataa tagggtttag tataggagta tggcagaaaa ctaacgggtg 1380

cgtggatgta taggcatggg tggcctgacc tgcgctttgg ctctggcaaa ggaaggtttc 1440

aagcacatcg acgtttatga gaatgcacac gatctgggct tcgttggagc tggtatccaa 1500

ctggccccga acatggcccg cgtgctggat cgcctgggcg tctggaagcc cattgaggcc 1560

gaagctgtta acatcacttc cactagcgtt agaggtatgt gctatattcc ctgctggagg 1620

attaaaagat ctacatgtct atctatatga aacaccgaga tcaaacgcta ataactccga 1680

acagtcggtg cctcggactc agaattggcc cacgttccat tggaatatat ccacgatacc 1740

tacggatatc ctcacatggt tggccaccgg tcttccctcg ccggccacct gtaccaaggt 1800

tgcctgcagc accctgacca gatccacttc cacttcgcca ctaccgccag cgacaccacc 1860

ttcggcccca agtcctcctt cactgccacc ccccgcgacg cccccgagaa ggcctaccgc 1920

gtcgaggccg atgtcctcct cgcctgtgat ggcatcaagt cccaggcccg tgtggccatg 1980

ctcgatctcc tcaacatcaa ggccagcgtc aaggacaccc accaggccgc ctaccgtatc 2040

atgatccaca aggaccagat caaggacgac ccggagctcc tggagctcat gaacggcaac 2100

cgcgtgactc gctggatcgg cgagaagcgc catatcatcg cctaccccgt ctccaacaac 2160

accatctaca acctctccac cacccaaccc gacactcact tcgccgccgc caccaatgcc 2220

acctacacca cccgcggtga caagtccgtc atgctgggcg tctttggcga cttctgcccc 2280

atggtccagc gtatgctgaa ctacgtcccc gagggtgaag tctgcgagtg gaagctgcgt 2340

gtccatgagc ccctccccac ctgggtgcac gagaccgtcg ccctcgtcgg tgatgcctgc 2400

caccctaccc tgccccatct cgcccagggt gccgcccagg ccatcgaaga cggtggtgtc 2460

attggtgccg tgctctccaa gctccccgat accaccccgg cttccatcca caaggccctc 2520

aaggtttacg agaaggtgcg caaggaccgc gcctacgcta tcgtcgaact ggccgccgct 2580

tccggtcgtg ctctgcacct gggtgaaggt gccgccaagg aggagcgcga tcgccaattc 2640

gctgctctcc gccagggcaa gggccctgtg cccgacaagt gggcggatgc cgatgtccag 2700

aaggagctgt atggcgttga tgcgactaag atcgcgctgg agcaattcga cgagctcttt 2760

gcttctgtgt aagctttggc ctttatacct ttttcgttta atttttcctt ttcccattca 2820

ttgcctgcac gattgaaccc gatgtatatt gtatatagag ctgattggtt gtattgatga 2880

ttgaaggtcg ggtcgtgagg caaagctggc ttgattgatt gattgattga ttgatcggcc 2940

ggaattagtg ttcttttatc tagcattgca gccccttgca tatacaatga tgatgttatg 3000

attgttatac agagttattt tctttgttcc tagatatact ccttttcgta gatgcatcca 3060

ttgtgtgaat gtatacaccc gtagttgtac caggacggaa gtggttggtt gcatagttag 3120

tctcgtttgt tagtgtcatg cgctggaatt taccccggat tttcagcccc tcctttagga 3180

atgaaaatcc taccaaatcc aaatcaaacc cgaggggttg gaccccttgg tccttgcact 3240

ccggccaggc aacaacaatt gtattggttt acccctcgag tttgtatggg agatacacga 3300

ttctagcgct gcacttccaa gacaaatcag tatctggggt ctacaatttt ccccccttcc 3360

ccaccattgc ggggatgcta atttgaaaag aatttatccc agatacacgt gagtaactgg 3420

ccattagtaa ttatgtgatg tcttatgtag tctacatact agagcacaaa ttttggtaca 3480

aattatggtc cccgataata tctgaaatcg ggatttgaat cagacgtgcg ggattagcta 3540

ggatccattt ctgccttgat ttgatagatc caaagaatag gtatcggaca aaagggtttc 3600

gacccaaatt tggagaatgg aaaaatctac actagcgacg ttctcgcatg ttagtggttt 3660

tacttagatc gaaatgtatt cacggaagca aagtccatga taatgaactc tatgtacttg 3720

gcatagccag cttatgcaca agcctcggca gcaaaaagta acctggtcta agctatgatt 3780

taaccttctt atcagattgt tggtgacggt ggcaaatcga ag 3822

<210> 18
<211> 24
<212> DNA
<213> Artificial Sequence

<220>

<223>  synthetic oligonucleotide

<400>  18
cgaactactt tcggcatctt ctag                                              24


<210>  19
<211>  49
<212>  DNA
<213>  Artificial Sequence

<220>
<223>  synthetic oligonucleotide

<400>  19
gcacttacct tcgcattttc tggtatattc gtaaatccag agacgtgcg                   49


<210>  20
<211>  49
<212>  DNA
<213>  Artificial Sequence

<220>
<223>  synthetic oligonucleotide

<400>  20
cctgtgttgg ttctcaggaa ctgcgaatat ggatacggaa gcggaaacg                   49


<210>  21
<211>  20
<212>  DNA
<213>  Artificial Sequence

<220>
<223>  synthetic oligonucleotide

<400>  21
ccgccatgtt agatcttggc                                                   20


<210>  22
<211>  21
<212>  DNA
<213>  Artificial Sequence

<220>
<223>  synthetic oligonucleotide

<400>  22
cgcaagttca ttgacatcgg c                                                 21


<210>  23
<211>  23
<212>  DNA
<213>  Artificial Sequence

<220>
<223>  synthetic oligonucleotide

<400>  23

ggaagaaatg ttcacaccag tcg 23

<210> 24
<211> 24
<212> DNA
<213> Artificial Sequence

<220>
<223> synthetic oligonucleotide

<400> 24
cgaactactt tcggcatctt ctag 24

<210> 25
<211> 49
<212> DNA
<213> Artificial Sequence

<220>
<223> synthetic oligonucleotide

<400> 25
gcacttacct tcgcattttc tggtatattc gtaaatccag agacgtgcg 49

<210> 26
<211> 49
<212> DNA
<213> Artificial Sequence

<220>
<223> synthetic oligonucleotide

<400> 26
cctgtgttgg ttctcaggaa ctgcgaatat ggatacggaa gcggaaacg 49

<210> 27
<211> 20
<212> DNA
<213> Artificial Sequence

<220>
<223> synthetic oligonucleotide

<400> 27
ccgccatgtt agatcttggc 20

<210> 28
<211> 21
<212> DNA
<213> Artificial Sequence

<220>
<223> synthetic oligonucleotide

<400> 28
cgcaagttca ttgacatcgg c 21

<210>  29
<211>  23
<212>  DNA
<213>  Artificial Sequence

<220>
<223>  synthetic oligonucleotide

<400>  29
ggaagaaatg ttcacaccag tcg                                              23


<210>  30
<211>  20
<212>  DNA
<213>  Artificial Sequence

<220>
<223>  synthetic oligonucleotide

<400>  30
gtcagcgcca atcagtaacc                                                  20


<210>  31
<211>  55
<212>  DNA
<213>  Artificial Sequence

<220>
<223>  synthetic oligonucleotide

<400>  31
gaaagagtca ccggtcactg tacagagctc gtgtggtaaa aggggagaaa ggtag           55


<210>  32
<211>  56
<212>  DNA
<213>  Artificial Sequence

<220>
<223>  synthetic oligonucleotide

<400>  32
tgtaagcgcc cactccacat ctccactcga gaagacttgc catgatttgg tgatac          56


<210>  33
<211>  25
<212>  DNA
<213>  Artificial Sequence

<220>
<223>  synthetic oligonucleotide

<400>  33
ctggcaagaa tgaaatgagt gagag                                            25


<210>  34
<211>  24
<212>  DNA

<213>  Artificial Sequence

<220>
<223>  synthetic oligonucleotide

<400>  34
ctgatcagac acatccacga cttc                                              24


<210>  35
<211>  23
<212>  DNA
<213>  Artificial Sequence

<220>
<223>  synthetic oligonucleotide

<400>  35
gaggggtaga tctggttgat gac                                               23


<210>  36
<211>  20
<212>  DNA
<213>  Artificial Sequence

<220>
<223>  synthetic oligonucleotide

<400>  36
ctgggtgcca ttggcagtag                                                   20


<210>  37
<211>  52
<212>  DNA
<213>  Artificial Sequence

<220>
<223>  synthetic oligonucleotide

<400>  37
gcacttacct tcgcattttc tggtatattc ggagtttgcg ccatgatgat ag               52


<210>  38
<211>  54
<212>  DNA
<213>  Artificial Sequence

<220>
<223>  synthetic oligonucleotide

<400>  38
cctgtgttgg ttctcaggaa ctgcgaatat cgagctcttt gcttctgtgt aagc             54


<210>  39
<211>  21
<212>  DNA
<213>  Artificial Sequence

<220>

<223>  synthetic oligonucleotide

<400>  39
cttcgatttg ccaccgtcac c                                                21


<210>  40
<211>  22
<212>  DNA
<213>  Artificial Sequence

<220>
<223>  synthetic oligonucleotide

<400>  40
cactagcgtt agaggtatgt gc                                               22


<210>  41
<211>  21
<212>  DNA
<213>  Artificial Sequence

<220>
<223>  synthetic oligonucleotide

<400>  41
gtggatcatg atacggtagg c                                                21


<210>  42
<211>  20
<212>  DNA
<213>  Artificial Sequence

<220>
<223>  synthetic oligonucleotide

<400>  42
ctgggtgcca ttggcagtag                                                  20


<210>  43
<211>  52
<212>  DNA
<213>  Artificial Sequence

<220>
<223>  synthetic oligonucleotide

<400>  43
gcacttacct tcgcattttc tggtatattc ggagtttgcg ccatgatgat ag              52


<210>  44
<211>  54
<212>  DNA
<213>  Artificial Sequence

<220>
<223>  synthetic oligonucleotide

<400>  44

cctgtgttgg ttctcaggaa ctgcgaatat cgagctcttt gcttctgtgt aagc 54

<210> 45
<211> 21
<212> DNA
<213> Artificial Sequence

<220>
<223> synthetic oligonucleotide

<400> 45
cttcgatttg ccaccgtcac c 21

<210> 46
<211> 22
<212> DNA
<213> Artificial Sequence

<220>
<223> synthetic oligonucleotide

<400> 46
cactagcgtt agaggtatgt gc 22

<210> 47
<211> 21
<212> DNA
<213> Artificial Sequence

<220>
<223> synthetic oligonucleotide

<400> 47
gtggatcatg atacggtagg c 21

<210> 48
<211> 20
<212> DNA
<213> Artificial Sequence

<220>
<223> synthetic oligonucleotide

<400> 48
gtcagcgcca atcagtaacc 20

<210> 49
<211> 55
<212> DNA
<213> Artificial Sequence

<220>
<223> synthetic oligonucleotide

<400> 49
gaaagagtca ccggtcactg tacagagctc gtgtggtaaa aggggagaaa ggtag 55

<210>  50
<211>  56
<212>  DNA
<213>  Artificial Sequence

<220>
<223>  synthetic oligonucleotide

<400>  50
tgtaagcgcc cactccacat ctccactcga gaagacttgc catgatttgg tgatac          56


<210>  51
<211>  25
<212>  DNA
<213>  Artificial Sequence

<220>
<223>  synthetic oligonucleotide

<400>  51
ctggcaagaa tgaaatgagt gagag                                            25


<210>  52
<211>  24
<212>  DNA
<213>  Artificial Sequence

<220>
<223>  synthetic oligonucleotide

<400>  52
ctgatcagac acatccacga cttc                                             24


<210>  53
<211>  23
<212>  DNA
<213>  Artificial Sequence

<220>
<223>  synthetic oligonucleotide

<400>  53
gaggggtaga tctggttgat gac                                              23


<210>  54
<211>  4579
<212>  DNA
<213>  Artificial Sequence

<220>
<223>  synthetic oligonucleotide


<220>
<221>  misc_feature
<222>  (3133)..(3133)
<223>  n is a, c, g, or t

<220>

<221>  misc_feature
<222>  (3341)..(3341)
<223>  n is a, c, g, or t

<220>
<221>  misc_feature
<222>  (3432)..(3432)
<223>  n is a, c, g, or t

<400>  54
gtcagcgcca atcagtaacc gtcaaaaagt aaaggcggac acaccgctgt cctcttttgg      60

agaagagcgc aggtttttgt tttccccccc ggcagtcacc ccgcccccgc ccccggattg     120

ccagccgtcg acttgccggc agaatttttg ggcgaagaaa gatttgctag gacattgatt     180

gtgagaggag ttttgtgtct ttctatgtca atgaggagag ttgaaaggct atgaaaatag     240

attatcttat gtgtccataa ataattgtat gctgtagcat cgataccaaa gatatgctgt     300

cagaaggctc acctgccttc cagagtagga ttactacttg aatcaacttg attggtccac     360

actaaagagc gtaatgtgca tagaattcct ggtccagggg ctaatcaacc aattccagta     420

aggctgttgg ccggttggtg aatagcaggg cggaaaaaca gttcgtcgag gaggagtcag     480

tccggatagg aaggcggaga gactggggaa gccagggagc acttacccgc gggtaagagc     540

atcccccgga tcaccgcgca gcaatgcagc ttgatatcaa aatcaattat cgattttgat     600

atgcaggtaa tcctgctaat tcggcaaggc aaatgcaacc tgccgtcgtt cggctgttcc     660

acttacgaac tgcgaaaact gaaacatcgc atcatgcaac ccatcagatc cgtcggcatg     720

tggcaacggt aactttgggt gtctggtacc gtcgcactgt ccccacacgg agttgtagcc     780

gatcgatttg ccgtcgctcg ccgttctatc cagacttgcc acgaccaaat tcaccgtgta     840

tatagacctc cagacctcgc tggatttttg tctctcatca ctcctctcca tctcatcctc     900

tttccaactt ctactcttct acctttctcc ccttttacca cacgagctct gtacagtgac     960

cggtgactct ttctggcatg cggagagacg gacggacgca gagagaaggg ctgagtaata    1020

agccactggc cagacagctc tggcggctct gaggtgcagt ggatgattat taatccggga    1080

ccggccgccc ctccgccccg aagtggaaag gctggtgtgc ccctcgttga ccaagaatct    1140

attgcatcat cggagaatat ggagcttcat cgaatcaccg gcagtaagcg aaggagaatg    1200

tgaagccagg ggtgtatagc cgtcggcgaa atagcatgcc attaacctag gtacagaagt    1260

ccaattgctt ccgatctggt aaaagattca cgagatagta ccttctccga agtaggtaga    1320

gcgagtaccc ggcgcgtaag ctccctaatt ggcccatccg gcatctgtag ggcgtccaaa    1380

tatcgtgcct ctcctgcttt gcccggtgta tgaaaccgga aaggccgctc aggagctggc    1440

cagcggcgca gaccgggaac acaagctggc agtcgaccca tccggtgctc tgcactcgac    1500

ctgctgaggt ccctcagtcc ctggtaggca gctttgcccc gtctgtccgc ccggtgtgtc    1560

ggcggggttg acaaggtcgt tgcgtcagtc caacatttgt tgccatattt tcctgctctc    1620

cccaccagct gctcttttct tttctctttc ttttcccatc ttcagtatat tcatcttccc 1680

atccaagaac ctttatttcc cctaagtaag tactttgcta catccatact ccatccttcc 1740

catcccttat tcctttgaac ctttcagttc gagctttccc acttcatcgc agcttgacta 1800

acagctaccc cgcttgagca gacatcacca tgcctgaact caccgcgacg tctgtcgaga 1860

agtttctgat cgaaaagttc gacagcgtct ccgacctgat gcagctctcg gagggcgaag 1920

aatctcgtgc tttcagcttc gatgtaggag ggcgtggata tgtcctgcgg gtaaatagct 1980

gcgccgatgg tttctacaaa gatcgttatg tttatcggca ctttgcatcg gccgcgctcc 2040

cgattccgga agtgcttgac attggggaat tcagcgagag cctgacctat tgcatctccc 2100

gccgtgcaca gggtgtcacg ttgcaagacc tgcctgaaac cgaactgccc gctgttctgc 2160

agccggtcgc ggaggccatg gatgcgatcg ctgcggccga tcttagccag acgagcgggt 2220

tcggcccatt cggaccgcaa ggaatcggtc aatacactac atggcgtgat ttcatatgcg 2280

cgattgctga tccccatgtg tatcactggc aaactgtgat ggacgacacc gtcagtgcgt 2340

ccgtcgcgca ggctctcgat gagctgatgc tttgggccga ggactgcccc gaagtccggc 2400

acctcgtgca cgcggatttc ggctccaaca atgtcctgac ggacaatggc cgcataacag 2460

cggtcattga ctggagcgag gcgatgttcg gggattccca atacgaggtc gccaacatct 2520

tcttctggag gccgtggttg gcttgtatgg agcagcagac gcgctacttc gagcggaggc 2580

atccggagct tgcaggatcg ccgcggctcc gggcgtatat gctccgcatt ggtcttgacc 2640

aactctatca gagcttggtt gacggcaatt tcgatgatgc agcttgggcg cagggtcgat 2700

gcgacgcaat cgtccgatcc ggagccggga ctgtcgggcg tacacaaatc gcccgcagaa 2760

gcgcggccgt ctggaccgat ggctgtgtag aagtactcgc cgatagtgga aaccgacgcc 2820

ccagcactcg tccgagggca aaggaataga gtagatgccg accgcgggat ccacttaacg 2880

ttactgaaat catcaaacag cttgacgaat ctggatataa gatcgttggt gtcgatgtca 2940

gctccggagt tgagacaaat ggtgttcagg atctcgataa gatacgttca tttgtccaag 3000

cagcaaagag tgccttctag tgatttaata gctccatgtc aacaagaata aaacgcgttt 3060

tcgggtttac ctcttccaga tacagctcat ctgcaatgca ttaatgcatt gactgcaacc 3120

tagtaacgcc ttncaggctc cggcgaagag aagaatagct tagcagagct attttcattt 3180

tcgggagacg agatcaagca gatcaacggt cgtcaagaga cctacgagac tgaggaatcc 3240

gctcttggct ccacgcgact atatatttgt ctctaattgt actttgacat gctcctcttc 3300

tttactctga tagcttgact atgaaaattc cgtcaccagc ncctgggttc gcaaagataa 3360

ttgcatgttt cttccttgaa ctctcaagcc tacaggacac acattcatcg taggtataaa 3420

cctcgaaatc anttcctact aagatggtat acaatagtaa ccatgcatgg ttgcctagtg 3480

aatgctccgt aacacccaat acgccggccg aaactttttt acaactctcc tatgagtcgt 3540

ttacccagaa tgcacaggta cacttgttta gaggtaatcc ttctttctag aagtcctcgt 3600

gtactgtgta agcgcccact ccacatctcc actcgagaag acttgccatg atttggtgat 3660

acgaagcatt gatgtacata gttttgcatt tgtcgaactt tcagcgatga cccttgaaca 3720

tactgtttga atacagactt ttttcatacc cttcagattg aactttttta gtgccttcgt 3780

atgtagttag tagacccacc atgccttcga ctgttcttcc agatcatcat agcacctgca 3840

taagcatact gggtccagtg cggggtcccc gggttaccct ccgcaaatcg gatcctggcc 3900

aatgtcatcg tcccacgtag ttagtagtac ccagactggg tgcatcccgt cgacagatcc 3960

tcccatctag taccagctcg tagtacttct attggccccc acagagctgc accaggcccc 4020

ttcaaatagt cagacacgaa ggaagaatta tttttggcat actgaatggt tcatgtgtca 4080

tgacagtgct gggatataca gggtggcaaa agccggaatt gcggatatac tgatgccatt 4140

accgattcga agcagatgtc aaagtgatca ctgttgttgg atgtgcctgt atatcgtggt 4200

aacatcagcg atctgcctgt tgcgaatcga aacaattctt ggtccttgat attgttttac 4260

gcagtttgag caccagcctc cataaagttg ggaatcggta agatgtagtt tcgtctgcat 4320

caacgaggag tgatatcacg ccaataatag taaattctat gcctaatcct atcgctcaga 4380

gctccccaca tactgattca gacttctcta gtgaaacact ttcgtagaac agagaaaaca 4440

gttggtgtct ccagttattg cgctcgaagc gattaatacc catctgcggg atgatcagac 4500

tgagaggtac tgtgttgtgc tcgccaagga gatatgctgc attcaaatat ccaactctca 4560

ctcatttcat tcttgccag 4579

<210> 55
<211> 4446
<212> DNA
<213> Artificial Sequence

<220>
<223> synthetic oligonucleotide

<400> 55
gacgatgatc cggatgccaa ccagcggctg atgcaggcga ttctggatac ggtggaccac 60

cacacgcggg tggcggcggc ggtgaaggag gggcatgaag tgagcaacga gcgggagcgg 120

gagcgcgagc aggaacgaga gcgggagcgg aagttggtgc gggcgtagct ttgacccgaa 180

tttttgtttg ggaattatgg atatggatac cttggacagg acatgggagt tgtatttagg 240

tttttgattg agcgagttag cggatatccc ggtttgaatt ctggatgaaa tgatggattg 300

gatggattgg atagcatgga tgggcatagc ataatgtgac tgcatatgca tacaggatct 360

actctgtaca gaagaaccag tgaatgtgtg tcggagtttg tcattcccgc cgcggcacca 420

actgccgatg ggcgatccgc accccgcaga cacttgatcc taatagatct gaacactact 480

actttatact actatttttc aaacaaggat actcagacat ccaaccccac agatcgtcac 540

gtcaatctct cgatccagta ttccttatat ccatatatcc gcaaagtagt aatgaatctc 600

gggaatggcc atagccgatc tcctcgctga gtgatgctta cgtagcacca ccatccgaac 660

tacccagcca atgagaccgc gtcaatttac acccatccac cacccacttt tctttcccat 720

ccatagcaat gtggggtgtc cccgcagtct tcattcacct atatagtaga ttccccccca 780

ccccataccc attgcaattg cgccctctat tcttctcacc cctcaccact aatcaatata 840

ccagaaaatg cgaaggtaag tgcttctata ttgatcctta gtgctttcaa actgtgatgt 900

agaagttgct cggtagctga ttaaatattc tagacccaag ccgctgctgg aattgacatt 960

attatggccg atagggttgg gcttattgct atgtccctga aaggatatca aaagcaggca 1020

aaaagccagg cataatcccc gcgtggacgg taccctaagg ataggcccta atcttatcta 1080

catgtgactg catcgatgtg tttggtcaaa atgaggcatg tggctcaccc cacaggcgga 1140

gaaacgtgtg gctagtgcat gacagtcccc tccatagatt caatttaatt tttcgcggca 1200

attgtcgtgc agtttgtatc tacatttcat tccatatatc aagagttagt agttggacat 1260

cctgattatt ttgtctaatt actgaaaact cgaagtacta acctactaat aagccagttt 1320

caaccactaa gtgctcattt atacaatatt tgcagaaccc cgcgctaccc ctccatcgcc 1380

aacatgtctt ccaagtcgca attgacctac agcgcacgcg ctagcaagca ccccaatgcg 1440

ctcgtaaaga agctcttcga ggttgccgag gccaagaaaa ccaatgtcac cgtttccgcc 1500

gacgtgacaa ccaccaaaga gctgctggat ttggctgacc gtatgcgcac cggggatgcc 1560

acttacatgt gatctagtaa tggttaatgg tggattatat aacaggactc ggtccgtaca 1620

ttgccgtgat caaaactcac atcgatatcc tctccgattt cagcgaagaa accatcaccg 1680

gtctgaaggc ccttgcagag aagcacaatt tcctcatctt cgaagatcgc aagttcatcg 1740

atatcggaaa cacagtccaa aagcagtacc atggcggcac tctgcgtatc tctgagtggg 1800

cccacatcat caactgcagt attctgcccg gtgagggtat cgtcgaggct ctggcccaga 1860

ctgcttcggc cgaggacttc ccctacggct ccgagagggg ccttttgatc cttgcggaga 1920

tgacctccaa gggatctttg gctaccggtc aatatactac ttcttctgtt gactatgctc 1980

ggaagtataa gaagtttgtg atgggattcg tctcgacacg tcaccttggc gaggttcagt 2040

ctgaagttag ctcgccttcg gaggaggaag attttgtcgt cttcacgaca ggtgtcaacc 2100

tctcctcgaa gggtgacaag ctgggacagc agtaccaaac tcctgagtcg gctgttggac 2160

gcggtgccga ctttattatt gctggccgtg gaatttatgc tgctcctgat cccgtggagg 2220

cggcgaagca gtaccagaag gagggatggg atgcatacct gaagcgtgtt ggtgcgcaat 2280

aagtagtggt ggatacgtac tccttttatg gcagtatgtc gcaagtatga tgcgatttat 2340

aaattcagca ctcgaaatga ctactactat gtgtctacga cagataccct ctccgtacga 2400

ataagacacc tgcctcgata tatggacaaa ttcaaaatca gggtcaaggg tcatgtttca 2460

aagtcacaac aatctccaac atagacgaga atttgtaccg gagtgtctga aggtgcagct 2520

ggagattggt ctattttctt agagtggggt atcactaatg tacagtcggt cactatcgta 2580

caaacaatca caattatata caagatttcc caccaccccc tactctaaca cggcacaatt 2640

atccatcgag tcagagccta gccaccattt ggtgctctcg tagagaccaa agtataatcc 2700

tgatccgaca gcggccataa acgtgttgat agcacaccct cggaatagtc ctctcgggcc 2760

atctgttcgt acaatctccc gtacggtatt gatcatcctt ttcttctgag gtgcagttgt 2820

atctgcagca tcgagcatga ttcgtgtccg gaccatatcc atgggtgctg tcaagacact 2880

agctataccg cccgagaccg cagcacttat tgcggctgtc gctgcagcct ctccgattgt 2940

cgaatgggcc tctttctttc catactctct tggtctttct agcaccttct ctcgatctcc 3000

gaatctatat tcaaaaattc gataccgaaa agactcgtac agaggcatct gaatcgccga 3060

cactggcaag ctatgcgcca caagagccgg gtatccgctc caaagctgtc tagggttgat 3120

aaacttcttg aaagctagcc gtgtcgcttt ctgggctaca ccacctaccc ttcccccagc 3180

tacaggtgct gatgcgtctg gatggtgtga ttggatcatc tgcgcgttgt gttttaatgc 3240

atcagccgga gcaaagactc cgcaagcagc aagatccgca acggaggctg cgcaaaaatc 3300

ggaaaagagc cgagctgagc tagactcatg cgttccaagt ttttgatgta tgacttggag 3360

tcctgactgt gcatactcgt atgtgatgaa gaatgcgccc gctttgaccc gttatttttt 3420

gctcaatgaa tatgagttgg gtaaggataa tgttgggtat caccaacctg tgggaaatga 3480

ggcagcggtt acgctcgcga taccttggta aaggccgcgg aatatccctg ggtgtctcca 3540

tatgctggtt cctgtgttgg ttctcaggaa ctgcgaatat gacattggtg ttggcttctg 3600

cttctgcatt caccatctct ccaaagtcac ttccctccac tgctgccgca ccatccttca 3660

atcgtcgcag tccactctca tcttccaccc agccaccgac cttctccact cctgcgatat 3720

actctgtccc aatgcgcagg ttcagccctg cgctcaacgc ccgttcgatc cgcacttcgc 3780

gtccctcagc aatcagcacc cggaacacgt ggcccaggaa cccgctcagg aggtccacgc 3840

tccactcgtc cggcacgaca cgcagcacct cgcccacgtc gaaccacgcc gcgaatcgtc 3900

ctagcaggtc gctcgtccgc tcgagccgct ctgacggatc ctggatctgc aggaactcgt 3960

ccagcaggta ccggaagagc tctgattgca gggatcgctc ggcgaattcg accagcgctc 4020

ctgtcgtgct ggtgctgcgc gggccgccga agaggcagta tcgtacggcg gagtcgtagt 4080

cgcctagtcc gtgggtgagc aggtgaagcg cctcgcggtg tcgaccttgt agtccgtcta 4140

gaatcactga ttctgatacc agttcgttct ggaacggctc gatccgcgcc agcaccgccg 4200

ggatggagta tgtcagattg cgcgcgggca gggaggtgaa gtgcccgccg ctgccgcctc 4260

cgaggagttg aagaaggcgg agccgtgatt gccaccatgg ctcggcaggg gtgttttcgg 4320

tgatgaagtt catgtaggtg ggtttgggcg gctgcagggc gcggtacgta gagtaggatt 4380

ctgcgagtga tgcacgcgct tctggggaag actcgagcac ggagagtacg gtgtccaggt 4440

agtagg 4446

<210> 56
<211> 20
<212> DNA
<213> Artificial Sequence

<220>
<223> synthetic oligonucleotide

<400> 56
cgacaacatc accgagaacg 20

<210> 57
<211> 20
<212> DNA
<213> Artificial Sequence

<220>
<223> synthetic oligonucleotide

<400> 57
gtgggtatct ttgacggtgc 20

<210> 58
<211> 20
<212> DNA
<213> Artificial Sequence

<220>
<223> synthetic oligonucleotide

<400> 58
gacgatgatc cggatgccaa 20

<210> 59
<211> 52
<212> DNA
<213> Artificial Sequence

<220>
<223> synthetic oligonucleotide

<400> 59
gcacttacct tcgcattttc tggtatattg attagtggtg aggggtgaga ag 52

<210> 60
<211> 51
<212> DNA
<213> Artificial Sequence

<220>
<223>  synthetic oligonucleotide

<400>  60
cctgtgttgg ttctcaggaa ctgcgaatat gacattggtg ttggcttctg c                51


<210>  61
<211>  21
<212>  DNA
<213>  Artificial Sequence

<220>
<223>  synthetic oligonucleotide

<400>  61
cctactacct ggacaccgta c                                                 21